# EUROPEAN PATENT APPLICATION

(11) **EP 3 868 874 A1**
(43) Date of publication of application: **25.08.2021**
(21) Application number: 19870535.2
(22) Date of filing: 14.10.2019
(51) Int. Cl.: C12N 7/00, C07K 14/005, A61K 39/155, A61K 39/295, A61P 31/14, A61K 39/00

(54) **RECOMBINANT RSV LIVE VACCINE STRAIN AND PRODUCTION METHOD THEREFOR**

(30) Priority: 12.10.2018 KR 20180122162
(71) Applicant: SK Bioscience Co., Ltd., Seongnam-si, Gyeonggi-do 13494 (KR)
(72) Inventor: SEO, Ki-weon, Seongnam-si, Gyeonggi-do 13494 (KR); KIM, Eun-som, Seongnam-si, Gyeonggi-do 13494 (KR); KWON, Teawoo, Seongnam-si, Gyeonggi-do 13494 (KR); HONG, Seung-hye, Seongnam-si, Gyeonggi-do 13494 (KR); KIM, Hun, Seongnam-si, Gyeonggi-do 13494 (KR); LEE, Su Jeen, Seongnam-si, Gyeonggi-do 13494 (KR)
(74) Representative: Engelhard, Markus
(86) International application number: PCT/KR2019/013447
(87) International publication number: WO 2020/076141

(57) **Abstract**

The present invention provides a gene recombinant respiratory syncytial virus (RSV) in which genes encoding the envelope proteins of an RSV are rearranged, wherein in the RSV, a gene encoding the fusion protein (F protein) derived from a heterologous virus belonging to the family Paramyxoviridae or the family Pneumoviridae is inserted between the genes respectively encoding the glycoprotein (G protein) and the F protein of the RSV, or the gene encoding the F protein of the RSV is substituted with a gene encoding the F protein of a heterologous virus belonging to the family Paramyxoviridae or the family Pneumoviridae. The recombinant RSV of the present invention can be used as an RSV vaccine strain, and can be used as a vaccine due to having excellent stability and safety.

## Description

### TECHNICAL FIELD

The present application claims priority to Korean Patent Application No. 10-2018-0122162 filed on October 12, 2018 in the Republic of Korea, the disclosures of which are incorporated herein by reference. The present disclosure relates to an RSV live vaccine strain and a method for producing the same. More specifically, it relates to a recombinant RSV live vaccine and a method for preparing the same.

### BACKGROUND ART

Respiratory syncytial virus (RSV) is a very common, contagious virus that causes respiratory diseases. Particularly, it is the main cause of death of infants due to severe respiratory tract infections. Although infants are at highest risk for infection, it is also known to cause fatal respiratory diseases in immunocompromised patients and the elderly by causing infections of the respiratory tract. While it is the second cause of respiratory diseases next to influenza, it is known that deaths caused by RSV are about 1.3-2.5 times more than influenza in infants. According to the WHO's report in 2002, 64 million people are infected with RSV every year, and 160,000 of them die.

For defense against RSV, a vaccine using inactivated virus (inactivated vaccine) was developed first. However, the use of the inactivated vaccine has become impossible due to a severe side effect (ERD; enhanced respiratory disease). Therefore, researches have made efforts to develop a vaccine with excellent ability of inducing neutralizing antibodies without causing ERD.

Meanwhile, although a live vaccine is advantageous in that it has excellent ability of inducing neutralizing antibodies without causing ERD, vaccine development is difficult when considering stability and safety issues of the virus because RSV is very unstable (metastable).

RSV belongs to the subfamily *Orthopneumoviridae* of the family *Pneumoviridae,* order *Mononegavirales.* RSV is a medium-sized virus of 120-200 nm. It has a surface structure consisting of an envelope and three transmembrane proteins (F and G glycoproteins and SH protein), and has a linear, negative-sense, single-stranded RNA genome of about 15,000 nucleotides in length. RSV is divided into serotypes A and B based on the G protein. The genome of RSV encodes 11 proteins, and is composed of a portion encoding structural proteins and a portion encoding non-structural proteins. The non-structural proteins, nonstructural (NS) 1 and nonstructural (NS) 2, act on the immune escape mechanism as type I interferon antagonists. Among the structural proteins, fusion (F) protein, glyco (G) protein and small hydrophobic (SH) protein constitute the viral surface as glycoproteins, and nucleocapsid (N) protein, phosphoprotein (P), matrix (M) protein, viral polymerase (L), etc. constitute the internal structure of the virus or play important roles in viral replication.

RSV's F protein is an important factor involved in the initial stage of virus entry and fusion with cell membranes, and is known as a major target for vaccines and antiviral drugs due to high antigenicity. However, it is difficult to produce and purify the F protein antigen as a stable prefused protein, and it is difficult to maintain its stability and efficacy.

Therefore, the inventors of the present disclosure aim at solving the instability of the F protein and developing a new type of safe RSV live vaccine.

### DISCLOSURE

### Technical Problem

The present disclosure is directed to developing a live RSV vaccine strain with superior stability by substituting or inserting a heterogeneous F protein with high stability.

The present disclosure is directed to developing a live RSV vaccine strain with superior safety (attenuated) by substituting or inserting a heterogeneous F protein with high stability.

The present disclosure is directed to developing a new recombinant RSV.

The present disclosure is directed to providing a new type of RSV vaccine capable of inducing a defense mechanism against RSV. The present disclosure is directed to providing a new type of vaccine allowing effective operation of the immune system by inhibiting the immune escape mechanism through mutation of the G protein of RSV.

The present disclosure is directed to producing a bivalent vaccine by inserting or substituting a heterogeneous F protein.

### Technical Solution

In an exemplary embodiment, the present disclosure provides a recombinant respiratory syncytial virus (RSV). It provides a recombinant respiratory syncytial virus (RSV) in which the genes encoding the envelope proteins of RSV are rearranged. That is to say, the present disclosure provides a recombinant respiratory syncytial virus wherein a foreign gene is inserted in an RSV gene. In an exemplary embodiment, the present disclosure provides a recombinant respiratory syncytial virus wherein an F protein derived from a virus other than RSV is inserted between the genes encoding the glycoprotein (G protein) and the fusion protein (F protein) of RSV or substituted instead of the F protein of RSV. Specifically, (1) a gene encoding the F protein derived from a heterologous virus belonging to the family *Paramyxoviridae* or the family *Pneumoviridae* may be inserted, or (2) a gene encoding the F protein of RSV may be substituted with a gene encoding the F protein derived from a heterologous virus belonging to the family *Paramyxoviridae* or the family *Pneumoviridae.* The heterologous virus belonging to the family *Paramyxoviridae* or the family *Pneumoviridae* may be one or more virus selected from a group consisting of the genera *Aquaparamyxovirus, Avulavirus, Ferlavirus, Henipavirus, Morbillivirus, Respirovirus, Rubulavirus, Metapneumovirus* and *Orthopneumovirus,* specifically one or more virus selected from a group consisting of measles virus and Newcastle disease virus. The recombinant respiratory syncytial virus (RSV) may be a recombinant RSV which is based on the human RSV A virus strain of SEQ ID NO 1 and wherein the genes encoding the envelope proteins of the RSV A virus strain (G protein- and F protein-expressing domains) are rearranged in the recombinant RSV backbone of SEQ ID NO 3. Specifically, it may have cDNA sequences of SEQ ID NOS 4-7. A sequence wherein a restriction enzyme sequence is added to the antigenome of RSV may be represented by SEQ ID NO 2.

In another exemplary embodiment, the present disclosure provides a method for producing an attenuated recombinant RSV using an expression vector including a polynucleotide encoding a recombinant RSV antigenome. In an exemplary embodiment, the recombinant RSV may be produced by expressing a first expression vector including a polynucleotide encoding a recombinant RSV antigenome and a second expression vector including a polynucleotide encoding one or more protein selected from a group consisting of N, P, L and M2-1 proteins together. The polynucleotide encoding a recombinant RSV antigenome includes a polynucleotide encoding a domain of a protein derived from a heterogeneous virus wholly or partially. The polynucleotide encoding a recombinant RSV antigenome included in the first expression vector is any one selected from SEQ ID NOS 4-7. The polynucleotide selected from SEQ ID NOS 4-7 may be a cDNA. The first expression vector may include the polynucleotide encoding a recombinant RSV antigenome in a pCC1 plasmid.

In an exemplary embodiment, the present disclosure provides a recombinant RSV expression vector. The expression vector may include the genes encoding the glycoprotein (G protein) and the fusion protein (F protein) of RSV, wherein a gene encoding the F protein derived from a heterologous virus belonging to the family *Paramyxoviridae* or the family *Pneumoviridae* is inserted between the genes encoding the G protein and the F protein, or may include a recombinant RSV gene in which a gene encoding the F protein derived from a heterologous virus belonging to the family *Paramyxoviridae* or the family *Pneumoviridae* is substituted instead of a gene encoding the fusion protein (F protein) of RSV. The recombinant RSV gene may be any one selected from SEQ ID NOS 4-7. The recombinant RSV expression vector includes a T7 promoter, a hammerhead ribozyme, a recombinant RSV gene, a hepatitis delta virus ribozyme and a T7 terminator in sequence. The heterologous virus belonging to the family *Paramyxoviridae* or the family *Pneumoviridae* may be one or more virus selected from the genera *Aquaparamyxovirus, Avulavirus, Ferlavirus, Henipavirus, Morbillivirus, Respirovirus, Rubulavirus, Metapneumovirus* and *Orthopneumovirus,* specifically one or more selected from a group consisting of measles virus and Newcastle disease virus.

In another exemplary embodiment, the present disclosure provides an attenuated RSV live vaccine composition containing the recombinant respiratory syncytial virus (RSV) according to an exemplary embodiment of the present disclosure and a pharmaceutically acceptable carrier. The present disclosure also provides a method for preventing respiratory syncytial virus (RSV) infection by administering the live vaccine composition into the human body.

The present disclosure provides a live RSV strain, which is a recombinant respiratory syncytial virus (RSV) in which a polynucleotide encoding the domain of an F protein derived from a heterologous virus wholly or partially is inserted in the RSV genome. The polynucleotide is i) inserted instead of a polynucleotide encoding the domain of the RSV F protein partially, or ii) inserted in any portion of the polynucleotide constituting the RSV gene. The recombinant RSV of the present disclosure may be used as an RSV vaccine strain and may be used as a vaccine because it exhibits excellent stability and safety.

Hereinafter, the present disclosure is described specifically.

The present disclosure is directed to providing a new type of RSV live vaccine in which a gene encoding the F protein of RSV is substituted with a gene encoding the F protein of a heterogeneous virus, or a gene encoding the F protein of a heterogeneous virus is introduced further.

### [Definition of terms]

The term "recombinant" may mean that cells with different hereditary characteristics exist together in an organism. Particularly, the term "recombinant" used in the present disclosure refers to the occurrence of new genetic variation as the genetic information (nucleic acid) of different individuals based on one genetic backbone is inserted.

A recombinant RSV refers to an RSV or RSV-like virus derived directly or indirectly or produced from a recombinant expression system or proliferated from subviral particles. The recombinant expression system includes a recombinant expression vector, which includes a functionally linked transcription unit including at least one genetic element or a combination of elements having regulatory functions in the expression of the RSV gene. Examples of the element include a promoter, a structural or coding sequence transcribed to RSV mRNA, or an appropriate initiation or termination sequence.

The term "genome" used in the present disclosure refers to all the base sequences of the genes of an individual, which is a collection of all the genetic information of an organism. For example, the genome of a virus is used to mean all the genetic information of the virus.

The term "gene" used in the present disclosure may be understood as a portion which encodes a protein, which may be a DNA or an RNA.

The term "introduction" used in the present disclosure refers to addition of a new element to the original elements. Mutation of a gene, such as deletion, substitution, addition, etc., may be induced by the introduction.

For example, the expression that "a new gene was introduced to a genome" used in the present disclosure may mean that a portion of the whole gene base sequence of an individual was substituted or inserted with a gene derived from a new individual. As a result of the introduction, the original genome base sequence (e.g., backbone) of the individual may become longer or shorter, or may remain unchanged.

The term "vaccine strain" used in the present disclosure refers to a population of viruses isolated for use as a vaccine.

The term "live attenuated vaccine" used in the present disclosure refers to a vaccine prepared from attenuated respiratory syncytial virus (RSV), which has attenuated virulence of a pathogen so as not to cause a disease in a host but still keeps the pathogen viable.

The term "antigenome" used in the present disclosure refers to a complementary (+)-sense polynucleotide molecule that functions as a template for synthesis of a progeny RSV genome. The native RSV genome generally includes (-)-sense polynucleotide molecules, which encode viral proteins, such as glycoproteins (G), fusion proteins (F), N, P, etc. through complementary viral mRNAs. The attenuated RSV live vaccine strain may be prepared through recombination of cDNAs encoding the RSV genome or antigenome.

The term "gene encoding a protein" or "protein-encoding gene" used in the present disclosure refers to a gene which produces a protein.

In an exemplary embodiment, the present disclosure provides a live RSV vaccine strain with superior stability and safety in which an F protein of a heterologous virus is inserted into the RSV genome, or the F protein of RSV is substituted with an F protein of a heterologous virus.

In an exemplary embodiment, the present disclosure provides a recombinant respiratory syncytial virus (RSV) vaccine strain in which a polynucleotide encoding some domains of an F protein derived from a heterologous virus is inserted into the RSV genome.

The polynucleotide may be i) inserted instead of a polynucleotide encoding some domains of the RSV F protein, or ii) inserted between the RSV F protein and the polynucleotide encoding the G protein domain.

FIG. 2 shows substitution of the polynucleotide encoding some domains of the F protein of RSV by a polynucleotide encoding some domains of an F protein derived from a foreign virus.

FIG. 3 shows insertion of some domains of an F protein derived from a foreign virus into the polynucleotide encoding the F protein of RSV.

The heterologous virus may be substituted with a gene encoding the F protein derived from a heterologous virus belonging to the family *Paramyxoviridae* or the family *Pneumoviridae.* The heterologous virus belonging to the family *Paramyxoviridae* or the family *Pneumoviridae* may be one or more selected from a group consisting of the genera *Aquaparamyxovirus, Avulavirus, Ferlavirus, Henipavirus, Morbillivirus, Respirovirus, Rubulavirus, Metapneumovirus* and *Orthopneumovirus,* specifically one or more selected from a group consisting of measles virus and Newcastle disease virus. In particular, the measles virus and Newcastle disease virus may improve the stability and safety of the live vaccine virus strain.

Specifically, the RSV genome, which is the basis of the present disclosure, may be a wild-type human RSV A virus strain of SEQ ID NO 1. Although various RSV strains are available, including RSV A, RSV B, HRSV A, HRSV B, BRSV and avian RSV strains, the RSV backbone to which a foreign gene is inserted may be specifically an RSV A virus strain. When a foreign gene is introduced into the RSV A virus strain, the produced recombinant virus may have superior stability and safety and low mutation rate.

In an exemplary embodiment, the present disclosure provides a method for producing an attenuated recombinant RSV by expressing a first expression vector including a polynucleotide encoding a recombinant RSV antigenome and a second expression vector including a polynucleotide encoding one or more protein selected from a group consisting of N, P, L and M2-1 proteins together. As the first expression vector, pCC1, pBR322, pUC, pcDNA3.1, etc. may be used. However, when considering size, virulence, etc., pCC1 may be specifically used for the purpose of the present disclosure. As the second expression vector, a pCI neo vector may be used, although not being limited thereto.

RSV is a negative-sense RNA virus and requires a helper gene for viral production and gene synthesis through reverse genetics in vitro. The polynucleotide encoding the recombinant RSV antigenome or the polynucleotide encoding the N, P, L or M2-1 protein may be introduced into a cell by transfection, electroporation, mechanical insertion or transduction. Viral infection may occur in such cells as Vero cells, Hep 2 cells, MRC 5 cells, etc. Vero cells may be favorable for production of the recombinant RSV strain of the present disclosure.

As the method for introducing the polynucleotide encoding the recombinant RSV antigenome or the polynucleotide encoding the N, P, L or M2-1 protein, methods commonly used in the art may be used without special limitation.

In another exemplary embodiment, an RSV antigenome RNA may be synthesized in vitro and then transfected into a cell expressing the RSV protein.

The polynucleotide encoding a recombinant RSV antigenome may be included in a first expression vector, and the helper gene may be included in a vector (second expression vector) which is different from the first expression vector. Alternatively, they may be included in the same vector.

The polynucleotide encoding a recombinant RSV antigenome included in the first expression vector may include one or more polynucleotide selected from SEQ ID NOS 4-7, and the polynucleotide selected from SEQ ID NOS 4-7 may be a cDNA.

The first expression vector may include the polynucleotide encoding a recombinant RSV antigenome in a pCC1 plasmid. For the purpose of easier combination, the vector may be modified by inducing mutation, modifying the restriction enzyme site or inserting a polylinker including a modified restriction enzyme site.

The pCC1 plasmid of the present disclosure may stabilize the recombinant RSV antigenome.

Specifically, the first expression vector including the recombinant RSV antigenome may be an expression vector including the cDNA of a polynucleotide, including a T7 promoter, a hammerhead ribozyme, an RSV antigenome, a hepatitis delta virus ribozyme and a T7 terminator.

The RSV antigenome includes the F protein of measles virus, Newcastle disease virus and parainfluenza virus (PIV), and a polynucleotide encoding the transmembrane (TM) and cytoplasmic tail (CT) domains of the F protein may be one derived from RSV or one derived from the above-described viruses. In an exemplary embodiment, the present disclosure provides an attenuated RSV vaccine strain produced by the method described above.

In an exemplary embodiment, the present disclosure provides an RSV live vaccine including the RSV strain of the present disclosure and a pharmaceutically acceptable carrier.

The recombinant RSV vaccine strain produced according to an exemplary embodiment of the present disclosure may be used as a vaccine as it is, after being freeze-dried or after being mixed in a liquid.

The vaccine composition of the present disclosure contains any pharmaceutical substance which does not cause an immune response harmful to a vertebrate receiving the composition on its own, and contains a pharmaceutically acceptable carrier including any appropriate diluent or excipient that can be administered together with the recombinant RSV vaccine strain without unreasonable toxicity. The term "pharmaceutically acceptable" means listed in the United States Pharmacopeia, European Pharmacopeia or other generally recognized pharmacopeia for use in vertebrates, more specifically in human. The RSV F antigen of the present disclosure is administered with an effective amount (defined above) sufficient for stimulating an immune response in response to one or more strain of RSV. The composition may be used as a vaccine and/or immunogenic composition for inducing a protective immune response in vertebrates.

In a non-limiting exemplary embodiment, the concentration of the recombinant RSV antigen included in the vaccine is at least about 10 µg/mL, about 20 µg/mL, about 30 µg/mL, about 40 µg/mL, about 50 µg/mL, about 60 µg/mL, about 100 µg/mL, about 200 µg/mL or about 500 µg/mL. In a specific exemplary embodiment, the concentration of the immunogen is from about 10 µg/mL to about 1 mg/mL, from about 20 µg/mL to about 500 µg/mL, from about 30 µg/mL to about 100 µg/mL, or from about 30 µg/mL to about 50 µg/mL. In another exemplary embodiment, the concentration of the immunogen may be 10-200 µg/mL.

The vaccine or immunogenic composition of the present disclosure may be administered to an animal to induce an immune response against RSV. In an exemplary embodiment, the animal is human. Generally, the administration dosage may be may be adjusted on the basis of, for example, age, physical condition, body weight, diet, administration time and other clinical factors. Accordingly, the present disclosure includes a method for preparing a vaccine or immunogenic composition inducing substantial immunity against infection of a subject or at least one symptom thereof, which includes a step of adding an effective amount of an immunogen in the composition. Although it is preferred that the substantial immunity is induced by a single administration dosage, an additional administration dosage may be administered through the same or different route to achieve desired effect. For newborns and infants, a number of administrations may be necessary, for example, to induce a sufficient level of immunity. The administration may be continued over the infancy if it is necessary to provide a sufficient level of protection against infection. In an exemplary embodiment, a method for inducing substantial immunity against viral infection or at least one symptom thereof in a subject includes a step of administering an effective amount of the RSV recombinant F protein, a fragment thereof or an aggregate thereof at least once. The vaccine and/or immunogen composition may be administered by parenteral (e.g., endothelial, intramuscular, intravenous or subcutaneous), epidural or mucosal (e.g., nasal, oral, pulmonary or suppository) administration, although not being limited thereto. In a specific exemplary embodiment, the composition is administered intramuscularly, intravenously, subcutaneously, orally or endothelially. The composition may be administered via any convenient route, for example, by infusion or bolus injection or absorption through the epithelium or mucous membrane (e.g., oral, colonic, conjunctival, nasopharyngeal, oropharyngeal, vaginal, urinary tract, bladder or intestinal mucosa) and may be administered together with another biologically active substance. The administration may be systemic or topical. The preventive vaccine composition may be administered via subcutaneous or intramuscular injection using a needle and a syringe or may be administered systemically using a needle-free injection device. Optionally, the vaccine composition may be administered nasally into the upper airway in the form of droplets or large-particle aerosols (larger than about 10 µm) through spraying. Whereas some of the above-mentioned pathways cause immune response at random sites, the nasal administration provides enhanced effect of inducing mucosal immunity at the virus-infected site. In another exemplary embodiment, the vaccine and/or immunogenic composition may be administered to target a mucosal tissue without inducing an immune response at the immunization site. The site of administration is not limited.

In an exemplary embodiment, the present disclosure provides a method for preventing respiratory syncytial virus (RSV) infection by administering the RSV vaccine strain of the present disclosure into the human body.

### Advantageous Effects

The present disclosure provides a live RSV vaccine strain with superior stability in which a heterogeneous F protein with high stability is substituted or inserted. It provides a new recombinant RSV strain which has an antibody titer comparable to that of wild-type RSV while solving the unsafety and instability of common live vaccines.

The present disclosure provides a live RSV vaccine strain with superior safety in which a heterogeneous F protein with high stability is substituted or inserted.

The present disclosure provides a new type of RSV vaccine capable of inducing a defense mechanism against RSV.

The present disclosure provides a new type of vaccine allowing effective operation of the immune system by inhibiting the immune escape mechanism through mutation of the G protein of RSV.

The present disclosure allows production of a bivalent vaccine by inserting or substituting a heterogeneous F protein.

The present disclosure provides a new recombinant RSV.

### DESCRIPTION OF DRAWINGS

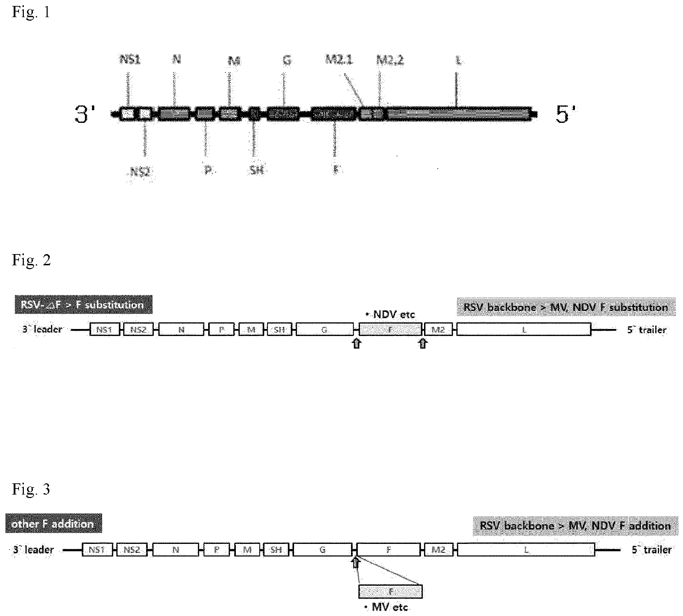

FIG. 1 is a schematic diagram of the RSV genome.
FIG. 2 schematically shows the cleavage of the F protein region of the RSV genome (arrows indicate the cleavage sites) and the substitution with an F protein of a heterogeneous virus (e.g., NDV).
FIG. 3 schematically shows the addition of an F protein of a heterogeneous virus (e.g., MV) between the G protein and the F protein of the RSV genome.

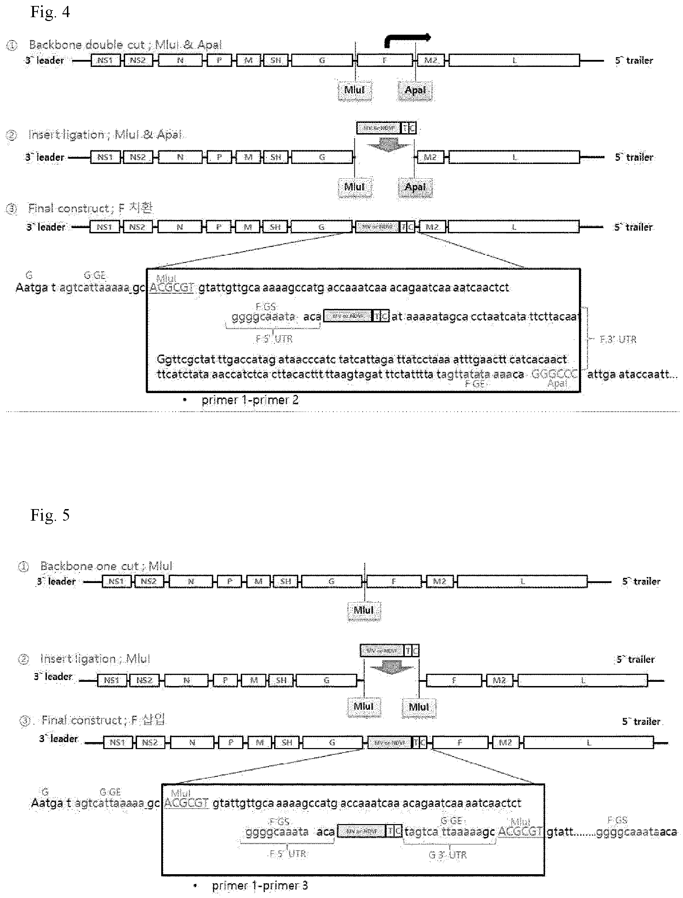

FIG. 4 shows the cleavage of the MluI and ApaI sites from the backbone of SEQ ID NO 3 and insertion of an F protein derived from a foreign virus. In the final construct ③, G GE indicates the gene end region of the G protein, F GS indicates the gene starting region of the F protein, and F GE indicates the gene end region of the F protein. The F protein of MV or NDV is inserted, but the T and C domains of the F protein are those of RSV.
FIG. 5 shows the cleavage of the MluI and MluI sites from the backbone of SEQ ID NO 3 and insertion of an F protein derived from a foreign virus. The MluI and MluI cleavage sites are located between the G protein and the F protein of RSV. In the final construct ③, G GE indicates the gene end region of the G protein, F GS indicates the gene starting region of the F protein, and F GE indicates the gene end region of the F protein. The F protein of MV or NDVF is inserted, but the T and C domains of the F protein are those of RSV.

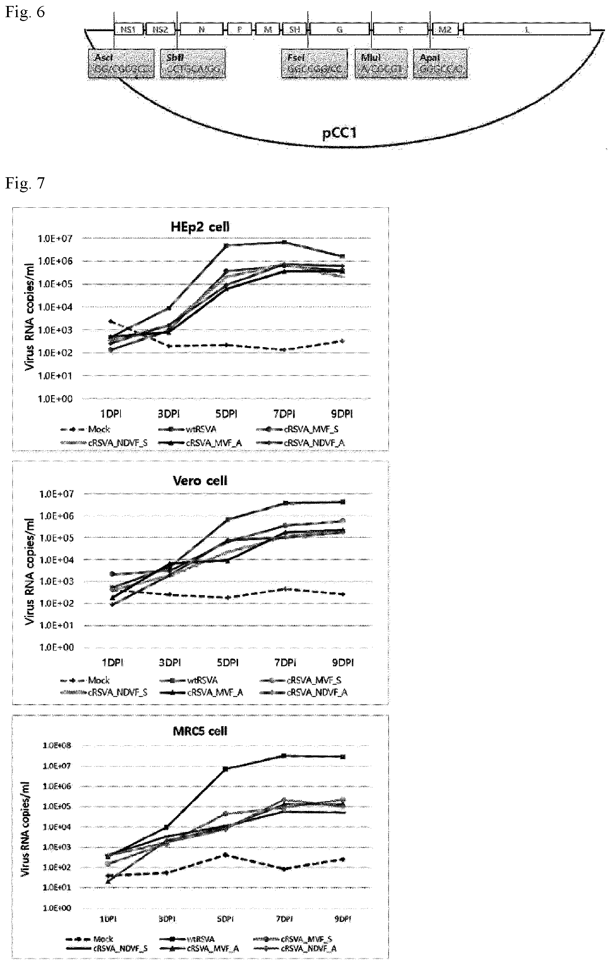

FIG. 6 schematically shows the cloning of the cDNA of the recombinant RSV of the present disclosure virus into a vector.
FIG. 7 shows the attenuation of recombinant RSV strains for different cells (HEp2 cells, Vero cells and MRC5 cells) through comparison of proliferation rate and proliferation titer. The proliferation rate and titer were decreased for the vaccine strains as compared to the wild-type RSV.

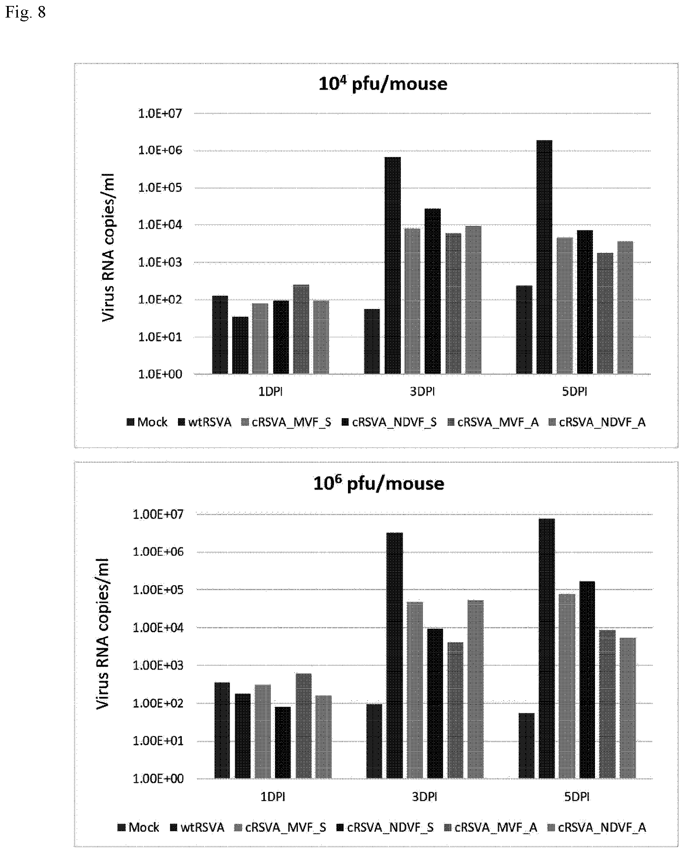

FIG. 8 shows the attenuation of recombinant RSV strains in mouse through comparison of proliferation rate and proliferation titer. The proliferation rate and titer were decreased for the vaccine strains as compared to the wild-type RSV.

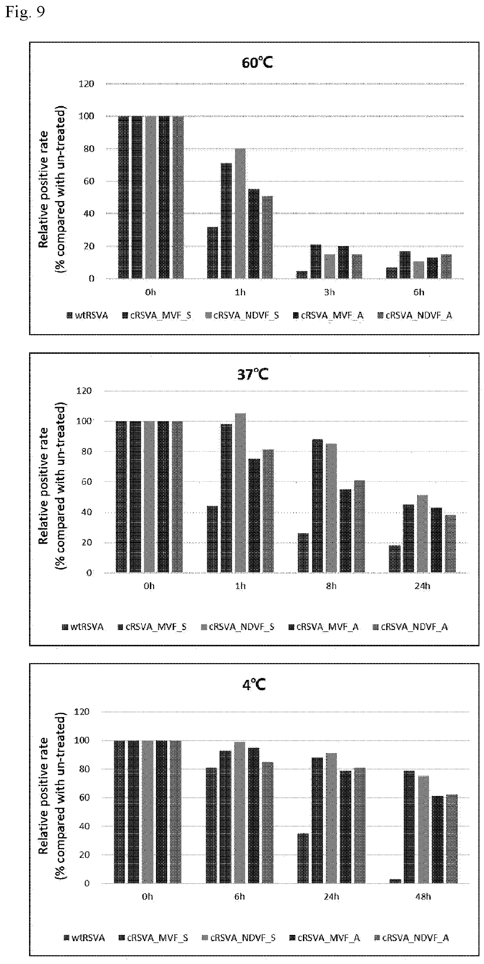

FIG. 9 shows the stability of recombinant RSV strains depending on temperature. The recombinant RSV strains showed improved thermal stability as compared to the wild-type RSV at all the temperatures of 4 °C, 37 °C and 60 °C.

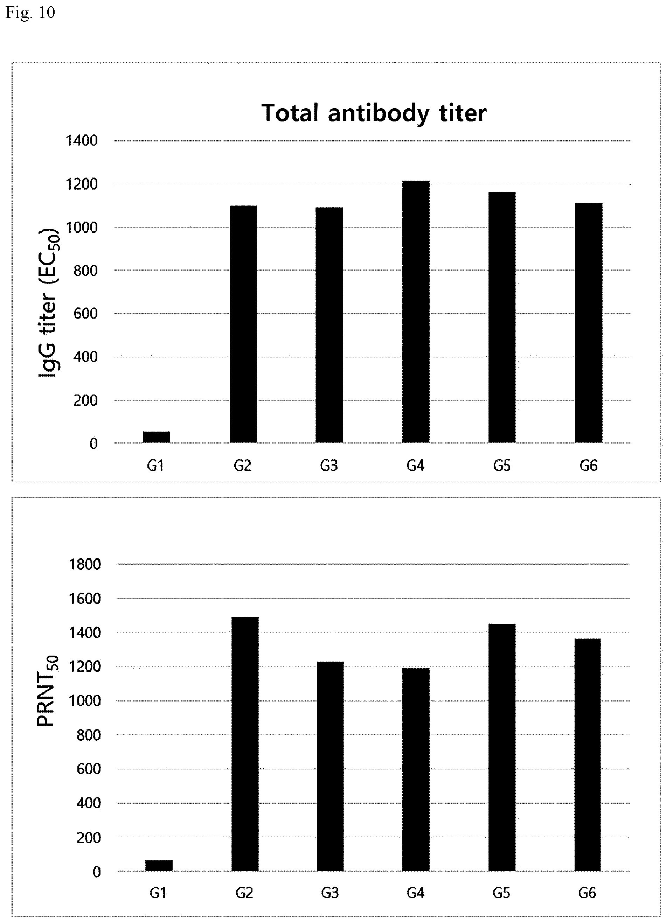

FIG. 10 shows a result of investigating total antibody titer and neutralizing antibody titer in a mouse immunity test of recombinant RSV. Sufficient antigen specificity and neutralizing antibodies were induced in all groups except Mock. However, the formalin-inactivated wild-type virus G7 showed relatively very low total antibody titer and neutralizing antibody titer. The RSV backbone was denoted as wtRSVA_TH10654, RSV backbone-MV F substitution as cRSVA_MVF_S, RSV backbone-NDV F substitution as cRSVA_NDVF_S, RSV backbone-MV F insertion as cRSVA_MVF_A, and RSV backbone-NDV F insertion as cRSVA NDVFA.
   In FIG. 10, Mock was denoted as G1, WtRSVA as G2, cRSVA_MVF_S as G3, cRSVA_NDVF_S as G4, cRSVA MVFA as G5, cRSVA NDVFA as G6, and FIwtRSVA as G7.

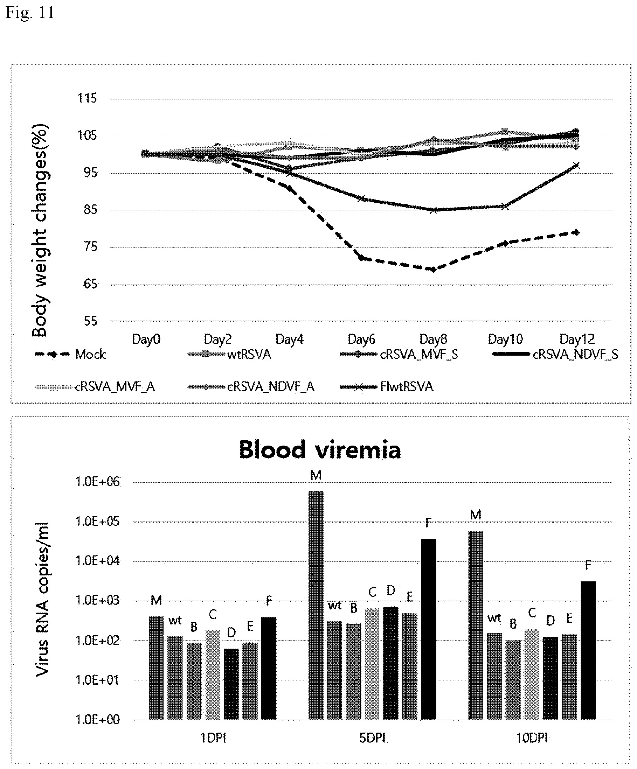

FIG. 11 shows a result of measuring defensive power after immunization with recombinant RSV by challenging with wild-type RSV. Sufficient protective immunity was induced in all groups except Mock. However, the formalin-inactivated wild-type virus G7 exhibited slightly insufficient protective immunity.

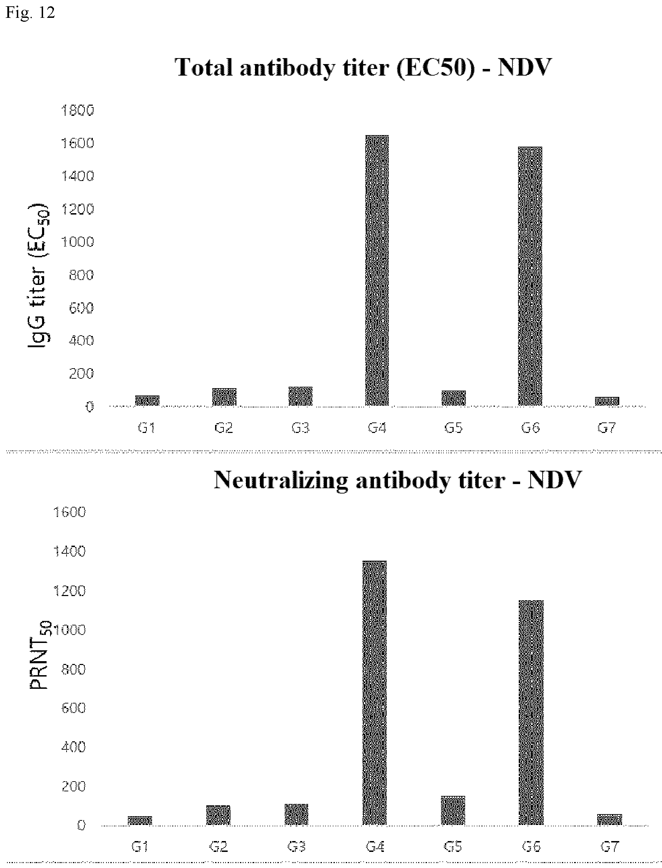

FIG. 12 shows a test result of immunogenicity and neutralizing antibody titer for NDV.

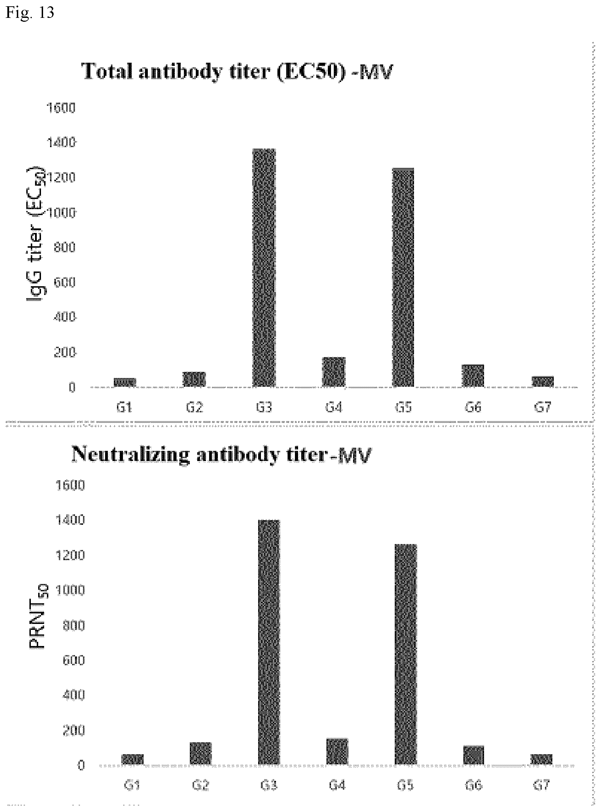

FIG. 13 shows a test result of immunogenicity and neutralizing antibody titer for MV immunogenic.

In FIGS. 12 and 13, Mock was denoted as G1, WtRSVA as G2, cRSVA_MVF_S as G3, cRSVA_NDVF_S as G4, cRSVA MVFA as G5, cRSVA NDVFA as G6, and FIwtRSVA as G7.

### MODE FOR DISCLOSURE

Hereinafter, the present disclosure is described in detail through examples, etc. to help understanding. However, the examples according to the present disclosure may be changed into various other forms and it should not be interpreted that the scope of the present disclosure is limited by the following examples. The examples of the present disclosure are provided such that the present disclosure is more completely described to those having average knowledge in the art.

### 1. Preparation of wild-type RSV A virus strain

A wild-type RSV A virus strain having the following information was prepared described below.
- Definition: human respiratory syncytial virus strain RSVA / TH_10654 / complete genome
- Accession No.: KU950464.1
- Length: 15232 bp
- Host: *Homo sapiens* / female / 12 weeks
- Collection date: 19-Feb-2014
- Country: USA
- Subtype: RSV A

The strain is represented by SEQ ID NO 1.

### 2. Preparation of F protein donor virus

Measles virus (MV) and Newcastle disease virus (NDV) were selected as F protein donors.

### 3. Preparation of cDNA encoding RSV antigenome

A polynucleotide encoding the F protein of the RSV antigenome was constructed by inserting or substituting a polynucleotide encoding an F protein derived from a heterologous virus to backbone construct A described below.

### (1) Designing of backbone construct A (SEQ ID NO 3)

The gene sequence was T7 promoter - hammerhead ribozyme - RSV antigenome - hepatitis delta virus ribozyme - T7 terminator.

A T7 promoter sequence (TAATACGACTCACTATAGG) was inserted to the 5'-end. After the T7 promoter sequence, a hammerhead ribozyme sequence (T TTTTTCGCGT CTGATGAGGC CGTTAGGCCG AAACTCCTCT CCGGAGTC) was inserted. After the hammerhead ribozyme sequence, a wild-type RSV antigenome sequence was inserted and the following mutation was induced.

An AscI restriction enzyme sequence was produced by inserting GCGCGCC between 77 nt and 78 nt on the basis of the antigenome sequence of wild-type RSV (Although the AscI restriction enzyme sequence was GGCGCGCC <8 bp>, only GCGCGCC <7 bp> was inserted beceause the 77 nt sequence was G.)
1) CCTGCAGG (Sbfl restriction enzyme sequence) was inserted between 1079 nt and 1080 nt.
2) GGCCGGCC (FseI restriction enzyme sequence) was inserted between 4600 nt and 4601 nt.
3) The base ATG(M) at 4800 nt and 4802 nt was substituted with ATT(I).
4) ACGCGT (MluI restriction enzyme sequence) was inserted between 5639 nt and 5640 nt.
5) GGGCCC (ApaI restriction enzyme sequence) was inserted between 7595 nt and 7596 nt.
6) After the wild-type RSV antigenome sequence, a hepatitis delta virus ribozyme sequence
   (GGCCGGCATGGTCCCAGCCTCCTCGCTGGCGCCGGCTGGGCAACATGCTTCGGC ATGGCGAATGGGAC) was inserted.
7) After the hepatitis delta virus ribozyme sequence, a T7 terminator sequence (TAGCATAACCCCTTGGGGCCTCTAAACGGGTCTTGAGGGGTTTTTTG) was inserted.
8) The ACGCGAAAAAATGCGTACAAC sequence was inserted at the 5'-end, and the GTTTTTGACACTTTTTTTCTCGT sequence was inserted at the 3'-end. The ACGCGAAAAAATGCGTACAAC inserted at the 5'-end was denoted as a 3' leader, and the GTTTTTGACACTTTTTTTCTCGT inserted at the 3'-end was denoted as a 5' trailer.

The designed gene (SEQ ID NO 3) was synthesized in vitro and then cloned into a pCC1 vector.

A point mutation (M48I) of the G protein sequence was introduced.

### (2) Designing of Measles virus F-substituted construct B (SEQ ID NO 4)

On the basis of the backbone construct A, the F protein gene sequence was removed and a corresponding measles virus F gene sequence was inserted.

The antigenome data of the Edmonston strain (Moraten vaccine) (Accession No. AF266287.1), which is a measles virus strain, were used.

The F protein gene of the Edmonston strain antigenome corresponds to sequences 5449-7110. Among them, sequences 5449-6939 excluding the transmembrane region and cytoplasmic tail region sequences were used. As the transmembrane region and cytoplasmic tail region sequences, the sequences of the wild-type human respiratory syncytial virus strain RSVA / TH_10654 were used. As 5' UTR and 3' UTR, the UTR sequences of the F protein gene of the wild-type human respiratory syncytial virus strain RSVA / TH_10654 were used.

After cleaving the backbone construct A with MluI and ApaI restriction enzymes, the designed measles virus F gene was inserted.

### (3) Designing of Newcastle disease virus F-substituted construct C (SEQ ID NO 5)

After removing the F protein gene sequence from the backbone construct A, the Newcastle disease virus F gene sequence corresponding thereto was inserted.

The antigenome data of the 1-2 strain (Accession No. AY935499), which is a Newcastle disease virus, were used.

The F protein gene of the 1-2 strain genome corresponds to sequences 4544-6205 sequence. Among them, sequences 4544-6043 excluding the transmembrane region and cytoplasmic tail region sequences were used. As the transmembrane region and cytoplasmic tail region sequences, the sequences of the wild-type human respiratory syncytial virus strain RSVA / TH_10654 were used. As 5' UTR and 3' UTR, the UTR sequences of the F protein gene of the wild-type human respiratory syncytial virus strain RSVA / TH_10654 were used.

After cleaving the backbone construct A with MluI and ApaI restriction enzymes, the Newcastle measles virus F gene was inserted.

As seen from FIG. 4, the constructs B and C recognize the restriction enzymes MluI/ApaI and amplify the inserts using primers 1 and 2. The primer 1 (forward) was 5' ATAT ACGCGT gtattgttgcaaaaagccatg, and the primer 2 (reverse) was 5' ATAT GGGCCC tgttttatataactataaaatagaa.

### (4) Designing of Measles virus F-inserted construct D (SEQ ID NO 6)

The measles virus F gene sequence was inserted between the G protein and F protein genes of the backbone construct A.

The antigenome data of the Edmonston strain (Moraten vaccine) (Accession No. AF266287.1), which is a measles virus strain, were used.

The F protein gene of the Edmonston strain antigenome corresponds to sequences 5449-7110. Among them, sequences 5449-6939 excluding the transmembrane region and cytoplasmic tail region sequences were used. As the transmembrane region and cytoplasmic tail region sequences, the sequences of the wild-type human respiratory syncytial virus strain RSVA / TH_10654 were used. As 5' UTR, the 5' UTR sequence of the F protein gene of the wild-type human respiratory syncytial virus strain RSVA / TH_10654 was used. As 3' UTR, the 3' UTR sequence of the G protein gene of the wild-type human respiratory syncytial virus strain RSVA / TH_10654 was used.

After cleaving the backbone construct A with the MluI restriction enzyme, the designed measles virus F gene was inserted.

### (5) Designing of Newcastle disease virus F-inserted construct E (SEQ ID NO 7)

The Newcastle disease virus F gene sequence was inserted between the G protein and F protein genes of the backbone construct A.

The gene data of the 1-2 strain (Accession No. AY935499.1), which is a Newcastle disease virus strain, were used.

The F protein gene of the 1-2 strain genome corresponds to sequences 4544-6205 sequence. Among them, sequences 4544-6043 excluding the transmembrane region and cytoplasmic tail region sequences were used. As the transmembrane region and cytoplasmic tail region sequences, the sequences of the wild-type human respiratory syncytial virus strain RSVA / TH_10654 were used. As 5' UTR, the 5' UTR sequence of the F protein gene of the wild-type human respiratory syncytial virus strain RSVA / TH_10654 was used. As 3' UTR, the 3' UTR sequence of the G protein gene of the wild-type human respiratory syncytial virus strain RSVA / TH_10654 was used.

After cleaving the backbone construct A with the MluI restriction enzyme, the designed Newcastle disease virus F gene was inserted.

As seen from FIG. 5, the heterologous virus gene was inserted to the region recognized by the restriction enzyme MluI and the insert was amplified using primers 1 and 3. The primer 1 (forward) was 5' ATAT ACGCGT gtattgttgcaaaaagccatg, and the primer 3 (reverse) was 5' ATAT ACGCGT gctttttaatgacta tcagttactaaatgcaatat.

### 4. Designing of four helper genes

RSV is a negative-sense RNA virus. While producing the virus in vitro through reverse genetics, helper genes (genes encoding N, P, L and M2-1 proteins of RSV) necessary for gene synthesis were added together.
(1) The N protein gene clones the sequences 1119-2294 of the wild-type human respiratory syncytial virus strain RSVA / TH_10654 antigenome into a pCI neo vector using the restriction enzymes XhoI and MluI.
(2) The P protein gene clones the sequences 2326-3051 of the wild-type human respiratory syncytial virus strain RSVA / TH_10654 antigenome into a pCI neo vector using the restriction enzymes XhoI and MluI.
(3) The M2-1 protein gene clones the sequences 7647-8231 of the wild-type human respiratory syncytial virus strain RSVA / TH_10654 antigenome into a pCI neo vector using the restriction enzymes XhoI and MluI.
(4) The L protein gene clones the sequences 8539-15036 of the wild-type human respiratory syncytial virus strain RSVA / TH_10654 antigenome into a pCI neo vector using the restriction enzymes XhoI and MluI.

The N protein gene is represented by SEQ ID NO 8, the P protein gene by SEQ ID NO 9, the M2-1 protein gene by SEQ ID NO 10, and the L protein gene by SEQ ID NO 11.

### 5. Virus rescue

BHK cells were prepared on a 6-well plate at a concentration of 4×10⁵ cell/well.

Next day, after mixing six plasmids, i.e., 2 µg of a T7 polymerase expression vector, 2 µg of a full-length RSV antigenome vector and 1 µg of four helper gene vectors (N, P, M2-1 and L), with 10 µL of Lipofectamine 3000, and reacting for 10 minutes, the mixture was transfected to BHK cells. The mixture was added dropwise for uniform application to the cells and then shaken 2-3 times to ensure mixing.

After culturing the cells for 5 days in a CO₂ incubator at 37 °C, the culture was recovered and then transfected (1 mL each) to Vero cells (3×10⁵/well) on a freshly prepared 6-well plate. A virus solution was obtained by culturing again for 5 days. One recombinant wild-type RSV and four RSV vaccine strains were produced in this way.

This technology is an experimental method commonly used by researchers for RSV rescue and there is no special technical limitation. In this experiment, the literatures "BAC-Based Recovery of Recombinant Respiratory Syncytial Virus (RSV); Reverse Genetics of RNA Viruses, pp. 111-124" and "RNA Virus Reverse Genetics and Vaccine Design; Viruses, 2014, 6, 2531-2550" were referred to. After recovering the culture, the virus was detected through IFA, RT-PCR and gene sequencing.
① RSV backbone strain: wtRSVA_TH10654 (wild-type control)
② RSV backbone strain- MV F substitution: cRSVA_MVF_S (mutant virus)
③ RSV backbone strain - NDV F substitution: cRSVA_NDVF_S (mutant virus)
④ RSV backbone strain- MV F insertion: cRSVA_MVF_A (mutant virus)
⑤ RSV backbone strain- NDV F insertion: cRSVA_NDVF_A (mutant virus)

### 6. In vitro attenuation test

After infecting Vero cells, HEp2 cells and MRC-5 cells with 0.1 MOI of the five strains, the cells were cultured for 9 days. The virus culture was collected with 1-day intervals and viral titer was analyzed by q-PCR.

As shown in FIG. 7, it was confirmed that the mutant viral vaccine strains were attenuated, with decreased proliferation rate and proliferation titer as compared to the wild-type virus. Through this, it can be seen that the recombinant viruses can be used as live vaccine strains. In the figure, DPI stands for days post-infection.

### 7. In vivo attenuation test

The five strains of viruses were administered to BALB/c mouse at concentrations of 10¹-10⁷ pfu/mouse via the IN route. Viremia was detected by q-PCR in the blood on days 1-7, which confirms attenuation.

The result is shown in FIG. 8. As can be seen from FIG. 8, it was confirmed that the viruses were attenuated after being vaccinated in vivo as compared to the wild-type RSV A virus. Through this, it was confirmed that the recombinant RSVs can be used as new virus strains.

### 8. Virus stability test

The viability of the five strains of viruses with time was investigated at 60 °C, 37 °C and 4 °C. Live viral titer in the sample was analyzed through plaque assay using HEp2 cells. As a result, it was confirmed that the stability of the mutant viruses was improved over the wild type under all temperature conditions. The stability of isolated RSV is decreased greatly. However, as can be seen from FIG. 9, the recombinant RSVs exhibited superior stability as compared to the wild-type virus strain at high temperature, human body temperature and low temperature.

### 9. Immunogenicity test

Mouse IM was immunized twice with each of Mock, wtRSVA, cRSVA_MVF_S, cRSVA_NDVF_S, cRSVA_MVF_A and cRSVA_NDVF_A at a concentration of 1×10⁵ pfu/mouse. 2 weeks later, after isolating mouse serum, total antibody titer and neutralizing antibody titer were measured by ELISA and plaque reduction neutralization test. The result is shown in FIG. 10. It was confirmed that RSV antigen-specific IgG antibodies and viral infection-neutralizing antibodies were produced sufficiently in all groups except Mock. Mock was denoted as G1, WtRSVA as G2, cRSVA_MVF_S as G3, cRSVA_NDVF_S as G4, cRSVA_MVF_A as G5, cRSVA_NDVF_A as G6, and FIwtRSVA as G7. It can be seen that the recombinant RSV strains produced antibodies at similar levels to the wild-type RSV, and shoed much higher antibody titers as compared to the inactivated G7. From the result of FIG. 10, it was confirmed that the recombinant RSV strains can be used as excellent virus strains because they exhibit superior stability while producing antibodies at similar levels to the wild-type virus.

### 10. Immunization test

Mouse IM was immunized twice with each of the five strains of viruses at a concentration of 10⁵ pfu/mouse with a 2-week interval. 2 weeks after the immunization, the wild-type RSV was challenged into the nasal cavity of the mouse at a concentration of 2×10⁶ pfu/mouse. On days 1-12, viremia in blood was detected by q-PCR and the change in body weight was measured. The result is shown in FIG. 11. It was confirmed that viral infection was inhibited effectively in all the immunized groups. In particular, the level of inhibition of infection was similar to that of the wild-type virus strain, and the viral infection in blood was remarkably lower as compared to the inactivated virus strain.

### 11. Immunogenicity test for MV and NDV

Total antibody titer and neutralizing antibody titer for NDV and MV antigens were measured in a manner similar to 9 by using the same serum. For measurement of total antibody titer, ELISA was conducted after coating with each of MV and DNV antigens. For measurement of neutralizing antibody titer, the concentrations that inhibits MV and NDV infections were measured by plaque inhibition assay. As shown in FIG. 12 and FIG. 13, enough antigen-specific antibody titer and virus neutralizing antibody titer were attained only for the groups having the F antigens of MV and NDV. In contrast, the groups to which the heterogeneous F was not inserted did not produce the antibodies for each antigen and neutralizing antibodies were not induced either.

## Claims

1. A recombinant respiratory syncytial virus (RSV) in which genes encoding the envelope proteins of RSV are rearranged, wherein
a gene encoding the F protein derived from a heterologous virus belonging to the family *Paramyxoviridae* or the family *Pneumoviridae* is inserted between the genes encoding the glycoprotein (G protein) and the fusion protein (F protein) of RSV, or
a gene encoding the F protein of RSV is substituted with a gene encoding the F protein derived from a heterologous virus belonging to the family *Paramyxoviridae* or the family *Pneumoviridae.*

2. The recombinant respiratory syncytial virus (RSV) according to claim 1, wherein the heterologous virus belonging to the family *Paramyxoviridae* or the family *Pneumoviridae* is one or more virus selected from a group consisting of the genera *Aquaparamyxovirus, Avulavirus, Ferlavirus, Henipavirus, Morbillivirus, Respirovirus, Rubulavirus, Metapneumovirus* and *Orthopneumovirus.*

3. The recombinant respiratory syncytial virus (RSV) according to claim 1, wherein the heterologous virus is one or more virus selected from a group consisting of measles virus and Newcastle disease virus.

4. The recombinant respiratory syncytial virus (RSV) according to claim 1, wherein the recombinant respiratory syncytial virus (RSV) is one in which the genes encoding the envelope proteins of the wild-type human RSV A virus strain of SEQ ID NO 1 are rearranged.

5. A method for producing an attenuated recombinant RSV by expressing a first expression vector comprising a polynucleotide encoding a recombinant RSV antigenome and a second expression vector comprising a polynucleotide encoding one or more protein selected from a group consisting of N, P, L and M2-1 proteins, wherein the polynucleotide encoding a recombinant RSV antigenome comprises a polynucleotide encoding a domain of a protein derived from a heterogeneous virus wholly or partially.

6. The method according to claim 5, wherein the polynucleotide encoding a recombinant RSV antigenome comprised in the first expression vector is any one selected from SEQ ID NOS 4-7.

7. The method according to claim 6, wherein the polynucleotide selected from SEQ ID NOS 4-7 is a cDNA.

8. The method according to claim 5, wherein the first expression vector comprises the polynucleotide encoding a recombinant RSV antigenome in a pCC1 plasmid.

9. The method according to claim 5, wherein the protein derived from a heterogeneous virus of the polynucleotide encoding a recombinant RSV antigenome is an F protein.

10. A recombinant RSV expression vector,
comprising the genes encoding the glycoprotein (G protein) and the fusion protein (F protein) of RSV, wherein a gene encoding the F protein derived from a heterologous virus belonging to the family *Paramyxoviridae* or the family *Pneumoviridae* is inserted between the genes encoding the G protein and the F protein, or
comprising a recombinant RSV gene in which a gene encoding the F protein derived from a heterologous virus belonging to the family *Paramyxoviridae* or the family *Pneumoviridae* is substituted instead of a gene encoding the fusion protein (F protein) of RSV.

11. The recombinant RSV expression vector according to claim 10, wherein the recombinant RSV gene is any one selected from SEQ ID NOS 4-7.

12. The recombinant RSV expression vector according to claim 10, wherein the recombinant RSV expression vector comprises a T7 promoter, a hammerhead ribozyme, a recombinant RSV gene, a hepatitis delta virus ribozyme and a T7 terminator in sequence.

13. The recombinant RSV expression vector according to claim 10, wherein the heterologous virus belonging to the family *Paramyxoviridae* or the family *Pneumoviridae* is one or more selected from a group consisting of measles virus and Newcastle disease virus.

14. An attenuated RSV live vaccine composition, comprising:
the recombinant respiratory syncytial virus (RSV) according to any of claims 1 to 4; and
a pharmaceutically acceptable carrier.

15. A bivalent vaccine composition for preventing infection of
respiratory syncytial virus (RSV); and one of measles virus (MV) and Newcastle disease virus (NDV) comprising:
the recombinant respiratory syncytial virus (RSV) according to any of claims 1 to 4; and
a pharmaceutically acceptable carrier.

16. A method for preventing respiratory syncytial virus (RSV) infection by administering the RSV vaccine composition according to claim 14 into the human body.


<110>     SK bioscience Co., Ltd.

<120>     Recombinant RSV live vaccine strain and the preparing method
          thereof

<130>     PCT19-209

<150>     KR 10-2018-0122162
<151>     2018-10-12

<160>     11

<170>     KoPatentIn 3.0

<210>     1
<211>     15232
<212>     DNA
<213>     respiratory syncytial virus

<400>     1
aaacttgcgt aaaccaaaaa aatggggcaa ataagaattt gataagtacc acttaaattt        60

aactcctttg gttagagatg ggcagcaact cattgagtat gataaaagtt agattgcaaa       120

atctgtttga caatgatgaa gtagcattgt taaaaataac atgctatact gacaaattaa       180

tacagttaac taatgctttg gctaaggcag ttatacatac aatcaaattg aatggcattg       240

tatttgtgca tgttattaca agtagtgata tttgccctaa taataatatt gtagtgaaat       300

ccaatttcac aacaatgcca gtattacaaa atggaggtta tatatgggaa atgatggaat       360

taacacactg ctctcaacct aatggcctaa tagatgacaa ttgtgaaatt aaattctcca       420

aaaaactaag tgattcaaca atgaccaatt atatgaatca attatctgaa ttacttggat       480

ttgacctcaa tccataaatc ataataaata tcaactagca aatcaatgtc actaacacca       540

ttagttaata taaaacttga cagaagataa aaatggggca aataaatcaa ttcagccgac       600

ccaaccatgg acacaacaca caatgatacc acaccacaaa gactgatgat cacagacatg       660

aggccattat cgcttgagac tataataaca tctctaacca gagatatcat aacacataaa       720

tttatatact tgataaatca tgaatgcata gtaagaaaac ttgatgaaag acaggccaca       780

tttacatttc tggtcaacta tgaaatgaaa ctattgcaca aagtgggaag cactaaatat       840

aaaaaatata ctgaatacaa cacaaaatat ggcactttcc ctatgccaat atttatcaat       900

catgatgggt tcttagaatg cattggcatt aagcctacca agcacacacc cataatatac       960

aagtatgatc tcaatccatg aatatcaaac caagattcaa acaatccgaa ataacaactt      1020

tatgcataat cacactccat agtccaaatg gagcctgaaa attatagtta tttaaaatta      1080

aggagagaca taagatgaaa gatggggcaa atacaaaaat ggctcttagc aaagtcaagt      1140

tgaatgatac actcaacaaa gatcaacttc tatcatccag caaatatacc atccaacgga      1200

gcacaggaga cagcattgac actcctaatt atgatgtgca gaaacacatt aataagttat      1260

gtggcatgtt attaatcaca gaagatgcta atcataaatt cactgggtta ataggtatgt      1320

tatatgctat gtctagatta ggaagagaag acaccataaa aatactcaaa gatgcgggat 1380

atcatgttaa ggcaaatgga gtggatgtaa caacacatcg tcaagacatt aatgggaaag 1440

aaatgaaatt tgaagtgtta acattagcaa gcttaacaac tgaaattcaa atcaacattg 1500

agatagaatc tagaaaatcc tacaaaaaaa tgctaaaaga aatgggagag gtggctccag 1560

aatacaggca tgactctcct gattgtggga tgataatatt atgtatagcg gcattagtaa 1620

taaccaaatt agcagcagga gatagatcag gtcttacagc tgtgattagg agagctaata 1680

atgtcctaaa aaatgaaatg aaacgttata aaggtttatt acccaaggat atagccaaca 1740

gcttctatga agtgtttgaa aaatatcctc actttataga tgtttttgtt cattttggta 1800

tagcacaatc ttctaccaga ggtggcagta gagttgaagg gatttttgca ggattgttta 1860

tgaatgccta tggtgcaggg caagtgatgt tacggtgggg ggtcttagca aaatcagtta 1920

aaaacattat gttaggacac gctagtgtac aagcagaaat ggaacaagtt gtggaggtgt 1980

atgagtatgc tcagaaattg ggtggagaag caggattcta ccatatattg aacaacccaa 2040

aagcatcact attatctttg actcaatttc ctcacttctc tagtgtagta ttgggcaatg 2100

ctgctggcct aggcataatg ggagaataca gaggtacacc aaggaatcaa gatttatatg 2160

atgctgcaaa agcatatgct gaacaactca aagaaaatgg tgtgattaac tacagtgtat 2220

tagatttgac agcagaagaa ctagaggcta tcaaacatca gcttaatcca aaagataatg 2280

atgtagagct ttgagttaat aaaaaagtgg ggcaaataaa tcatcatgga aaagtttgct 2340

cctgaattcc atggagaaga tgcaaacaac agagccacca aattcctaga atcaataaag 2400

ggcaaattca catcacccaa agatcccaag aaaaaagata gtatcatatc tgtcaactca 2460

atagatatag aagtaaccaa agaaagccct ataacatcaa attcaaccat tataaaccca 2520

ataaatgaga cagatgatac tgtagggaac aagcccaatt atcaaagaaa gcctctagta 2580

agtttcaaag aagaccctac gccaagtgat aatccttttt caaaactata caaagaaacc 2640

atagaaacat ttgataacaa tgaagaagaa tctagctatt catatgaaga aataaatgat 2700

cagacaaacg ataatataac agcaagatta gataggattg atgagaaatt aagtgaaata 2760

ctaggaatgc ttcacacatt agtagtagcg agtgcaggac ccacatctgc tcgggatggt 2820

ataagagatg ccatggttgg tttaagagaa gaaatgatag aaaaaatcag aactgaagca 2880

ttaatgacca atgacagact agaagctatg gcaagactca ggaatgaaga aagtgaaaag 2940

atggcaaaag acacatcaga tgaagtgtct ctcaatccaa catcagagaa actgaacaac 3000

ctgttggaag ggaatgatag tgacaatgat ctatcacttg aagatttctg attagctacc 3060

aaactgtaca tcaaacacaa acaccaatag aaaaccaaca aacaaaccaa ctcacccatc 3120

caaccaaaca tctatctgct gattagccaa ccagccaaaa aacaaccagc caatctaaaa 3180

ctagccaccc ggaaaaaatc gatactatag ttacaaaaaa agatggggca aatatggaaa 3240

catacgtgaa taaacttcac gagggctcca catacacagc tgctgttcaa tacaatgtcc 3300

tagaaaaaga cgatgatcct gcatcactta caatatgggt gcccatgttc caatcatcca 3360

tgccagcaga tctactcata aaagaactag ccaatgtcaa tatactagtg aaacaaatat 3420

ccacacccaa gggaccctca ttaagagtca tgataaactc aagaagtgca gtgctagcac 3480

aaatgcccag caaatttacc atatgtgcca atgtgtcctt ggatgaaaga agcaagctgg 3540

catatgatgt aaccacaccc tgtgaaatta aggcatgcag tctaacatgc ctaaaatcaa 3600

aaaatatgtt aactacagtt aaagatctca ctatgaaaac actcaaccca acacatgaca 3660

tcattgcttt atgtgaattt gaaaatatag taacatcaaa aaaagtcata ataccaacat 3720

acctaagatc tatcagcgtc agaaataaag atctgaacac acttgaaaat ataacaacca 3780

ctgaattcaa aaatgccatt acaaatgcaa aaatcatccc ttactcagga ttattgttag 3840

tcatcacagt gactgacaac aaaggagcat tcaaatacat aaagccacaa agtcaattca 3900

tagtagatct tggagcttac ctagaaaaag aaagtatata ttatgttaca acaaattgga 3960

agcacacagc tacacgattt gcaatcaaac ccatggaaga ttaacctttt tcctctacat 4020

caatgagtag attcatacaa actttctaac tacattcttc acttcacaat cataatcacc 4080

aaccctctgt ggttcaatca atcaaacaaa actcatcaag agttccagat catcccaagt 4140

cattgttcat cagatccagt actcaaataa gttaataaaa aatccacatg gggcaaataa 4200

tcattgaggg aaatccaact aatcacaaca tctgtcaaca tagacaagtc aacacgctag 4260

ataaaatcaa ccaatggaaa atacatccat aactatagaa ttctcaagca aattctggcc 4320

ttactttaca ctaatacaca tgataacaac aataatctct ttgataatca taatctccat 4380

catgattgca atactaaaca aactctgcga atataatgta ttccataaca aaacctttga 4440

gctaccaaga gctcgagtca atacatagca ttcaccaatc tgatagctca aaacagtaac 4500

cttgcatttg taaatgaact accctcactt cttcacaaaa ccacatcaac atctcaccat 4560

gcaagccatc atctatacca taaagtagtt aattaaaaat agtcataaca atgaactagg 4620

atattaagac caaaaacaac gctggggcaa atgcaaacat gtccaaaacc gaggaccaac 4680

gcaccgccaa gacactagaa aggacctggg acacttttaa tcatctatta ttcatatcat 4740

cgtgcttata caagttaaat cttaaatcta tagcacaaat cacattatct attttggcaa 4800

tgataatctc aacctcactt ataattgcag ccatcatatt catagcctcg gcaaaccaca 4860

aagtcacact aacaactgca atcatacaag atgcaacgaa ccagatcaag aacacaaccc 4920

caacatacct cacccagaat ccccagcatg gaatcagctt ctccaatctg tccggaacta 4980

catcacaatc caccaccata ctagcttcaa caacaccaag tgctgattca accccacaat 5040

ccacaacagt caagatcaaa aacacaacaa caacccaaat attacctagc aaacccacca 5100

caaaacaacg ccaaaataaa ccacaaaaca aacccaacaa tgattttcac tttgaagtgt 5160

tcaattttgt accctgcagc atatgcagca acaatccaac ctgctgggcc atctgcaaga 5220

gaataccaaa caaaaaacct ggaaagaaaa ccaccaccaa gcccacaaaa aaaccaaccc 5280

tcaagacaac caaaaaagat cccaaatccc aaaccacaaa accaaaggaa gtactcacta 5340

ccaagcctac aggaaagcca accatcaaca ccactaaaac aaacatcaga actacactgc 5400

tcacctccaa caccaaagga aatccagaac acacaagtca agaggaaacc ctccactcaa 5460

ccacctccga aggctatcca agcccatcac aagtccacac aacatccggt caagaggaaa 5520

ccctccactc aaccacctcc gaaggctatc caagcccatc acaagtctac acaacatccg 5580

agtacctatc acaatctcta tcttcatcca acacaacaaa atgatagtca ttaaaaagcg 5640

tattgttgca aaaagccatg accaaatcaa acagaatcaa aatcaactct ggggcaaata 5700

acaatggagt tgccaatcct caaaacaaat gctattacca caatccttgc tgcagtcaca 5760

ctctgtttcg cttccagtca aaacatcact gaagaatttt atcaatcaac atgcagtgca 5820

gtcagcaaag gctatcttag tgctctaaga actggttggt atactagtgt tataactata 5880

gaattaagta atatcaagga aaataagtgt aatggtacag acgctaaggt aaaattaata 5940

aaacaagaat tagataaata taaaaatgct gtaacagaat tgcagttgct catgcaaagc 6000

acaccagcag ccaacaatcg agccagaaga gaactaccaa gatttatgaa ttatacactc 6060

aacaatacca aaaacaccaa tgtaacatta agtaagaaaa ggaaaagaag atttcttgga 6120

tttttgttag gtgttggatc tgcaatcgcc agtggcattg ccgtatccaa ggtcctgcac 6180

ctagaagggg aagtgaacaa aatcaaaagt gctctactat ccacaaacaa ggctgtagtc 6240

agcttatcta atggagtcag tgtcttaacc agcaaggtgt tagacctcaa aaactatata 6300

gataaacagt tgttacctat tgttaacaag caaagctgca gcatatcaaa cattgaaact 6360

gtgatagagt tccaacaaaa gaacaacaga ctactagaga ttaccagaga atttagtgtt 6420

aatgcaggtg taactactcc tgtaagcact tatatgttaa ctaatagtga gttattatca 6480

ttaatcaatg atatgcctat aacaaatgat cagaaaaagt taatgtccag caatgttcaa 6540

atagttagac agcaaagtta ctctatcatg tcaataataa aagaggaagt cttggcatat 6600

gtagtacaat taccactata tggtgtaata gatactcctt gttggaaact acacacatcc 6660

cctttatgta caaccaacac aaaggaagga tccaacatct gcttaacaag aaccgacaga 6720

ggatggtact gtgacaatgc aggatcagta tcctttttcc cacaagctga aacatgtaaa 6780

gttcaatcga atcgggtgtt ttgtgacaca atgaacagtt taacattacc aagtgaggta 6840

aatctctgca acattgacat attcaacccc aaatatgatt gcaaaattat gacttcaaaa 6900

acagatgtaa gcagctccgt tatcacatct ctaggagcca ttgtgtcatg ctatggcaaa 6960

accaaatgta cagcatccaa taaaaatcgt gggatcataa agacattctc taacgggtgt 7020

gattatgtat caaataaggg ggtggatact gtgtctgtag gtaatacatt atattatgta 7080

aataagcaag aaggcaaaag tctctatgta aaaggtgaac caataataaa tttctatgat 7140

ccattagtgt tcccctctga tgaatttgat gcatcaatat ctcaagtcaa tgagaaaatt 7200

aatcagagtc tagcatttat ccgtaaatca gatgaattat tacataatgt aaatgctggt 7260

aaatccacca caaatatcat gataactacc ataattatag taattatagt aatattgtta 7320

gcattaattg cagttggact gcttctatac tgcaaggcca gaagcacacc agtcacatta 7380

agtaaggatc aactgagtgg tataaataat attgcattta gtaactgaat aaaaatagca 7440

cctaatcata ttcttacaat ggttcgctat ttgaccatag ataacccatc tatcattaga 7500

ttatcctaaa atttgaactt catcacaact ttcatctata aaccatctca cttacacttt 7560

ttaagtagat ttctatttta tagttatata aaacaattga ataccaaatt aacttactat 7620

ttgtaaaaat gagaattggg gcaaatatgt cacgaaggaa tccttgcaaa ttcgaaattc 7680

gaggtcattg cttgaatggt aaaaggtgtc attttagtca taattatttt gaatggccac 7740

cccatgcact gcttgtaaga caaaacttta tgttaaacag aatacttaag tctatggata 7800

aaagcataga tactttgtca gaaataagtg gagctgcaga gttggacaga acagaagagt 7860

atgccctcgg tgtagttgga gtgctagaga gttatatagg atcaataaat aatataacta 7920

aacaatcagc atgtgttgcc atgagcaaac tccttactga actcaacagc gatgacatca 7980

aaaaactaag ggacaatgaa gagccaaact cacccaaagt aagagtgtac aatactgtca 8040

tatcatatat tgaaagcaac aggaagaaca ataaacaaac tatccatctg ttaaaaagat 8100

tgccagcaga cgtattgaag aaaaccatca aaaacacatt ggatatccac aagagcataa 8160

ccatcaataa cccaaaagaa tcaactgtta gtgatacgaa cgaccatgcc aaaaataatg 8220

atactacctg acaaatatcc ttgtagtata aattccatac taataacaag taattgtaga 8280

gtcactatgt ataatcaaaa aaacacacta tatatcaatc aaaacaacca aaatagccat 8340

atatacccac cggatcaacc attcaatgaa atccattgga cctctcaaga cttgattgat 8400

gcaactcaaa atttctacaa acatctaggt attactgatg atatatacac aatatatata 8460

ttagtgtcat aatactcaat cctaatactt accacatcat caaattatta actcaaacaa 8520

ttcaagctat gggacaaaat ggatcccatt attagtggaa attctgctaa tgtttatcta 8580

actgatagtt atttaaaagg tgttatttct ttctcagaat gtaacgcttt aggaagttac 8640

atattcaatg gtccttatct caaaaatgat tataccaact taattagtag acaaaatcca 8700

ttaatagaac acataaatct aaagaaacta aatataacac agtccttaat atctaagtat 8760

cataaaggtg aaataaaaat agaagaacct acttactttc agtcattact tatgacatac 8820

aagagtatga cctcttcaga acagactact actactaatt tacttaaaaa gataataaga 8880

agagctatag aaatcagtga tgtcaaagtc tatgctatat tgaataaact ggggctcaaa 8940

gaaaaagaca agattaaatc caataatgga caagatgaag acaactcagt cattactacc 9000

ataatcaaag atgatatact tttagctgtc aaggataatc aatctcatct taaagcagac 9060

aaaaatcaat ccacaaaaca aaaagataca atcaaaacaa cacttttgaa gaaattaatg 9120

tgttcgatgc aacatcctcc atcatggtta atacattggt ttaatttata cacaaaatta 9180

aacagcatat taacacaata tcgatctagt gaggtaaaaa accatggttt tatattgata 9240

gataatcata ctcttagtgg attccaattt attttgaatc aatatggttg tatagtttat 9300

cataaggaac tcaaaagaat tactgtgaca acttataatc aattcttgac atggaaagat 9360

attagcctta gtagattaaa tgtttgtttg attacatgga ttagtaactg tttgaacaca 9420

ttaaacaaaa gcttaggctt aagatgtgga ttcaataatg ttatcttgac acaattattc 9480

ctttatggag attgtatact aaaactattc cacaatgagg ggttctacat aataaaagag 9540

gtagagggat ttattatgtc tctaatttta aatataacag aagaagatca attcagaaaa 9600

cggttttata atagtatgct caacaacatc acagatgccg ccaacaaagc tcaaaaaaat 9660

ctgctatcaa gagtatgtca tacattatta gataagacaa tatcagataa tataataaat 9720

ggcagatgga taattctatt gagtaagttc ctaaaattaa ttaagcttgc aggtgacaat 9780

aacctcaaca atctgagtga attatatttt ttgttcagaa tatttggaca cccaatggta 9840

gatgaaagac aagccatgga tgctgttaaa gttaattgca acgagaccaa attttatttg 9900

ttaagtagtt tgagtatgtt aagaggagct tttatatata gaattataaa agggtttgta 9960

aataattaca acagatggcc tactttaaga aatgccattg tcttaccctt aagatggtta 10020

acttactata aactaaacac ttatccttcc ttgttggaac ttacagaaag agatttgatt 10080

gttctatcag gactacgttt ctatcgagag tttcggttgc ctaaaaaagt ggatcttgaa 10140

atgatcataa atgataaggc tatatcacct cctaaaaatt taatatggac tagtttccct 10200

agaaattata tgccgtcaca catacaaaat tatatagaac atgaaaaatt aaaattctct 10260

gatagtgata aatcaagaag agtattagag tattatttaa gagataacaa attcaatgaa 10320

tgtgatttac acaactgtgt agttaatcaa agttatctta acaacccgaa tcatgtggta 10380

tcattgacag gcaaagaaag agaactcagt gtaggtagaa tgtttgcaat gcaaccagga 10440

atgttcagac aagttcaaat attagcagag aaaatgatag cagaaaacat attacaattt 10500

ttccctgaaa gtcttacaag atatggtgat ctagaactac agaaaatatt agaattgaaa 10560

gcaggaataa gtaacaaatc aaatcgttac aatgataatt acaacaatta cattagtaag 10620

tgctctatca tcacagatct cagcaaattc aatcaagcat ttcgatatga aacatcatgt 10680

atttgtagtg atgtactgga tgaactgcat ggtgtacaat ctctattttc ctggttacat 10740

ttaactattc ctcatgtcac aataatatgc acatataggc atgcaccccc ctatataaag 10800

gatcatattg tagatcttaa caatgtagat gagcaaagtg gactatatag atatcatatg 10860

ggtggtatcg aagggtggtg tcaaaaacta tggaccatag aagctatatc actattagat 10920

ctaatatctc tcaaagggaa attctcaatt actgctttaa ttaatggtga caatcaatca 10980

atagatataa gtaaaccagt cagactcatg gaaggtcaaa ctcatgctca agcagattat 11040

ttgctagcat taaatagtct caaattactg tataaagagt atgcaggaat aggccacaaa 11100

ttaaaaggaa ctgagactta tatatcgaga gatatgcaat ttatgagtaa aacgatccaa 11160

cataacggtg tatattaccc agctagtata aagaaagtcc taagagtggg accgtggata 11220

aacactatac ttgatgactt caaagtgagt ctagaatcta taggtagttt gacacaagaa 11280

ttagaatata gaggtgaaag tctattatgc agtttaatat ttagaaatgt atggttatat 11340

aatcaaattg cattacaact taaaaatcat gcattatgta acaacaaatt atatttggat 11400

atattaaaag ttctaaaaca cttaaaaacc ttttttaatc ttgataacat tgatacagca 11460

ttaacattgt atatgaattt gcccatgtta tttggtggtg gtgatcccaa cttgttatat 11520

cgaagtttct atagaagaac tcctgatttc ctcacagagg ctatagttca ctctgtgttc 11580

atacttagtt attatacaaa ccatgattta aaagataaac ttcaagatct gtcagatgat 11640

agattgaata agttcttaac atgcataatc acgtttgata aaaaccccaa tgctgaattc 11700

gttacattga tgagagatcc tcaagcttta ggatctgaga ggcaagctaa aattactagc 11760

gaaatcaata gactggcagt taccgaggtt ttgagcacag ctccaaacaa aatattttcc 11820

aaaagtgcac aacactatac cactacagag atagatctta atgatattat gcaaaatata 11880

gaacctacat atcctcacgg gctaagagtt gtttatgaga gtttaccctt ttataaagca 11940

gagaaaatag taaatcttat atccggtaca aaatctataa ctaacatact ggaaaagact 12000

tctgccatag acttaacaga tattgataga gccactgaga tgatgaggaa aaacataact 12060

ttgcttataa ggatattacc attagattgt aacagagata aaagagaaat attgagtatg 12120

gaaaacctaa gtattactga attaagcaaa tacgttagag aaagatcttg gtctttatcc 12180

aatatagttg gtgttacatc acccagtatc atgtatacaa tggacataaa atatacaaca 12240

agcactatag ctagtggcat aatcatagag aaatataatg tcaacagttt aacacgtggt 12300

gagagaggac ccactaaacc atgggttggt tcatctacac aagagaaaaa gacaatgcca 12360

gtttataata gacaagtttt aaccaaaaaa cagagagatc aaatagatct attagcaaaa 12420

ttggattggg tgtatgcatc tatagataac aaggatgaat ttatggagga acttagcata 12480

ggaactcttg ggttaacata tgagaaggcc aaaaaattat tcccacaata tttgagtgtt 12540

aactatttgc atcgtcttac agtcagtagt agaccatgtg aattccctgc atctatacca 12600

gcttatagaa ctacaaatta tcactttgat actagcccta ttaatcgcat attaacagaa 12660

aagtatggtg atgaagatat tgatatagta ttccaaaact gtataagctt tggccttagc 12720

ttaatgtctg tagtagaaca atttactaat gtatgtccta acagaattat tctcataccc 12780

aagcttaatg agatacattt gatgaaacct cccatattca caggcgatgt tgatattcac 12840

aagttaaaac aagtgataca aaaacaacat atgtttttac cagacaaaat aagtttgact 12900

caatatgtgg aattattctt aagtaataaa acactcaaat ctggatctaa tgttaattct 12960

aatttaatat tggcgcataa gatatctgac tattttcata atacttacat tttgagtact 13020

aatttagctg gacattggat tcttattata caacttatga aagattctaa gggtattttt 13080

gaaaaagatt ggggagaggg atatataact gatcatatgt tcattaattt gaaagttttc 13140

ttcaatgctt ataagacata tctcttgtgt tttcataaag gttacggcag agcaaagctg 13200

gagtgtgata tgaatacttc agatctccta tgtgtattgg aattaataga cagtagttat 13260

tggaagtcta tgtctaaggt gtttttagaa caaaaagtta tcaaatacat tcttagccag 13320

gatgcaagtt tacatagagt aaaaggatgt catagcttca aactatggtt tcttaaacgt 13380

cttaatgtag cagaattcac agtttgccct tgggttgtta acatagatta tcatccaaca 13440

catatgaaag caatattaac ttatattgat cttgttagaa tgggattgat aaatatagat 13500

agaatataca ttaaaaataa acacaagttc aatgatgagt tttatacttc taatctgttt 13560

tacattaatt ataacttctc agataatact catctattaa ctaaacatat aaggattgct 13620

aattccgaat tagaaagtaa ttacaacaaa ttatatcatc ctacaccaga aaccctagaa 13680

aatatactaa ccaatccggt taaaagtaat gagaaaaaga cactgagtga ctattgtata 13740

ggtaaaaatg ttgactcaat aatgttacca tcgttatcta ataagaagct tattaaatcg 13800

tctacaatga ttagaaccaa ttacagcaga caagatttgt ataatttatt tcctacggtt 13860

gtgattgata aaattataga tcattcaggt aatacagcca aatctaacca actttacact 13920

actacttctc atcaaatatc cttagtgcac aatagcacat cactttattg catgcttcct 13980

tggcatcata ttaatagatt caattttgta tttagttcta caggttgtaa aattagtata 14040

gagtatattt taaaagatct taaaattaag gatcctaatt gtatagcatt cataggtgaa 14100

ggagcaggga atttattatt gcgtacagta gtggaacttc atcctgatat aagatatatt 14160

tacagaagtc tgaaagattg caatgatcat agtttaccaa ttgagttttt aaggctgtac 14220

aatggacata tcaacattga ttatggtgaa aatttgacca ttcctgctac agatgcaacc 14280

aacaacattc attggtctta tttacatata aagtttgctg aacctatcag tctttttgtc 14340

tgtgatgctg aattgcctgt aacagtcaac tggagtaaga ttataataga gtggagcaag 14400

catgtaagaa aatgcaagta ctgttcttca gttaataaat gtacattgat agtaaaatat 14460

catgctcaag atgatatcga tttcaaatta gacaacataa ctatattaaa aacttatgta 14520

tgcttaggta gtaagttaaa gggatctgaa gtttacttag tccttacaat aggtcctgca 14580

aatgtgttcc cagtatttaa tgtagtacaa aatgctaaat tgatactatc aagaactaaa 14640

aatttcatca tgcctaaaaa agctgataaa gagtctattg atgcaaatat taagagtttg 14700

ataccctttc tttgttaccc tataacaaaa aaaggaatta atactgcatt gtctaaatta 14760

aagagtgttg ttagtggaga tatactatca tattctatag ctggacgtaa tgaagttttc 14820

agcaataaac ttataaatca taagcatatg aacatcttaa agtggttcaa tcatgtttta 14880

aatttcagat caacagaatt aaactataat catttatata tggtagaatc tacttatcct 14940

catctaagtg aattgttaaa cagcttgaca accaatgaac ttaaaaaact gattaaaatc 15000

acaggtagtt tgttatacaa cttttataat gaataatgag caaaaatctt ataacaaaaa 15060

tagctacaca ctaacattgt attcaattat agttattgaa aattaataat tatataattt 15120

ttaataactt ctagtgaact aatcctaaaa ttatcatttt gatctaggaa gaataagttt 15180

aaatccaaat ctaattggtt tatatgtata ttaactaaat tacgagatat ta 15232

<210> 2
<211> 15311
<212> DNA
<213> respiratory syncytial virus

<400> 2
acgcgaaaaa atgcgtacaa caaacttgcg taaaccaaaa aaatggggca aataagaatt 60

tgataagtac cacttaaatt taactccttt ggttagaggc gcgccatggg cagcaactca 120

ttgagtatga taaaagttag attgcaaaat ctgtttgaca atgatgaagt agcattgtta 180

aaaataacat gctatactga caaattaata cagttaacta atgctttggc taaggcagtt 240

atacatacaa tcaaattgaa tggcattgta tttgtgcatg ttattacaag tagtgatatt 300

tgccctaata ataatattgt agtgaaatcc aatttcacaa caatgccagt attacaaaat 360

ggaggttata tatgggaaat gatggaatta acacactgct ctcaacctaa tggcctaata 420

gatgacaatt gtgaaattaa attctccaaa aaactaagtg attcaacaat gaccaattat 480

atgaatcaat tatctgaatt acttggattt gacctcaatc cataaatcat aataaatatc 540

aactagcaaa tcaatgtcac taacaccatt agttaatata aaacttgaca gaagataaaa 600

atggggcaaa taaatcaatt cagccgaccc aaccatggac acaacacaca atgataccac 660

accacaaaga ctgatgatca cagacatgag gccattatcg cttgagacta taataacatc 720

tctaaccaga gatatcataa cacataaatt tatatacttg ataaatcatg aatgcatagt 780

aagaaaactt gatgaaagac aggccacatt tacatttctg gtcaactatg aaatgaaact 840

attgcacaaa gtgggaagca ctaaatataa aaaatatact gaatacaaca caaaatatgg 900

cactttccct atgccaatat ttatcaatca tgatgggttc ttagaatgca ttggcattaa 960

gcctaccaag cacacaccca taatatacaa gtatgatctc aatccatgaa tatcaaacca 1020

agattcaaac aatccgaaat aacaacttta tgcataatca cactccatag tccaaatgga 1080

gcctgaaaat tatagttatt taaaattcct gcaggaagga gagacataag atgaaagatg 1140

gggcaaatac aaaaatggct cttagcaaag tcaagttgaa tgatacactc aacaaagatc 1200

aacttctatc atccagcaaa tataccatcc aacggagcac aggagacagc attgacactc 1260

ctaattatga tgtgcagaaa cacattaata agttatgtgg catgttatta atcacagaag 1320

atgctaatca taaattcact gggttaatag gtatgttata tgctatgtct agattaggaa 1380

gagaagacac cataaaaata ctcaaagatg cgggatatca tgttaaggca aatggagtgg 1440

atgtaacaac acatcgtcaa gacattaatg ggaaagaaat gaaatttgaa gtgttaacat 1500

tagcaagctt aacaactgaa attcaaatca acattgagat agaatctaga aaatcctaca 1560

aaaaaatgct aaaagaaatg ggagaggtgg ctccagaata caggcatgac tctcctgatt 1620

gtgggatgat aatattatgt atagcggcat tagtaataac caaattagca gcaggagata 1680

gatcaggtct tacagctgtg attaggagag ctaataatgt cctaaaaaat gaaatgaaac 1740

gttataaagg tttattaccc aaggatatag ccaacagctt ctatgaagtg tttgaaaaat 1800

atcctcactt tatagatgtt tttgttcatt ttggtatagc acaatcttct accagaggtg 1860

gcagtagagt tgaaggattt tttgcaggat tgtttatgaa tgcctatggt gcagggcaag 1920

tgatgttacg gtggggggtc ttagcaaaat cagttaaaaa cattatgtta ggacacgcta 1980

gtgtacaagc agaaatggaa caagttgtgg aggtgtatga gtatgctcag aaattgggtg 2040

gagaagcagg attctaccat atattgaaca acccaaaagc atcactatta tctttgactc 2100

aatttcctca cttctctagt gtagtattgg gcaatgctgc tggcctaggc ataatgggag 2160

aatacagagg tacaccaagg aatcaagatt tatatgatgc tgcaaaagca tatgctgaac 2220

aactcaaaga aaatggtgtg attaactaca gtgtattaga tttgacagca gaagaactag 2280

aggctatcaa acatcagctt aatccaaaag ataatgatgt agagctttga gttaataaaa 2340

aagtggggca aataaatcat catggaaaag tttgctcctg aattccatgg agaagatgca 2400

aacaacagag ccaccaaatt cctagaatca ataaagggca aattcacatc acccaaagat 2460

cccaagaaaa aagatagtat catatctgtc aactcaatag atatagaagt aaccaaagaa 2520

agccctataa catcaaattc aaccattata aacccaataa atgagacaga tgatactgta 2580

gggaacaagc ccaattatca aagaaagcct ctagtaagtt tcaaagaaga ccctacgcca 2640

agtgataatc ctttttcaaa actatacaaa gaaaccatag aaacatttga taacaatgaa 2700

gaagaatcta gctattcata tgaagaaata aatgatcaga caaacgataa tataacagca 2760

agattagata ggattgatga gaaattaagt gaaatactag gaatgcttca cacattagta 2820

gtagcgagtg caggacccac atctgctcgg gatggtataa gagatgccat ggttggttta 2880

agagaagaaa tgatagaaaa aatcagaact gaagcattaa tgaccaatga cagactagaa 2940

gctatggcaa gactcaggaa tgaagaaagt gaaaagatgg caaaagacac atcagatgaa 3000

gtgtctctca atccaacatc agagaaactg aacaacctgt tggaagggaa tgatagtgac 3060

aatgatctat cacttgaaga tttctgatta gctaccaaac tgtacatcaa aacacaacac 3120

caatagaaaa ccaacaaaca aaccaactca cccatccaac caaacatcta tctgctgatt 3180

agccaaccag ccaaaaaaca accagccaat ctaaaactag ccacccggaa aaaatcgata 3240

ctatagttac aaaaaaagat ggggcaaata tggaaacata cgtgaataaa cttcacgagg 3300

gctccacata cacagctgct gttcaataca atgtcctaga aaaagacgat gatcctgcat 3360

cacttacaat atgggtgccc atgttccaat catccatgcc agcagatcta ctcataaaag 3420

aactagccaa tgtcaatata ctagtgaaac aaatatccac acccaaggga ccctcattaa 3480

gagtcatgat aaactcaaga agtgcagtgc tagcacaaat gcccagcaaa tttaccatat 3540

gtgccaatgt gtccttggat gaaagaagca agctggcata tgatgtaacc acaccctgtg 3600

aaattaaggc atgcagtcta acatgcctaa aatcaaaaaa tatgttaact acagttaaag 3660

atctcactat gaaaacactc aacccaacac atgacatcat tgctttatgt gaatttgaaa 3720

atatagtaac atcaaaaaaa gtcataatac caacatacct aagatctatc agcgtcagaa 3780

ataaagatct gaacacactt gaaaatataa caaccactga attcaaaaat gccattacaa 3840

atgcaaaaat catcccttac tcaggattat tgttagtcat cacagtgact gacaacaaag 3900

gagcattcaa atacataaag ccacaaagtc aattcatagt agatcttgga gcttacctag 3960

aaaaagaaag tatatattat gttacaacaa attggaagca cacagctaca cgatttgcaa 4020

tcaaacccat ggaagattaa cctttttcct ctacatcaat gagtagattc atacaaactt 4080

tctaactaca ttcttcactt cacaatcata atcaccaacc ctctgtggtt caatcaatca 4140

aacaaaactc atcaagagtt ccagatcatc ccaagtcatt gttcatcaga tccagtactc 4200

aaataagtta ataaaaaatc cacatggggc aaataatcat tgagggaaat ccaactaatc 4260

acaacatctg tcaacataga caagtcaaca cgctagataa aatcaaccaa tggaaaatac 4320

atccataact atagaattct caagcaaatt ctggccttac tttacactaa tacacatgat 4380

aacaacaata atctctttga taatcataat ctccatcatg attgcaatac taaacaaact 4440

ctgcgaatat aatgtattcc ataacaaaac ctttgagcta ccaagagctc gagtcaatac 4500

atagcattca ccaatctgat agctcaaaac agtaaccttg catttgtaaa tgaactaccc 4560

tcacttcttc acaaaaccac atcaacatct caccatgcaa gccatcatct ataccataaa 4620

gtagttaatt aaaaatggcc ggccagtcat aacaatgaac taggatatta agaccaaaaa 4680

caacgctggg gcaaatgcaa acatgtccaa aaccgaggac caacgcaccg ccaagacact 4740

agaaaggacc tgggacactt ttaatcatct attattcata tcatcgtgct tatacaagtt 4800

aaatcttaaa tctatagcac aaatcacatt atctattttg gcaattataa tctcaacctc 4860

acttataatt gcagccatca tattcatagc ctcggcaaac cacaaagtca cactaacaac 4920

tgcaatcata caagatgcaa cgaaccagat caagaacaca accccaacat acctcaccca 4980

gaatccccag catggaatca gcttctccaa tctgtccgga actacatcac aatccaccac 5040

catactagct tcaacaacac caagtgctga ttcaacccca caatccacaa cagtcaagat 5100

caaaaacaca acaacaaccc aaatattacc tagcaaaccc accacaaaac aacgccaaaa 5160

taaaccacaa aacaaaccca acaatgattt tcactttgaa gtgttcaatt ttgtaccctg 5220

cagcatatgc agcaacaatc caacctgctg ggccatctgc aagagaatac caaacaaaaa 5280

acctggaaag aaaaccacca ccaagcccac aaaaaaacca accctcaaga caaccaaaaa 5340

agatcccaaa tcccaaacca caaaaccaaa ggaagtactc actaccaagc ctacaggaaa 5400

gccaaccatc aacaccacta aaacaaacat cagaactaca ctgctcacct ccaacaccaa 5460

aggaaatcca gaacacacaa gtcaagagga aaccctccac tcaaccacct ccgaaggcta 5520

tccaagccca tcacaagtcc acacaacatc cggtcaagag gaaaccctcc actcaaccac 5580

ctccgaaggc tatccaagcc catcacaagt ctacacaaca tccgagtacc tatcacaatc 5640

tctatcttca tccaacacaa caaaatgata gtcattaaaa agcacgcgtg tattgttgca 5700

aaaagccatg accaaatcaa acagaatcaa aatcaactct ggggcaaata acaatggagt 5760

tgccaatcct caaaacaaat gctattacca caatccttgc tgcagtcaca ctctgtttcg 5820

cttccagtca aaacatcact gaagaatttt atcaatcaac atgcagtgca gtcagcaaag 5880

gctatcttag tgctctaaga actggttggt atactagtgt tataactata gaattaagta 5940

atatcaagga aaataagtgt aatggtacag acgctaaggt aaaattaata aaacaagaat 6000

tagataaata taaaaatgct gtaacagaat tgcagttgct catgcaaagc acaccagcag 6060

ccaacaatcg agccagaaga gaactaccaa gatttatgaa ttatacactc aacaatacca 6120

aaaacaccaa tgtaacatta agtaagaaaa ggaaaagaag atttcttgga tttttgttag 6180

gtgttggatc tgcaatcgcc agtggcattg ccgtatccaa ggtcctgcac ctagaagggg 6240

aagtgaacaa aatcaaaagt gctctactat ccacaaacaa ggctgtagtc agcttatcta 6300

atggagtcag tgtcttaacc agcaaggtgt tagacctcaa aaactatata gataaacagt 6360

tgttacctat tgttaacaag caaagctgca gcatatcaaa cattgaaact gtgatagagt 6420

tccaacaaaa gaacaacaga ctactagaga ttaccagaga atttagtgtt aatgcaggtg 6480

taactactcc tgtaagcact tatatgttaa ctaatagtga gttattatca ttaatcaatg 6540

atatgcctat aacaaatgat cagaaaaagt taatgtccag caatgttcaa atagttagac 6600

agcaaagtta ctctatcatg tcaataataa aagaggaagt cttggcatat gtagtacaat 6660

taccactata tggtgtaata gatactcctt gttggaaact acacacatcc cctttatgta 6720

caaccaacac aaaggaagga tccaacatct gcttaacaag aaccgacaga ggatggtact 6780

gtgacaatgc aggatcagta tcctttttcc cacaagctga aacatgtaaa gttcaatcga 6840

atcgggtgtt ttgtgacaca atgaacagtt taacattacc aagtgaggta aatctctgca 6900

acattgacat attcaacccc aaatatgatt gcaaaattat gacttcaaaa acagatgtaa 6960

gcagctccgt tatcacatct ctaggagcca ttgtgtcatg ctatggcaaa accaaatgta 7020

cagcatccaa taaaaatcgt gggatcataa agacattctc taacgggtgt gattatgtat 7080

caaataaggg ggtggatact gtgtctgtag gtaatacatt atattatgta aataagcaag 7140

aaggcaaaag tctctatgta aaaggtgaac caataataaa tttctatgat ccattagtgt 7200

tcccctctga tgaatttgat gcatcaatat ctcaagtcaa tgagaaaatt aatcagagtc 7260

tagcatttat ccgtaaatca gatgaattat tacataatgt aaatgctggt aaatccacca 7320

caaatatcat gataactacc ataattatag taattatagt aatattgtta gcattaattg 7380

cagttggact gcttctatac tgcaaggcca gaagcacacc agtcacatta agtaaggatc 7440

aactgagtgg tataaataat attgcattta gtaactgaat aaaaatagca cctaatcata 7500

ttcttacaat ggttcgctat ttgaccatag ataacccatc tatcattaga ttatcctaaa 7560

atttgaactt catcacaact ttcatctata aaccatctca cttacacttt ttaagtagat 7620

ttctatttta tagttatata aaacaggccc cattgaatac caaattaact tactatttgt 7680

aaaaatgaga attggggcaa atatgtcacg aaggaatcct tgcaaattcg aaattcgagg 7740

tcattgcttg aatggtaaaa ggtgtcattt tagtcataat tattttgaat ggccacccca 7800

tgcactgctt gtaagacaaa actttatgtt aaacagaata cttaagtcta tggataaaag 7860

catagatact ttgtcagaaa taagtggagc tgcagagttg gacagaacag aagagtatgc 7920

cctcggtgta gttggagtgc tagagagtta tataggatca ataaataata taactaaaca 7980

atcagcatgt gttgccatga gcaaactcct tactgaactc aacagcgatg acatcaaaaa 8040

actaagggac aatgaagagc caaactcacc caaagtaaga gtgtacaata ctgtcatatc 8100

atatattgaa agcaacagga agaacaataa acaaactatc catctgttaa aaagattgcc 8160

agcagacgta ttgaagaaaa ccatcaaaaa cacattggat atccacaaga gcataaccat 8220

caataaccca aaagaatcaa ctgttagtga tacgaacgac catgccaaaa ataatgatac 8280

tacctgacaa atatccttgt agtataaatt ccatactaat aacaagtaat tgtagagtca 8340

ctatgtataa tcaaaaaaac acactatata tcaatcaaaa caaccaaaat agccatatat 8400

acccaccgga tcaaccattc aatgaaatcc attggacctc tcaagacttg attgatgcaa 8460

ctcaaaattt tctacaacat ctaggtatta ctgatgatat atacacaata tatatattag 8520

tgtcataata ctcaatccta atacttacca catcatcaaa ttattaactc aaacaattca 8580

agctatggga caaaatggat cccattatta gtggaaattc tgctaatgtt tatctaactg 8640

atagttattt aaaaggtgtt atttctttct cagaatgtaa cgctttagga agttacatat 8700

tcaatggtcc ttatctcaaa aatgattata ccaacttaat tagtagacaa aatccattaa 8760

tagaacacat aaatctaaag aaactaaata taacacagtc cttaatatct aagtatcata 8820

aaggtgaaat aaaaatagaa gaacctactt actttcagtc attacttatg acatacaaga 8880

gtatgacctc ttcagaacag actactacta ctaatttact taaaaagata ataagaagag 8940

ctatagaaat cagtgatgtc aaagtctatg ctatattgaa taaactgggg ctcaaagaaa 9000

aagacaagat taaatccaat aatggacaag atgaagacaa ctcagtcatt actaccataa 9060

tcaaagatga tatactttta gctgtcaagg ataatcaatc tcatcttaaa gcagacaaaa 9120

atcaatccac aaaacaaaaa gatacaatca aaacaacact tttgaagaaa ttaatgtgtt 9180

cgatgcaaca tcctccatca tggttaatac attggtttaa tttatacaca aaattaaaca 9240

gcatattaac acaatatcga tctagtgagg taaaaaacca tggttttata ttgatagata 9300

atcatactct tagtggattc caatttattt tgaatcaata tggttgtata gtttatcata 9360

aggaactcaa aagaattact gtgacaactt ataatcaatt cttgacatgg aaagatatta 9420

gccttagtag attaaatgtt tgtttgatta catggattag taactgtttg aacacattaa 9480

acaaaagctt aggcttaaga tgtggattca ataatgttat cttgacacaa ttattccttt 9540

atggagattg tatactaaaa ctattccaca atgaggggtt ctacataata aaagaggtag 9600

agggatttat tatgtctcta attttaaata taacagaaga agatcaattc agaaaacggt 9660

tttataatag tatgctcaac aacatcacag atgccgccaa caaagctcaa aaaaatctgc 9720

tatcaagagt atgtcataca ttattagata agacaatatc agataatata ataaatggca 9780

gatggataat tctattgagt aagttcctaa aattaattaa gcttgcaggt gacaataacc 9840

tcaacaatct gagtgaatta tattttttgt tcagaatatt tggacaccca atggtagatg 9900

aaagacaagc catggatgct gttaaagtta attgcaacga gaccaaattt tatttgttaa 9960

gtagtttgag tatgttaaga ggagctttta tatatagaat tataaaaggg tttgtaaata 10020

attacaacag atggcctact ttaagaaatg ccattgtctt acccttaaga tggttaactt 10080

actataaact aaacacttat ccttccttgt tggaacttac agaaagagat ttgattgttc 10140

tatcaggact acgtttctat cgagagtttc ggttgcctaa aaaagtggat cttgaaatga 10200

tcataaatga taaggctata tcacctccta aaaatttaat atggactagt ttccctagaa 10260

attatatgcc gtcacacata caaaattata tagaacatga aaaattaaaa ttctctgata 10320

gtgataaatc aagaagagta ttagagtatt atttaagaga taacaaattc aatgaatgtg 10380

atttacacaa ctgtgtagtt aatcaaagtt atcttaacaa cccgaatcat gtggtatcat 10440

tgacaggcaa agaaagagaa ctcagtgtag gtagaatgtt tgcaatgcaa ccaggaatgt 10500

tcagacaagt tcaaatatta gcagagaaaa tgatagcaga aaacatatta caatttttcc 10560

ctgaaagtct tacaagatat ggtgatctag aactacagaa aatattagaa ttgaaagcag 10620

gaataagtaa caaatcaaat cgttacaatg ataattacaa caattacatt agtaagtgct 10680

ctatcatcac agatctcagc aaattcaatc aagcatttcg atatgaaaca tcatgtattt 10740

gtagtgatgt actggatgaa ctgcatggtg tacaatctct attttcctgg ttacatttaa 10800

ctattcctca tgtcacaata atatgcacat ataggcatgc acccccctat ataaaggatc 10860

atattgtaga tcttaacaat gtagatgagc aaagtggact atatagatat catatgggtg 10920

gtatcgaagg gtggtgtcaa aaactatgga ccatagaagc tatatcacta ttagatctaa 10980

tatctctcaa agggaaattc tcaattactg ctttaattaa tggtgacaat caatcaatag 11040

atataagtaa accagtcaga ctcatggaag gtcaaactca tgctcaagca gattatttgc 11100

tagcattaaa tagtctcaaa ttactgtata aagagtatgc aggaataggc cacaaattaa 11160

aaggaactga gacttatata tcgagagata tgcaatttat gagtaaaacg atccaacata 11220

acggtgtata ttacccagct agtataaaga aagtcctaag agtgggaccg tggataaaca 11280

ctatacttga tgacttcaaa gtgagtctag aatctatagg tagtttgaca caagaattag 11340

aatatagagg tgaaagtcta ttatgcagtt taatatttag aaatgtatgg ttatataatc 11400

aaattgcatt acaacttaaa aatcatgcat tatgtaacaa caaattatat ttggatatat 11460

taaaagttct aaaacactta aaaacctttt ttaatcttga taacattgat acagcattaa 11520

cattgtatat gaatttgccc atgttatttg gtggtggtga tcccaacttg ttatatcgaa 11580

gtttctatag aagaactcct gatttcctca cagaggctat agttcactct gtgttcatac 11640

ttagttatta tacaaaccat gatttaaaag ataaacttca agatctgtca gatgatagat 11700

tgaataagtt cttaacatgc ataatcacgt ttgataaaaa ccccaatgct gaattcgtta 11760

cattgatgag agatcctcaa gctttaggat ctgagaggca agctaaaatt actagcgaaa 11820

tcaatagact ggcagttacc gaggttttga gcacagctcc aaacaaaata ttttccaaaa 11880

gtgcacaaca ctataccact acagagatag atcttaatga tattatgcaa aatatagaac 11940

ctacatatcc tcacgggcta agagttgttt atgagagttt acccttttat aaagcagaga 12000

aaatagtaaa tcttatatcc ggtacaaaat ctataactaa catactggaa aagacttctg 12060

ccatagactt aacagatatt gatagagcca ctgagatgat gaggaaaaac ataactttgc 12120

ttataaggat attaccatta gattgtaaca gagataaaag agaaatattg agtatggaaa 12180

acctaagtat tactgaatta agcaaatacg ttagagaaag atcttggtct ttatccaata 12240

tagttggtgt tacatcaccc agtatcatgt atacaatgga cataaaatat acaacaagca 12300

ctatagctag tggcataatc atagagaaat ataatgtcaa cagtttaaca cgtggtgaga 12360

gaggacccac taaaccatgg gttggttcat ctacacaaga gaaaaagaca atgccagttt 12420

ataatagaca agttttaacc aaaaaacaga gagatcaaat agatctatta gcaaaattgg 12480

attgggtgta tgcatctata gataacaagg atgaatttat ggaggaactt agcataggaa 12540

ctcttgggtt aacatatgag aaggccaaaa aattattccc acaatatttg agtgttaact 12600

atttgcatcg tcttacagtc agtagtagac catgtgaatt ccctgcatct ataccagctt 12660

atagaactac aaattatcac tttgatacta gccctattaa tcgcatatta acagaaaagt 12720

atggtgatga agatattgat atagtattcc aaaactgtat aagctttggc cttagcttaa 12780

tgtctgtagt agaacaattt actaatgtat gtcctaacag aattattctc atacccaagc 12840

ttaatgagat acatttgatg aaacctccca tattcacagg cgatgttgat attcacaagt 12900

taaaacaagt gatacaaaaa caacatatgt ttttaccaga caaaataagt ttgactcaat 12960

atgtggaatt attcttaagt aataaaacac tcaaatctgg atctaatgtt aattctaatt 13020

taatattggc gcataagata tctgactatt ttcataatac ttacattttg agtactaatt 13080

tagctggaca ttggattctt attatacaac ttatgaaaga ttctaagggt atttttgaaa 13140

aagattgggg agagggatat ataactgatc atatgttcat taatttgaaa gttttcttca 13200

atgcttataa gacatatctc ttgtgttttc ataaaggtta cggcagagca aagctggagt 13260

gtgatatgaa tacttcagat ctcctatgtg tattggaatt aatagacagt agttattgga 13320

agtctatgtc taaggtgttt ttagaacaaa aagttatcaa atacattctt agccaggatg 13380

caagtttaca tagagtaaaa ggatgtcata gcttcaaact atggtttctt aaacgtctta 13440

atgtagcaga attcacagtt tgcccttggg ttgttaacat agattatcat ccaacacata 13500

tgaaagcaat attaacttat attgatcttg ttagaatggg attgataaat atagatagaa 13560

tatacattaa aaataaacac aagttcaatg atgagtttta tacttctaat ctgttttaca 13620

ttaattataa cttctcagat aatactcatc tattaactaa acatataagg attgctaatt 13680

ccgaattaga aagtaattac aacaaattat atcatcctac accagaaacc ctagaaaata 13740

tactaaccaa tccggttaaa agtaatgaga aaaagacact gagtgactat tgtataggta 13800

aaaatgttga ctcaataatg ttaccatcgt tatctaataa gaagcttatt aaatcgtcta 13860

caatgattag aaccaattac agcagacaag atttgtataa tttatttcct acggttgtga 13920

ttgataaaat tatagatcat tcaggtaata cagccaaatc taaccaactt tacactacta 13980

cttctcatca aatatcctta gtgcacaata gcacatcact ttattgcatg cttccttggc 14040

atcatattaa tagattcaat tttgtattta gttctacagg ttgtaaaatt agtatagagt 14100

atattttaaa agatcttaaa attaaggatc ctaattgtat agcattcata ggtgaaggag 14160

cagggaattt attattgcgt acagtagtgg aacttcatcc tgatataaga tatatttaca 14220

gaagtctgaa agattgcaat gatcatagtt taccaattga gtttttaagg ctgtacaatg 14280

gacatatcaa cattgattat ggtgaaaatt tgaccattcc tgctacagat gcaaccaaca 14340

acattcattg gtcttattta catataaagt ttgctgaacc tatcagtctt tttgtctgtg 14400

atgctgaatt gcctgtaaca gtcaactgga gtaagattat aatagagtgg agcaagcatg 14460

taagaaaatg caagtactgt tcttcagtta ataaatgtac attgatagta aaatatcatg 14520

ctcaagatga tatcgatttc aaattagaca acataactat attaaaaact tatgtatgct 14580

taggtagtaa gttaaaggga tctgaagttt acttagtcct tacaataggt cctgcaaatg 14640

tgttcccagt atttaatgta gtacaaaatg ctaaattgat actatcaaga actaaaaatt 14700

tcatcatgcc taaaaaagct gataaagagt ctattgatgc aaatattaag agtttgatac 14760

cctttctttg ttaccctata acaaaaaaag gaattaatac tgcattgtct aaattaaaga 14820

gtgttgttag tggagatata ctatcatatt ctatagctgg acgtaatgaa gttttcagca 14880

ataaacttat aaatcataag catatgaaca tcttaaagtg gttcaatcat gttttaaatt 14940

tcagatcaac agaattaaac tataatcatt tatatatggt agaatctact tatcctcatc 15000

taagtgaatt gttaaacagc ttgacaacca atgaacttaa aaaactgatt aaaatcacag 15060

gtagtttgtt atacaacttt tataatgaat aatgagcaaa aatcttataa caaaaatagc 15120

tacacactaa cattgtattc aattatagtt attgaaaatt aataattata taatttttaa 15180

taacttctag tgaactaatc ctaaaattat cattttgatc taggaagaat aagtttaaat 15240

ccaaatctaa ttggtttata tgtatattaa ctaaattacg agatattagt ttttgacact 15300

tttttctcgt t 15311

<210> 3
<211> 15494
<212> DNA
<213> Artificial Sequence

<220>
<223> RSV backbone sequence_A

<400> 3
taatacgact cactataggt tttttcgcgt ctgatgaggc cgttaggccg aaactcctct 60

ccggagtcac gcgaaaaaat gcgtacaaca aacttgcgta aaccaaaaaa atggggcaaa 120

taagaatttg ataagtacca cttaaattta actcctttgg ttagaggcgc gccatgggca 180

gcaactcatt gagtatgata aaagttagat tgcaaaatct gtttgacaat gatgaagtag 240

cattgttaaa aataacatgc tatactgaca aattaataca gttaactaat gctttggcta 300

aggcagttat acatacaatc aaattgaatg gcattgtatt tgtgcatgtt attacaagta 360

gtgatatttg ccctaataat aatattgtag tgaaatccaa tttcacaaca atgccagtat 420

tacaaaatgg aggttatata tgggaaatga tggaattaac acactgctct caacctaatg 480

gcctaataga tgacaattgt gaaattaaat tctccaaaaa actaagtgat tcaacaatga 540

ccaattatat gaatcaatta tctgaattac ttggatttga cctcaatcca taaatcataa 600

taaatatcaa ctagcaaatc aatgtcacta acaccattag ttaatataaa acttgacaga 660

agataaaaat ggggcaaata aatcaattca gccgacccaa ccatggacac aacacacaat 720

gataccacac cacaaagact gatgatcaca gacatgaggc cattatcgct tgagactata 780

ataacatctc taaccagaga tatcataaca cataaattta tatacttgat aaatcatgaa 840

tgcatagtaa gaaaacttga tgaaagacag gccacattta catttctggt caactatgaa 900

atgaaactat tgcacaaagt gggaagcact aaatataaaa aatatactga atacaacaca 960

aaatatggca ctttccctat gccaatattt atcaatcatg atgggttctt agaatgcatt 1020

ggcattaagc ctaccaagca cacacccata atatacaagt atgatctcaa tccatgaata 1080

tcaaaccaag attcaaacaa tccgaaataa caactttatg cataatcaca ctccatagtc 1140

caaatggagc ctgaaaatta tagttattta aaattcctgc aggaaggaga gacataagat 1200

gaaagatggg gcaaatacaa aaatggctct tagcaaagtc aagttgaatg atacactcaa 1260

caaagatcaa cttctatcat ccagcaaata taccatccaa cggagcacag gagacagcat 1320

tgacactcct aattatgatg tgcagaaaca cattaataag ttatgtggca tgttattaat 1380

cacagaagat gctaatcata aattcactgg gttaataggt atgttatatg ctatgtctag 1440

attaggaaga gaagacacca taaaaatact caaagatgcg ggatatcatg ttaaggcaaa 1500

tggagtggat gtaacaacac atcgtcaaga cattaatggg aaagaaatga aatttgaagt 1560

gttaacatta gcaagcttaa caactgaaat tcaaatcaac attgagatag aatctagaaa 1620

atcctacaaa aaaatgctaa aagaaatggg agaggtggct ccagaataca ggcatgactc 1680

tcctgattgt gggatgataa tattatgtat agcggcatta gtaataacca aattagcagc 1740

aggagataga tcaggtctta cagctgtgat taggagagct aataatgtcc taaaaaatga 1800

aatgaaacgt tataaaggtt tattacccaa ggatatagcc aacagcttct atgaagtgtt 1860

tgaaaaatat cctcacttta tagatgtttt tgttcatttt ggtatagcac aatcttctac 1920

cagaggtggc agtagagttg aagggatttt tgcaggattg tttatgaatg cctatggtgc 1980

agggcaagtg atgttacggt ggggggtctt agcaaaatca gttaaaaaca ttatgttagg 2040

acacgctagt gtacaagcag aaatggaaca agttgtggag gtgtatgagt atgctcagaa 2100

attgggtgga gaagcaggat tctaccatat attgaacaac ccaaaagcat cactattatc 2160

tttgactcaa tttcctcact tctctagtgt agtattgggc aatgctgctg gcctaggcat 2220

aatgggagaa tacagaggta caccaaggaa tcaagattta tatgatgctg caaaagcata 2280

tgctgaacaa ctcaaagaaa atggtgtgat taactacagt gtattagatt tgacagcaga 2340

agaactagag gctatcaaac atcagcttaa tccaaaagat aatgatgtag agctttgagt 2400

taataaaaaa gtggggcaaa taaatcatca tggaaaagtt tgctcctgaa ttccatggag 2460

aagatgcaaa caacagagcc accaaattcc tagaatcaat aaagggcaaa ttcacatcac 2520

ccaaagatcc caagaaaaaa gatagtatca tatctgtcaa ctcaatagat atagaagtaa 2580

ccaaagaaag ccctataaca tcaaattcaa ccattataaa cccaataaat gagacagatg 2640

atactgtagg gaacaagccc aattatcaaa gaaagcctct agtaagtttc aaagaagacc 2700

ctacgccaag tgataatcct ttttcaaaac tatacaaaga aaccatagaa acatttgata 2760

acaatgaaga agaatctagc tattcatatg aagaaataaa tgatcagaca aacgataata 2820

taacagcaag attagatagg attgatgaga aattaagtga aatactagga atgcttcaca 2880

cattagtagt agcgagtgca ggacccacat ctgctcggga tggtataaga gatgccatgg 2940

ttggtttaag agaagaaatg atagaaaaaa tcagaactga agcattaatg accaatgaca 3000

gactagaagc tatggcaaga ctcaggaatg aagaaagtga aaagatggca aaagacacat 3060

cagatgaagt gtctctcaat ccaacatcag agaaactgaa caacctgttg gaagggaatg 3120

atagtgacaa tgatctatca cttgaagatt tctgattagc taccaaactg tacatcaaaa 3180

cacaacacca atagaaaacc aacaaacaaa ccaactcacc catccaacca aacatctatc 3240

tgctgattag ccaaccagcc aaaaaacaac cagccaatct aaaactagcc acccggaaaa 3300

aatcgatact atagttacaa aaaaagatgg ggcaaatatg gaaacatacg tgaataaact 3360

tcacgagggc tccacataca cagctgctgt tcaatacaat gtcctagaaa aagacgatga 3420

tcctgcatca cttacaatat gggtgcccat gttccaatca tccatgccag cagatctact 3480

cataaaagaa ctagccaatg tcaatatact agtgaaacaa atatccacac ccaagggacc 3540

ctcattaaga gtcatgataa actcaagaag tgcagtgcta gcacaaatgc ccagcaaatt 3600

taccatatgt gccaatgtgt ccttggatga aagaagcaag ctggcatatg atgtaaccac 3660

accctgtgaa attaaggcat gcagtctaac atgcctaaaa tcaaaaaata tgttaactac 3720

agttaaagat ctcactatga aaacactcaa cccaacacat gacatcattg ctttatgtga 3780

atttgaaaat atagtaacat caaaaaaagt cataatacca acatacctaa gatctatcag 3840

cgtcagaaat aaagatctga acacacttga aaatataaca accactgaat tcaaaaatgc 3900

cattacaaat gcaaaaatca tcccttactc aggattattg ttagtcatca cagtgactga 3960

caacaaagga gcattcaaat acataaagcc acaaagtcaa ttcatagtag atcttggagc 4020

ttacctagaa aaagaaagta tatattatgt tacaacaaat tggaagcaca cagctacacg 4080

atttgcaatc aaacccatgg aagattaacc tttttcctct acatcaatga gtagattcat 4140

acaaactttc taactacatt cttcacttca caatcataat caccaaccct ctgtggttca 4200

atcaatcaaa caaaactcat caagagttcc agatcatccc aagtcattgt tcatcagatc 4260

cagtactcaa ataagttaat aaaaaatcca catggggcaa ataatcattg agggaaatcc 4320

aactaatcac aacatctgtc aacatagaca agtcaacacg ctagataaaa tcaaccaatg 4380

gaaaatacat ccataactat agaattctca agcaaattct ggccttactt tacactaata 4440

cacatgataa caacaataat ctctttgata atcataatct ccatcatgat tgcaatacta 4500

aacaaactct gcgaatataa tgtattccat aacaaaacct ttgagctacc aagagctcga 4560

gtcaatacat agcattcacc aatctgatag ctcaaaacag taaccttgca tttgtaaatg 4620

aactaccctc acttcttcac aaaaccacat caacatctca ccatgcaagc catcatctat 4680

accataaagt agttaattaa aaatggccgg ccagtcataa caatgaacta ggatattaag 4740

accaaaaaca acgctggggc aaatgcaaac atgtccaaaa ccgaggacca acgcaccgcc 4800

aagacactag aaaggacctg ggacactttt aatcatctat tattcatatc atcgtgctta 4860

tacaagttaa atcttaaatc tatagcacaa atcacattat ctattttggc aattataatc 4920

tcaacctcac ttataattgc agccatcata ttcatagcct cggcaaacca caaagtcaca 4980

ctaacaactg caatcataca agatgcaacg aaccagatca agaacacaac cccaacatac 5040

ctcacccaga atccccagca tggaatcagc ttctccaatc tgtccggaac tacatcacaa 5100

tccaccacca tactagcttc aacaacacca agtgctgatt caaccccaca atccacaaca 5160

gtcaagatca aaaacacaac aacaacccaa atattaccta gcaaacccac cacaaaacaa 5220

cgccaaaata aaccacaaaa caaacccaac aatgattttc actttgaagt gttcaatttt 5280

gtaccctgca gcatatgcag caacaatcca acctgctggg ccatctgcaa gagaatacca 5340

aacaaaaaac ctggaaagaa aaccaccacc aagcccacaa aaaaaccaac cctcaagaca 5400

accaaaaaag atcccaaatc ccaaaccaca aaaccaaagg aagtactcac taccaagcct 5460

acaggaaagc caaccatcaa caccactaaa acaaacatca gaactacact gctcacctcc 5520

aacaccaaag gaaatccaga acacacaagt caagaggaaa ccctccactc aaccacctcc 5580

gaaggctatc caagcccatc acaagtccac acaacatccg gtcaagagga aaccctccac 5640

tcaaccacct ccgaaggcta tccaagccca tcacaagtct acacaacatc cgagtaccta 5700

tcacaatctc tatcttcatc caacacaaca aaatgatagt cattaaaaag cacgcgtgta 5760

ttgttgcaaa aagccatgac caaatcaaac agaatcaaaa tcaactctgg ggcaaataac 5820

aatggagttg ccaatcctca aaacaaatgc tattaccaca atccttgctg cagtcacact 5880

ctgtttcgct tccagtcaaa acatcactga agaattttat caatcaacat gcagtgcagt 5940

cagcaaaggc tatcttagtg ctctaagaac tggttggtat actagtgtta taactataga 6000

attaagtaat atcaaggaaa ataagtgtaa tggtacagac gctaaggtaa aattaataaa 6060

acaagaatta gataaatata aaaatgctgt aacagaattg cagttgctca tgcaaagcac 6120

accagcagcc aacaatcgag ccagaagaga actaccaaga tttatgaatt atacactcaa 6180

caataccaaa aacaccaatg taacattaag taagaaaagg aaaagaagat ttcttggatt 6240

tttgttaggt gttggatctg caatcgccag tggcattgcc gtatccaagg tcctgcacct 6300

agaaggggaa gtgaacaaaa tcaaaagtgc tctactatcc acaaacaagg ctgtagtcag 6360

cttatctaat ggagtcagtg tcttaaccag caaggtgtta gacctcaaaa actatataga 6420

taaacagttg ttacctattg ttaacaagca aagctgcagc atatcaaaca ttgaaactgt 6480

gatagagttc caacaaaaga acaacagact actagagatt accagagaat ttagtgttaa 6540

tgcaggtgta actactcctg taagcactta tatgttaact aatagtgagt tattatcatt 6600

aatcaatgat atgcctataa caaatgatca gaaaaagtta atgtccagca atgttcaaat 6660

agttagacag caaagttact ctatcatgtc aataataaaa gaggaagtct tggcatatgt 6720

agtacaatta ccactatatg gtgtaataga tactccttgt tggaaactac acacatcccc 6780

tttatgtaca accaacacaa aggaaggatc caacatctgc ttaacaagaa ccgacagagg 6840

atggtactgt gacaatgcag gatcagtatc ctttttccca caagctgaaa catgtaaagt 6900

tcaatcgaat cgggtgtttt gtgacacaat gaacagttta acattaccaa gtgaggtaaa 6960

tctctgcaac attgacatat tcaaccccaa atatgattgc aaaattatga cttcaaaaac 7020

agatgtaagc agctccgtta tcacatctct aggagccatt gtgtcatgct atggcaaaac 7080

caaatgtaca gcatccaata aaaatcgtgg gatcataaag acattctcta acgggtgtga 7140

ttatgtatca aataaggggg tggatactgt gtctgtaggt aatacattat attatgtaaa 7200

taagcaagaa ggcaaaagtc tctatgtaaa aggtgaacca ataataaatt tctatgatcc 7260

attagtgttc ccctctgatg aatttgatgc atcaatatct caagtcaatg agaaaattaa 7320

tcagagtcta gcatttatcc gtaaatcaga tgaattatta cataatgtaa atgctggtaa 7380

atccaccaca aatatcatga taactaccat aattatagta attatagtaa tattgttagc 7440

attaattgca gttggactgc ttctatactg caaggccaga agcacaccag tcacattaag 7500

taaggatcaa ctgagtggta taaataatat tgcatttagt aactgaataa aaatagcacc 7560

taatcatatt cttacaatgg ttcgctattt gaccatagat aacccatcta tcattagatt 7620

atcctaaaat ttgaacttca tcacaacttt catctataaa ccatctcact tacacttttt 7680

aagtagattt ctattttata gttatataaa acagggccca ttgaatacca aattaactta 7740

ctatttgtaa aaatgagaat tggggcaaat atgtcacgaa ggaatccttg caaattcgaa 7800

attcgaggtc attgcttgaa tggtaaaagg tgtcatttta gtcataatta ttttgaatgg 7860

ccaccccatg cactgcttgt aagacaaaac tttatgttaa acagaatact taagtctatg 7920

gataaaagca tagatacttt gtcagaaata agtggagctg cagagttgga cagaacagaa 7980

gagtatgccc tcggtgtagt tggagtgcta gagagttata taggatcaat aaataatata 8040

actaaacaat cagcatgtgt tgccatgagc aaactcctta ctgaactcaa cagcgatgac 8100

atcaaaaaac taagggacaa tgaagagcca aactcaccca aagtaagagt gtacaatact 8160

gtcatatcat atattgaaag caacaggaag aacaataaac aaactatcca tctgttaaaa 8220

agattgccag cagacgtatt gaagaaaacc atcaaaaaca cattggatat ccacaagagc 8280

ataaccatca ataacccaaa agaatcaact gttagtgata cgaacgacca tgccaaaaat 8340

aatgatacta cctgacaaat atccttgtag tataaattcc atactaataa caagtaattg 8400

tagagtcact atgtataatc aaaaaaacac actatatatc aatcaaaaca accaaaatag 8460

ccatatatac ccaccggatc aaccattcaa tgaaatccat tggacctctc aagacttgat 8520

tgatgcaact caaaattttc tacaacatct aggtattact gatgatatat acacaatata 8580

tatattagtg tcataatact caatcctaat acttaccaca tcatcaaatt attaactcaa 8640

acaattcaag ctatgggaca aaatggatcc cattattagt ggaaattctg ctaatgttta 8700

tctaactgat agttatttaa aaggtgttat ttctttctca gaatgtaacg ctttaggaag 8760

ttacatattc aatggtcctt atctcaaaaa tgattatacc aacttaatta gtagacaaaa 8820

tccattaata gaacacataa atctaaagaa actaaatata acacagtcct taatatctaa 8880

gtatcataaa ggtgaaataa aaatagaaga acctacttac tttcagtcat tacttatgac 8940

atacaagagt atgacctctt cagaacagac tactactact aatttactta aaaagataat 9000

aagaagagct atagaaatca gtgatgtcaa agtctatgct atattgaata aactggggct 9060

caaagaaaaa gacaagatta aatccaataa tggacaagat gaagacaact cagtcattac 9120

taccataatc aaagatgata tacttttagc tgtcaaggat aatcaatctc atcttaaagc 9180

agacaaaaat caatccacaa aacaaaaaga tacaatcaaa acaacacttt tgaagaaatt 9240

aatgtgttcg atgcaacatc ctccatcatg gttaatacat tggtttaatt tatacacaaa 9300

attaaacagc atattaacac aatatcgatc tagtgaggta aaaaaccatg gttttatatt 9360

gatagataat catactctta gtggattcca atttattttg aatcaatatg gttgtatagt 9420

ttatcataag gaactcaaaa gaattactgt gacaacttat aatcaattct tgacatggaa 9480

agatattagc cttagtagat taaatgtttg tttgattaca tggattagta actgtttgaa 9540

cacattaaac aaaagcttag gcttaagatg tggattcaat aatgttatct tgacacaatt 9600

attcctttat ggagattgta tactaaaact attccacaat gaggggttct acataataaa 9660

agaggtagag ggatttatta tgtctctaat tttaaatata acagaagaag atcaattcag 9720

aaaacggttt tataatagta tgctcaacaa catcacagat gccgccaaca aagctcaaaa 9780

aaatctgcta tcaagagtat gtcatacatt attagataag acaatatcag ataatataat 9840

aaatggcaga tggataattc tattgagtaa gttcctaaaa ttaattaagc ttgcaggtga 9900

caataacctc aacaatctga gtgaattata ttttttgttc agaatatttg gacacccaat 9960

ggtagatgaa agacaagcca tggatgctgt taaagttaat tgcaacgaga ccaaatttta 10020

tttgttaagt agtttgagta tgttaagagg agcttttata tatagaatta taaaagggtt 10080

tgtaaataat tacaacagat ggcctacttt aagaaatgcc attgtcttac ccttaagatg 10140

gttaacttac tataaactaa acacttatcc ttccttgttg gaacttacag aaagagattt 10200

gattgttcta tcaggactac gtttctatcg agagtttcgg ttgcctaaaa aagtggatct 10260

tgaaatgatc ataaatgata aggctatatc acctcctaaa aatttaatat ggactagttt 10320

ccctagaaat tatatgccgt cacacataca aaattatata gaacatgaaa aattaaaatt 10380

ctctgatagt gataaatcaa gaagagtatt agagtattat ttaagagata acaaattcaa 10440

tgaatgtgat ttacacaact gtgtagttaa tcaaagttat cttaacaacc cgaatcatgt 10500

ggtatcattg acaggcaaag aaagagaact cagtgtaggt agaatgtttg caatgcaacc 10560

aggaatgttc agacaagttc aaatattagc agagaaaatg atagcagaaa acatattaca 10620

atttttccct gaaagtctta caagatatgg tgatctagaa ctacagaaaa tattagaatt 10680

gaaagcagga ataagtaaca aatcaaatcg ttacaatgat aattacaaca attacattag 10740

taagtgctct atcatcacag atctcagcaa attcaatcaa gcatttcgat atgaaacatc 10800

atgtatttgt agtgatgtac tggatgaact gcatggtgta caatctctat tttcctggtt 10860

acatttaact attcctcatg tcacaataat atgcacatat aggcatgcac ccccctatat 10920

aaaggatcat attgtagatc ttaacaatgt agatgagcaa agtggactat atagatatca 10980

tatgggtggt atcgaagggt ggtgtcaaaa actatggacc atagaagcta tatcactatt 11040

agatctaata tctctcaaag ggaaattctc aattactgct ttaattaatg gtgacaatca 11100

atcaatagat ataagtaaac cagtcagact catggaaggt caaactcatg ctcaagcaga 11160

ttatttgcta gcattaaata gtctcaaatt actgtataaa gagtatgcag gaataggcca 11220

caaattaaaa ggaactgaga cttatatatc gagagatatg caatttatga gtaaaacgat 11280

ccaacataac ggtgtatatt acccagctag tataaagaaa gtcctaagag tgggaccgtg 11340

gataaacact atacttgatg acttcaaagt gagtctagaa tctataggta gtttgacaca 11400

agaattagaa tatagaggtg aaagtctatt atgcagttta atatttagaa atgtatggtt 11460

atataatcaa attgcattac aacttaaaaa tcatgcatta tgtaacaaca aattatattt 11520

ggatatatta aaagttctaa aacacttaaa aacctttttt aatcttgata acattgatac 11580

agcattaaca ttgtatatga atttgcccat gttatttggt ggtggtgatc ccaacttgtt 11640

atatcgaagt ttctatagaa gaactcctga tttcctcaca gaggctatag ttcactctgt 11700

gttcatactt agttattata caaaccatga tttaaaagat aaacttcaag atctgtcaga 11760

tgatagattg aataagttct taacatgcat aatcacgttt gataaaaacc ccaatgctga 11820

attcgttaca ttgatgagag atcctcaagc tttaggatct gagaggcaag ctaaaattac 11880

tagcgaaatc aatagactgg cagttaccga ggttttgagc acagctccaa acaaaatatt 11940

ttccaaaagt gcacaacact ataccactac agagatagat cttaatgata ttatgcaaaa 12000

tatagaacct acatatcctc acgggctaag agttgtttat gagagtttac ccttttataa 12060

agcagagaaa atagtaaatc ttatatccgg tacaaaatct ataactaaca tactggaaaa 12120

gacttctgcc atagacttaa cagatattga tagagccact gagatgatga ggaaaaacat 12180

aactttgctt ataaggatat taccattaga ttgtaacaga gataaaagag aaatattgag 12240

tatggaaaac ctaagtatta ctgaattaag caaatacgtt agagaaagat cttggtcttt 12300

atccaatata gttggtgtta catcacccag tatcatgtat acaatggaca taaaatatac 12360

aacaagcact atagctagtg gcataatcat agagaaatat aatgtcaaca gtttaacacg 12420

tggtgagaga ggacccacta aaccatgggt tggttcatct acacaagaga aaaagacaat 12480

gccagtttat aatagacaag ttttaaccaa aaaacagaga gatcaaatag atctattagc 12540

aaaattggat tgggtgtatg catctataga taacaaggat gaatttatgg aggaacttag 12600

cataggaact cttgggttaa catatgagaa ggccaaaaaa ttattcccac aatatttgag 12660

tgttaactat ttgcatcgtc ttacagtcag tagtagacca tgtgaattcc ctgcatctat 12720

accagcttat agaactacaa attatcactt tgatactagc cctattaatc gcatattaac 12780

agaaaagtat ggtgatgaag atattgatat agtattccaa aactgtataa gctttggcct 12840

tagcttaatg tctgtagtag aacaatttac taatgtatgt cctaacagaa ttattctcat 12900

acccaagctt aatgagatac atttgatgaa acctcccata ttcacaggcg atgttgatat 12960

tcacaagtta aaacaagtga tacaaaaaca acatatgttt ttaccagaca aaataagttt 13020

gactcaatat gtggaattat tcttaagtaa taaaacactc aaatctggat ctaatgttaa 13080

ttctaattta atattggcgc ataagatatc tgactatttt cataatactt acattttgag 13140

tactaattta gctggacatt ggattcttat tatacaactt atgaaagatt ctaagggtat 13200

ttttgaaaaa gattggggag agggatatat aactgatcat atgttcatta atttgaaagt 13260

tttcttcaat gcttataaga catatctctt gtgttttcat aaaggttacg gcagagcaaa 13320

gctggagtgt gatatgaata cttcagatct cctatgtgta ttggaattaa tagacagtag 13380

ttattggaag tctatgtcta aggtgttttt agaacaaaaa gttatcaaat acattcttag 13440

ccaggatgca agtttacata gagtaaaagg atgtcatagc ttcaaactat ggtttcttaa 13500

acgtcttaat gtagcagaat tcacagtttg cccttgggtt gttaacatag attatcatcc 13560

aacacatatg aaagcaatat taacttatat tgatcttgtt agaatgggat tgataaatat 13620

agatagaata tacattaaaa ataaacacaa gttcaatgat gagttttata cttctaatct 13680

gttttacatt aattataact tctcagataa tactcatcta ttaactaaac atataaggat 13740

tgctaattcc gaattagaaa gtaattacaa caaattatat catcctacac cagaaaccct 13800

agaaaatata ctaaccaatc cggttaaaag taatgagaaa aagacactga gtgactattg 13860

tataggtaaa aatgttgact caataatgtt accatcgtta tctaataaga agcttattaa 13920

atcgtctaca atgattagaa ccaattacag cagacaagat ttgtataatt tatttcctac 13980

ggttgtgatt gataaaatta tagatcattc aggtaataca gccaaatcta accaacttta 14040

cactactact tctcatcaaa tatccttagt gcacaatagc acatcacttt attgcatgct 14100

tccttggcat catattaata gattcaattt tgtatttagt tctacaggtt gtaaaattag 14160

tatagagtat attttaaaag atcttaaaat taaggatcct aattgtatag cattcatagg 14220

tgaaggagca gggaatttat tattgcgtac agtagtggaa cttcatcctg atataagata 14280

tatttacaga agtctgaaag attgcaatga tcatagttta ccaattgagt ttttaaggct 14340

gtacaatgga catatcaaca ttgattatgg tgaaaatttg accattcctg ctacagatgc 14400

aaccaacaac attcattggt cttatttaca tataaagttt gctgaaccta tcagtctttt 14460

tgtctgtgat gctgaattgc ctgtaacagt caactggagt aagattataa tagagtggag 14520

caagcatgta agaaaatgca agtactgttc ttcagttaat aaatgtacat tgatagtaaa 14580

atatcatgct caagatgata tcgatttcaa attagacaac ataactatat taaaaactta 14640

tgtatgctta ggtagtaagt taaagggatc tgaagtttac ttagtcctta caataggtcc 14700

tgcaaatgtg ttcccagtat ttaatgtagt acaaaatgct aaattgatac tatcaagaac 14760

taaaaatttc atcatgccta aaaaagctga taaagagtct attgatgcaa atattaagag 14820

tttgataccc tttctttgtt accctataac aaaaaaagga attaatactg cattgtctaa 14880

attaaagagt gttgttagtg gagatatact atcatattct atagctggac gtaatgaagt 14940

tttcagcaat aaacttataa atcataagca tatgaacatc ttaaagtggt tcaatcatgt 15000

tttaaatttc agatcaacag aattaaacta taatcattta tatatggtag aatctactta 15060

tcctcatcta agtgaattgt taaacagctt gacaaccaat gaacttaaaa aactgattaa 15120

aatcacaggt agtttgttat acaactttta taatgaataa tgagcaaaaa tcttataaca 15180

aaaatagcta cacactaaca ttgtattcaa ttatagttat tgaaaattaa taattatata 15240

atttttaata acttctagtg aactaatcct aaaattatca ttttgatcta ggaagaataa 15300

gtttaaatcc aaatctaatt ggtttatatg tatattaact aaattacgag atattagttt 15360

ttgacacttt ttttctcgtg gccggcatgg tcccagcctc ctcgctggcg ccggctgggc 15420

aacatgcttc ggcatggcga atgggactag cataacccct tggggcctct aaacgggtct 15480

tgaggggttt tttg 15494

<210>    4
<211>    15398
<212>    DNA
<213>    Artificial Sequence

<220>
<223>    cRSVA_MVF_S


<400>    4
   taatacgact cactataggt tttttcgcgt ctgatgaggc cgttaggccg aaactcctct      60
   ccggagtcac gcgaaaaaat gcgtacaaca aacttgcgta aaccaaaaaa atggggcaaa     120
   taagaatttg ataagtacca cttaaattta actcctttgg ttagaggcgc gccatgggca     180
   gcaactcatt gagtatgata aaagttagat tgcaaaatct gtttgacaat gatgaagtag     240
   cattgttaaa aataacatgc tatactgaca aattaataca gttaactaat gctttggcta     300
   aggcagttat acatacaatc aaattgaatg gcattgtatt tgtgcatgtt attacaagta     360
   gtgatatttg ccctaataat aatattgtag tgaaatccaa tttcacaaca atgccagtat     420
   tacaaaatgg aggttatata tgggaaatga tggaattaac acactgctct caacctaatg     480
   gcctaataga tgacaattgt gaaattaaat tctccaaaaa actaagtgat tcaacaatga     540
   ccaattatat gaatcaatta tctgaattac ttggatttga cctcaatcca taaatcataa     600
   taaatatcaa ctagcaaatc aatgtcacta acaccattag ttaatataaa acttgacaga     660
   agataaaaat ggggcaaata aatcaattca gccgacccaa ccatggacac aacacacaat     720
   gataccacac cacaaagact gatgatcaca gacatgaggc cattatcgct tgagactata     780
   ataacatctc taaccagaga tatcataaca cataaattta tatacttgat aaatcatgaa     840
   tgcatagtaa gaaaacttga tgaaagacag gccacattta catttctggt caactatgaa     900
   atgaaactat tgcacaaagt gggaagcact aaatataaaa aatatactga atacaacaca     960
   aaatatggca ctttccctat gccaatattt atcaatcatg atgggttctt agaatgcatt    1020
   ggcattaagc ctaccaagca cacacccata atatacaagt atgatctcaa tccatgaata    1080
   tcaaaccaag attcaaacaa tccgaaataa caactttatg cataatcaca ctccatagtc    1140
   caaatggagc ctgaaaatta tagttattta aaattcctgc aggaaggaga gacataagat    1200
   gaaagatggg gcaaatacaa aaatggctct tagcaaagtc aagttgaatg atacactcaa    1260
   caaagatcaa cttctatcat ccagcaaata taccatccaa cggagcacag gagacagcat    1320
   tgacactcct aattatgatg tgcagaaaca cattaataag ttatgtggca tgttattaat    1380
   cacagaagat gctaatcata aattcactgg gttaataggt atgttatatg ctatgtctag    1440
   attaggaaga gaagacacca taaaaatact caaagatgcg ggatatcatg ttaaggcaaa    1500
   tggagtggat gtaacaacac atcgtcaaga cattaatggg aaagaaatga aatttgaagt    1560
   gttaacatta gcaagcttaa caactgaaat tcaaatcaac attgagatag aatctagaaa 1620
   atcctacaaa aaaatgctaa aagaaatggg agaggtggct ccagaataca ggcatgactc 1680
   tcctgattgt gggatgataa tattatgtat agcggcatta gtaataacca aattagcagc 1740
   aggagataga tcaggtctta cagctgtgat taggagagct aataatgtcc taaaaaatga 1800
   aatgaaacgt tataaaggtt tattacccaa ggatatagcc aacagcttct atgaagtgtt 1860
   tgaaaaatat cctcacttta tagatgtttt tgttcatttt ggtatagcac aatcttctac 1920
   cagaggtggc agtagagttg aagggatttt tgcaggattg tttatgaatg cctatggtgc 1980
   agggcaagtg atgttacggt ggggggtctt agcaaaatca gttaaaaaca ttatgttagg 2040
   acacgctagt gtacaagcag aaatggaaca agttgtggag gtgtatgagt atgctcagaa 2100
   attgggtgga gaagcaggat tctaccatat attgaacaac ccaaaagcat cactattatc 2160
   tttgactcaa tttcctcact tctctagtgt agtattgggc aatgctgctg gcctaggcat 2220
   aatgggagaa tacagaggta caccaaggaa tcaagattta tatgatgctg caaaagcata 2280
   tgctgaacaa ctcaaagaaa atggtgtgat taactacagt gtattagatt tgacagcaga 2340
   agaactagag gctatcaaac atcagcttaa tccaaaagat aatgatgtag agctttgagt 2400
   taataaaaaa gtggggcaaa taaatcatca tggaaaagtt tgctcctgaa ttccatggag 2460
   aagatgcaaa caacagagcc accaaattcc tagaatcaat aaagggcaaa ttcacatcac 2520
   ccaaagatcc caagaaaaaa gatagtatca tatctgtcaa ctcaatagat atagaagtaa 2580
   ccaaagaaag ccctataaca tcaaattcaa ccattataaa cccaataaat gagacagatg 2640
   atactgtagg gaacaagccc aattatcaaa gaaagcctct agtaagtttc aaagaagacc 2700
   ctacgccaag tgataatcct ttttcaaaac tatacaaaga aaccatagaa acatttgata 2760
   acaatgaaga agaatctagc tattcatatg aagaaataaa tgatcagaca aacgataata 2820
   taacagcaag attagatagg attgatgaga aattaagtga aatactagga atgcttcaca 2880
   cattagtagt agcgagtgca ggacccacat ctgctcggga tggtataaga gatgccatgg 2940
   ttggtttaag agaagaaatg atagaaaaaa tcagaactga agcattaatg accaatgaca 3000
   gactagaagc tatggcaaga ctcaggaatg aagaaagtga aaagatggca aaagacacat 3060
   cagatgaagt gtctctcaat ccaacatcag agaaactgaa caacctgttg gaagggaatg 3120
   atagtgacaa tgatctatca cttgaagatt tctgattagc taccaaactg tacatcaaaa 3180
   cacaacacca atagaaaacc aacaaacaaa ccaactcacc catccaacca aacatctatc 3240
   tgctgattag ccaaccagcc aaaaaacaac cagccaatct aaaactagcc acccggaaaa 3300
   aatcgatact atagttacaa aaaaagatgg ggcaaatatg gaaacatacg tgaataaact 3360
   tcacgagggc tccacataca cagctgctgt tcaatacaat gtcctagaaa aagacgatga 3420
   tcctgcatca cttacaatat gggtgcccat gttccaatca tccatgccag cagatctact 3480
   cataaaagaa ctagccaatg tcaatatact agtgaaacaa atatccacac ccaaggaccc 3540
   ctcattaaga gtcatgataa actcaagaag tgcagtgcta gcacaaatgc ccagcaaatt 3600
   taccatatgt gccaatgtgt ccttggatga aagaagcaag ctggcatatg atgtaaccac 3660
   accctgtgaa attaaggcat gcagtctaac atgcctaaaa tcaaaaaata tgttaactac 3720
   agttaaagat ctcactatga aaacactcaa cccaacacat gacatcattg ctttatgtga 3780
   atttgaaaat atagtaacat caaaaaaagt cataatacca acatacctaa gatctatcag 3840
   cgtcagaaat aaagatctga acacacttga aaatataaca accactgaat tcaaaaatgc 3900
   cattacaaat gcaaaaatca tcccttactc aggattattg ttagtcatca cagtgactga 3960
   caacaaagga gcattcaaat acataaagcc acaaagtcaa ttcatagtag atcttggagc 4020
   ttacctagaa aaagaaagta tatattatgt tacaacaaat tggaagcaca cagctacacg 4080
   atttgcaatc aaacccatgg aagattaacc tttttcctct acatcaatga gtagattcat 4140
   acaaactttc taactacatt cttcacttca caatcataat caccaaccct ctgtggttca 4200
   atcaatcaaa caaaactcat caagagttcc agatcatccc aagtcattgt tcatcagatc 4260
   cagtactcaa ataagttaat aaaaaatcca catggggcaa ataatcattg agggaaatcc 4320
   aactaatcac aacatctgtc aacatagaca agtcaacacg ctagataaaa tcaaccaatg 4380
   gaaaatacat ccataactat agaattctca agcaaattct ggccttactt tacactaata 4440
   cacatgataa caacaataat ctctttgata atcataatct ccatcatgat tgcaatacta 4500
   aacaaactct gcgaatataa tgtattccat aacaaaacct ttgagctacc aagagctcga 4560
   gtcaatacat agcattcacc aatctgatag ctcaaaacag taaccttgca tttgtaaatg 4620
   aactaccctc acttcttcac aaaaccacat caacatctca ccatgcaagc catcatctat 4680
   accataaagt agttaattaa aaatggccgg ccagtcataa caatgaacta ggatattaag 4740
   accaaaaaca acgctggggc aaatgcaaac atgtccaaaa ccgaggacca acgcaccgcc 4800
   aagacactag aaaggacctg ggacactttt aatcatctat tattcatatc atcgtgctta 4860
   tacaagttaa atcttaaatc tatagcacaa atcacattat ctattttggc aattataatc 4920
   tcaacctcac ttataattgc agccatcata ttcatagcct cggcaaacca caaagtcaca 4980
   ctaacaactg caatcataca agatgcaacg aaccagatca agaacacaac cccaacatac 5040
   ctcacccaga atccccagca tggaatcagc ttctccaatc tgtccggaac tacatcacaa 5100
   tccaccacca tactagcttc aacaacacca agtgctgatt caaccccaca atccacaaca 5160
   gtcaagatca aaaacacaac aacaacccaa atattaccta gcaaacccac cacaaaacaa 5220
   cgccaaaata aaccacaaaa caaacccaac aatgattttc actttgaagt gttcaatttt 5280
   gtaccctgca gcatatgcag caacaatcca acctgctggg ccatctgcaa gagaatacca 5340
   aacaaaaaac ctggaaagaa aaccaccacc aagcccacaa aaaaaccaac cctcaagaca 5400
   accaaaaaag atcccaaatc ccaaaccaca aaaccaaagg aagtactcac taccaagcct 5460
   acaggaaagc caaccatcaa caccactaaa acaaacatca gaactacact gctcacctcc 5520
   aacaccaaag gaaatccaga acacacaagt caagaggaaa ccctccactc aaccacctcc 5580
   gaaggctatc caagcccatc acaagtccac acaacatccg gtcaagagga aaccctccac 5640
   tcaaccacct ccgaaggcta tccaagccca tcacaagtct acacaacatc cgagtaccta 5700
   tcacaatctc tatcttcatc caacacaaca aaatgatagt cattaaaaag cacgcgtgta 5760
   ttgttgcaaa aagccatgac caaatcaaac agaatcaaaa tcaactctgg ggcaaataac 5820
   aatgtccatc atgggtctca aggtgaacgt ctctgccata ttcatggcag tactgttaac 5880
   tctccaaaca cccaccggtc aaatccattg gggcaatctc tctaagatag gggtggtagg 5940
   aataggaagt gcaagctaca aagttatgac tcgttccagc catcaatcat tagtcataaa 6000
   attaatgccc aatataactc tcctcaataa ctgcacgagg gtagagattg cagaatacag 6060
   gagactactg agaacagttt tggaaccaat tagagatgca cttaatgcaa tgacccagaa 6120
   tataagaccg gttcagagtg tagcttcaag taggagacac aagagatttg cgggagtagt 6180
   cctggcaggt gcggccctag gcgttgccac agctgctcag ataacagccg gcattgcact 6240
   tcaccagtcc atgctgaact ctcaagccat cgacaatctg agagcgagcc tggaaactac 6300
   taatcaggca attgagacaa tcagacaagc agggcaggag atgatattgg ctgttcaggg 6360
   tgtccaagac tacatcaata atgagctgat accgtctatg aaccaactat cttgtgattt 6420
   aatcggccag aagctcgggc tcaaattgct cagatactat acagaaatcc tgtcattatt 6480
   tggccccagt ttacggaccc ccatatctgc ggagatatct atccaggctt tgagctatgc 6540
   gcttggagga gacatcaata aggtgttaga aaagctcgga tacagtggag gtgatttact 6600
   gggcatctta gagagcggag gaataaaggc ccggataact cacgtcgaca cagagtccta 6660
   cttcattgtc ctcagtatag cctatccgac gctgtccgag attaaggggg tgattgtcca 6720
   ccggctagag ggggtctcgt acaacatagg ctctcaagag tggtatacca ctgtgcccaa 6780
   gtatgttgca acccaagggt accttatctc gaattttgat gagtcatcgt gtactttcat 6840
   gccagagggg actgtgtgca gccaaaatgc cttgtacccg atgagtcctc tgctccaaga 6900
   atgcctccgg gggtacacca agtcctgtgc tcgtacactc gtatccgggt cttttgggaa 6960
   ccggttcatt ttatcacaag ggaacctaat agccaattgt gcatcaatcc tttgcaagtg 7020
   ttacacaaca ggaacgatca ttaatcaaga ccctgacaag atcctaacat acattgctgc 7080
   cgatcactgc ccggtagtcg aggtgaacgg cgtgaccatc caagtcggga gcaggaggta 7140
   tccagacgct gtgtacttgc acagaattga cctcggtcct cccatatcat tggagaggtt 7200
   ggacgtaggg acaaatctgg ggaatgcaat tgctaagttg gaggatgcca aggaattgtt 7260
   ggagtcatcg gaccagatat tgaggagtat gaaaggttta tcgagcacta gcataattat 7320
   agtaattata gtaatattgt tagcattaat tgcagttgga ctgcttctat actgcaaggc 7380
   cagaagcaca ccagtcacat taagtaagga tcaactgagt ggtataaata atattgcatt 7440
   tagtaactga ataaaaatag cacctaatca tattcttaca atggttcgct atttgaccat 7500
   agataaccca tctatcatta gattatccta aaatttgaac ttcatcacaa ctttcatcta 7560
   taaaccatct cacttacact ttttaagtag atttctattt tatagttata taaaacaggg 7620
   cccattgaat accaaattaa cttactattt gtaaaaatga gaattggggc aaatatgtca 7680
   cgaaggaatc cttgcaaatt cgaaattcga ggtcattgct tgaatggtaa aaggtgtcat 7740
   tttagtcata attattttga atggccaccc catgcactgc ttgtaagaca aaactttatg 7800
   ttaaacagaa tacttaagtc tatggataaa agcatagata ctttgtcaga aataagtgga 7860
   gctgcagagt tggacagaac agaagagtat gccctcggtg tagttggagt gctagagagt 7920
   tatataggat caataaataa tataactaaa caatcagcat gtgttgccat gagcaaactc 7980
   cttactgaac tcaacagcga tgacatcaaa aaactaaggg acaatgaaga gccaaactca 8040
   cccaaagtaa gagtgtacaa tactgtcata tcatatattg aaagcaacag gaagaacaat 8100
   aaacaaacta tccatctgtt aaaaagattg ccagcagacg tattgaagaa aaccatcaaa 8160
   aacacattgg atatccacaa gagcataacc atcaataacc caaaagaatc aactgttagt 8220
   gatacgaacg accatgccaa aaataatgat actacctgac aaatatcctt gtagtataaa 8280
   ttccatacta ataacaagta attgtagagt cactatgtat aatcaaaaaa acacactata 8340
   tatcaatcaa aacaaccaaa atagccatat atacccaccg gatcaaccat tcaatgaaat 8400
   ccattggacc tctcaagact tgattgatgc aactcaaaat tttctacaac atctaggtat 8460
   tactgatgat atatacacaa tatatatatt agtgtcataa tactcaatcc taatacttac 8520
   cacatcatca aattattaac tcaaacaatt caagctatgg gacaaaatgg atcccattat 8580
   tagtggaaat tctgctaatg tttatctaac tgatagttat ttaaaaggtg ttatttcttt 8640
   ctcagaatgt aacgctttag gaagttacat attcaatggt ccttatctca aaaatgatta 8700
   taccaactta attagtagac aaaatccatt aatagaacac ataaatctaa agaaactaaa 8760
   tataacacag tccttaatat ctaagtatca taaaggtgaa ataaaaatag aagaacctac 8820
   ttactttcag tcattactta tgacatacaa gagtatgacc tcttcagaac agactactac 8880
   tactaattta cttaaaaaga taataagaag agctatagaa atcagtgatg tcaaagtcta 8940
   tgctatattg aataaactgg ggctcaaaga aaaagacaag attaaatcca ataatggaca 9000
   agatgaagac aactcagtca ttactaccat aatcaaagat gatatacttt tagctgtcaa 9060
   ggataatcaa tctcatctta aagcagacaa aaatcaatcc acaaaacaaa aagatacaat 9120
   caaaacaaca cttttgaaga aattaatgtg ttcgatgcaa catcctccat catggttaat 9180
   acattggttt aatttataca caaaattaaa cagcatatta acacaatatc gatctagtga 9240
   ggtaaaaaac catggtttta tattgataga taatcatact cttagtggat tccaatttat 9300
   tttgaatcaa tatggttgta tagtttatca taaggaactc aaaagaatta ctgtgacaac 9360
   ttataatcaa ttcttgacat ggaaagatat tagccttagt agattaaatg tttgtttgat 9420
   tacatggatt agtaactgtt tgaacacatt aaacaaaagc ttaggcttaa gatgtggatt 9480
   caataatgtt atcttgacac aattattcct ttatggagat tgtatactaa aactattcca 9540
   caatgagggg ttctacataa taaaagaggt agagggattt attatgtctc taattttaaa 9600
   tataacagaa gaagatcaat tcagaaaacg gttttataat agtatgctca acaacatcac 9660
   agatgccgcc aacaaagctc aaaaaaatct gctatcaaga gtatgtcata cattattaga 9720
   taagacaata tcagataata taataaatgg cagatggata attctattga gtaagttcct 9780
   aaaattaatt aagcttgcag gtgacaataa cctcaacaat ctgagtgaat tatatttttt 9840
   gttcagaata tttggacacc caatggtaga tgaaagacaa gccatggatg ctgttaaagt 9900
   taattgcaac gagaccaaat tttatttgtt aagtagtttg agtatgttaa gaggagcttt 9960
   tatatataga attataaaag ggtttgtaaa taattacaac agatggccta ctttaagaaa 10020
   tgccattgtc ttacccttaa gatggttaac ttactataaa ctaaacactt atccttcctt 10080
   gttggaactt acagaaagag atttgattgt tctatcagga ctacgtttct atcgagagtt 10140
   tcggttgcct aaaaaagtgg atcttgaaat gatcataaat gataaggcta tatcacctcc 10200
   taaaaattta atatggacta gtttccctag aaattatatg ccgtcacaca tacaaaatta 10260
   tatagaacat gaaaaattaa aattctctga tagtgataaa tcaagaagag tattagagta 10320
   ttatttaaga gataacaaat tcaatgaatg tgatttacac aactgtgtag ttaatcaaag 10380
   ttatcttaac aacccgaatc atgtggtatc attgacaggc aaagaaagag aactcagtgt 10440
   aggtagaatg tttgcaatgc aaccaggaat gttcagacaa gttcaaatat tagcagagaa 10500
   aatgatagca gaaaacatat tacaattttt ccctgaaagt cttacaagat atggtgatct 10560
   agaactacag aaaatattag aattgaaagc aggaataagt aacaaatcaa atcgttacaa 10620
   tgataattac aacaattaca ttagtaagtg ctctatcatc acagatctca gcaaattcaa 10680
   tcaagcattt cgatatgaaa catcatgtat ttgtagtgat gtactggatg aactgcatgg 10740
   tgtacaatct ctattttcct ggttacattt aactattcct catgtcacaa taatatgcac 10800
   atataggcat gcacccccct atataaagga tcatattgta gatcttaaca atgtagatga 10860
   gcaaagtgga ctatatagat atcatatggg tggtatcgaa gggtggtgtc aaaaactatg 10920
   gaccatagaa gctatatcac tattagatct aatatctctc aaagggaaat tctcaattac 10980
   tgctttaatt aatggtgaca atcaatcaat agatataagt aaaccagtca gactcatgga 11040
   aggtcaaact catgctcaag cagattattt gctagcatta aatagtctca aattactgta 11100
   taaagagtat gcaggaatag gccacaaatt aaaaggaact gagacttata tatcgagaga 11160
   tatgcaattt atgagtaaaa cgatccaaca taacggtgta tattacccag ctagtataaa 11220
   gaaagtccta agagtgggac cgtggataaa cactatactt gatgacttca aagtgagtct 11280
   agaatctata ggtagtttga cacaagaatt agaatataga ggtgaaagtc tattatgcag 11340
   tttaatattt agaaatgtat ggttatataa tcaaattgca ttacaactta aaaatcatgc 11400
   attatgtaac aacaaattat atttggatat attaaaagtt ctaaaacact taaaaacctt 11460
   ttttaatctt gataacattg atacagcatt aacattgtat atgaatttgc ccatgttatt 11520
   tggtggtggt gatcccaact tgttatatcg aagtttctat agaagaactc ctgatttcct 11580
   cacagaggct atagttcact ctgtgttcat acttagttat tatacaaacc atgatttaaa 11640
   agataaactt caagatctgt cagatgatag attgaataag ttcttaacat gcataatcac 11700
   gtttgataaa aaccccaatg ctgaattcgt tacattgatg agagatcctc aagctttagg 11760
   atctgagagg caagctaaaa ttactagcga aatcaataga ctggcagtta ccgaggtttt 11820
   gagcacagct ccaaacaaaa tattttccaa aagtgcacaa cactatacca ctacagagat 11880
   agatcttaat gatattatgc aaaatataga acctacatat cctcacgggc taagagttgt 11940
   ttatgagagt ttaccctttt ataaagcaga gaaaatagta aatcttatat ccggtacaaa 12000
   atctataact aacatactgg aaaagacttc tgccatagac ttaacagata ttgatagagc 12060
   cactgagatg atgaggaaaa acataacttt gcttataagg atattaccat tagattgtaa 12120
   cagagataaa agagaaatat tgagtatgga aaacctaagt attactgaat taagcaaata 12180
   cgttagagaa agatcttggt ctttatccaa tatagttggt gttacatcac ccagtatcat 12240
   gtatacaatg gacataaaat atacaacaag cactatagct agtggcataa tcatagagaa 12300
   atataatgtc aacagtttaa cacgtggtga gagaggaccc actaaaccat gggttggttc 12360
   atctacacaa gagaaaaaga caatgccagt ttataataga caagttttaa ccaaaaaaca 12420
   gagagatcaa atagatctat tagcaaaatt ggattgggtg tatgcatcta tagataacaa 12480
   ggatgaattt atggaggaac ttagcatagg aactcttggg ttaacatatg agaaggccaa 12540
   aaaattattc ccacaatatt tgagtgttaa ctatttgcat cgtcttacag tcagtagtag 12600
   accatgtgaa ttccctgcat ctataccagc ttatagaact acaaattatc actttgatac 12660
   tagccctatt aatcgcatat taacagaaaa gtatggtgat gaagatattg atatagtatt 12720
   ccaaaactgt ataagctttg gccttagctt aatgtctgta gtagaacaat ttactaatgt 12780
   atgtcctaac agaattattc tcatacccaa gcttaatgag atacatttga tgaaacctcc 12840
   catattcaca ggcgatgttg atattcacaa gttaaaacaa gtgatacaaa aacaacatat 12900
   gtttttacca gacaaaataa gtttgactca atatgtggaa ttattcttaa gtaataaaac 12960
   actcaaatct ggatctaatg ttaattctaa tttaatattg gcgcataaga tatctgacta 13020
   ttttcataat acttacattt tgagtactaa tttagctgga cattggattc ttattataca 13080
   acttatgaaa gattctaagg gtatttttga aaaagattgg ggagagggat atataactga 13140
   tcatatgttc attaatttga aagttttctt caatgcttat aagacatatc tcttgtgttt 13200
   tcataaaggt tacggcagag caaagctgga gtgtgatatg aatacttcag atctcctatg 13260
   tgtattggaa ttaatagaca gtagttattg gaagtctatg tctaaggtgt ttttagaaca 13320
   aaaagttatc aaatacattc ttagccagga tgcaagttta catagagtaa aaggatgtca 13380
   tagcttcaaa ctatggtttc ttaaacgtct taatgtagca gaattcacag tttgcccttg 13440
   ggttgttaac atagattatc atccaacaca tatgaaagca atattaactt atattgatct 13500
   tgttagaatg ggattgataa atatagatag aatatacatt aaaaataaac acaagttcaa 13560
   tgatgagttt tatacttcta atctgtttta cattaattat aacttctcag ataatactca 13620
   tctattaact aaacatataa ggattgctaa ttccgaatta gaaagtaatt acaacaaatt 13680
   atatcatcct acaccagaaa ccctagaaaa tatactaacc aatccggtta aaagtaatga 13740
   gaaaaagaca ctgagtgact attgtatagg taaaaatgtt gactcaataa tgttaccatc 13800
   gttatctaat aagaagctta ttaaatcgtc tacaatgatt agaaccaatt acagcagaca 13860
   agatttgtat aatttatttc ctacggttgt gattgataaa attatagatc attcaggtaa 13920
   tacagccaaa tctaaccaac tttacactac tacttctcat caaatatcct tagtgcacaa 13980
   tagcacatca ctttattgca tgcttccttg gcatcatatt aatagattca attttgtatt 14040
   tagttctaca ggttgtaaaa ttagtataga gtatatttta aaagatctta aaattaagga 14100
   tcctaattgt atagcattca taggtgaagg agcagggaat ttattattgc gtacagtagt 14160
   ggaacttcat cctgatataa gatatattta cagaagtctg aaagattgca atgatcatag 14220
   tttaccaatt gagtttttaa ggctgtacaa tggacatatc aacattgatt atggtgaaaa 14280
   tttgaccatt cctgctacag atgcaaccaa caacattcat tggtcttatt tacatataaa 14340
   gtttgctgaa cctatcagtc tttttgtctg tgatgctgaa ttgcctgtaa cagtcaactg 14400
   gagtaagatt ataatagagt ggagcaagca tgtaagaaaa tgcaagtact gttcttcagt 14460
   taataaatgt acattgatag taaaatatca tgctcaagat gatatcgatt tcaaattaga 14520
   caacataact atattaaaaa cttatgtatg cttaggtagt aagttaaagg gatctgaagt 14580
   ttacttagtc cttacaatag gtcctgcaaa tgtgttccca gtatttaatg tagtacaaaa 14640
   tgctaaattg atactatcaa gaactaaaaa tttcatcatg cctaaaaaag ctgataaaga 14700
   gtctattgat gcaaatatta agagtttgat accctttctt tgttacccta taacaaaaaa 14760
   aggaattaat actgcattgt ctaaattaaa gagtgttgtt agtggagata tactatcata 14820
   ttctatagct ggacgtaatg aagttttcag caataaactt ataaatcata agcatatgaa 14880
   catcttaaag tggttcaatc atgttttaaa tttcagatca acagaattaa actataatca 14940
   tttatatatg gtagaatcta cttatcctca tctaagtgaa ttgttaaaca gcttgacaac 15000
   caatgaactt aaaaaactga ttaaaatcac aggtagtttg ttatacaact tttataatga 15060
   ataatgagca aaaatcttat aacaaaaata gctacacact aacattgtat tcaattatag 15120
   ttattgaaaa ttaataatta tataattttt aataacttct agtgaactaa tcctaaaatt 15180
   atcattttga tctaggaaga ataagtttaa atccaaatct aattggttta tatgtatatt 15240
   aactaaatta cgagatatta gtttttgaca ctttttttct cgtggccggc atggtcccag 15300
   cctcctcgct ggcgccggct gggcaacatg cttcggcatg gcgaatggga ctagcataac 15360
   cccttggggc ctctaaacgg gtcttgaggg gttttttg 15398

<210> 5
<211> 15407
<212> DNA
<213> Artificial Sequence

<220>
<223> cRSVA_NDVF_S

<400> 5
taatacgact cactataggt tttttcgcgt ctgatgaggc cgttaggccg aaactcctct 60

ccggagtcac gcgaaaaaat gcgtacaaca aacttgcgta aaccaaaaaa atggggcaaa 120

taagaatttg ataagtacca cttaaattta actcctttgg ttagaggcgc gccatgggca 180

gcaactcatt gagtatgata aaagttagat tgcaaaatct gtttgacaat gatgaagtag 240

cattgttaaa aataacatgc tatactgaca aattaataca gttaactaat gctttggcta 300

aggcagttat acatacaatc aaattgaatg gcattgtatt tgtgcatgtt attacaagta 360

gtgatatttg ccctaataat aatattgtag tgaaatccaa tttcacaaca atgccagtat 420

tacaaaatgg aggttatata tgggaaatga tggaattaac acactgctct caacctaatg 480

gcctaataga tgacaattgt gaaattaaat tctccaaaaa actaagtgat tcaacaatga 540

ccaattatat gaatcaatta tctgaattac ttggatttga cctcaatcca taaatcataa 600

taaatatcaa ctagcaaatc aatgtcacta acaccattag ttaatataaa acttgacaga 660

agataaaaat ggggcaaata aatcaattca gccgacccaa ccatggacac aacacacaat 720

gataccacac cacaaagact gatgatcaca gacatgaggc cattatcgct tgagactata 780

ataacatctc taaccagaga tatcataaca cataaattta tatacttgat aaatcatgaa 840

tgcatagtaa gaaaacttga tgaaagacag gccacattta catttctggt caactatgaa 900

atgaaactat tgcacaaagt gggaagcact aaatataaaa aatatactga atacaacaca 960

aaatatggca ctttccctat gccaatattt atcaatcatg atgggttctt agaatgcatt 1020

ggcattaagc ctaccaagca cacacccata atatacaagt atgatctcaa tccatgaata 1080

tcaaaccaag attcaaacaa tccgaaataa caactttatg cataatcaca ctccatagtc 1140

caaatggagc ctgaaaatta tagttattta aaattcctgc aggaaggaga gacataagat 1200

gaaagatggg gcaaatacaa aaatggctct tagcaaagtc aagttgaatg atacactcaa 1260

caaagatcaa cttctatcat ccagcaaata taccatccaa cggagcacag gagacagcat 1320

tgacactcct aattatgatg tgcagaaaca cattaataag ttatgtggca tgttattaat 1380

cacagaagat gctaatcata aattcactgg gttaataggt atgttatatg ctatgtctag 1440

attaggaaga gaagacacca taaaaatact caaagatgcg ggatatcatg ttaaggcaaa 1500

tggagtggat gtaacaacac atcgtcaaga cattaatggg aaagaaatga aatttgaagt 1560

gttaacatta gcaagcttaa caactgaaat tcaaatcaac attgagatag aatctagaaa 1620

atcctacaaa aaaatgctaa aagaaatggg agaggtggct ccagaataca ggcatgactc 1680

tcctgattgt gggatgataa tattatgtat agcggcatta gtaataacca aattagcagc 1740

aggagataga tcaggtctta cagctgtgat taggagagct aataatgtcc taaaaaatga 1800

aatgaaacgt tataaaggtt tattacccaa ggatatagcc aacagcttct atgaagtgtt 1860

tgaaaaatat cctcacttta tagatgtttt tgttcatttt ggtatagcac aatcttctac 1920

cagaggtggc agtagagttg aagggatttt tgcaggattg tttatgaatg cctatggtgc 1980

agggcaagtg atgttacggt ggggggtctt agcaaaatca gttaaaaaca ttatgttagg 2040

acacgctagt gtacaagcag aaatggaaca agttgtggag gtgtatgagt atgctcagaa 2100

attgggtgga gaagcaggat tctaccatat attgaacaac ccaaaagcat cactattatc 2160

tttgactcaa tttcctcact tctctagtgt agtattgggc aatgctgctg gcctaggcat 2220

aatgggagaa tacagaggta caccaaggaa tcaagattta tatgatgctg caaaagcata 2280

tgctgaacaa ctcaaagaaa atggtgtgat taactacagt gtattagatt tgacagcaga 2340

agaactagag gctatcaaac atcagcttaa tccaaaagat aatgatgtag agctttgagt 2400

taataaaaaa gtggggcaaa taaatcatca tggaaaagtt tgctcctgaa ttccatggag 2460

aagatgcaaa caacagagcc accaaattcc tagaatcaat aaagggcaaa ttcacatcac 2520

ccaaagatcc caagaaaaaa gatagtatca tatctgtcaa ctcaatagat atagaagtaa 2580

ccaaagaaag ccctataaca tcaaattcaa ccattataaa cccaataaat gagacagatg 2640

atactgtagg gaacaagccc aattatcaaa gaaagcctct agtaagtttc aaagaagacc 2700

ctacgccaag tgataatcct ttttcaaaac tatacaaaga aaccatagaa acatttgata 2760

acaatgaaga agaatctagc tattcatatg aagaaataaa tgatcagaca aacgataata 2820

taacagcaag attagatagg attgatgaga aattaagtga aatactagga atgcttcaca 2880

cattagtagt agcgagtgca ggacccacat ctgctcggga tggtataaga gatgccatgg 2940

ttggtttaag agaagaaatg atagaaaaaa tcagaactga agcattaatg accaatgaca 3000

gactagaagc tatggcaaga ctcaggaatg aagaaagtga aaagatggca aaagacacat 3060

cagatgaagt gtctctcaat ccaacatcag agaaactgaa caacctgttg gaagggaatg 3120

atagtgacaa tgatctatca cttgaagatt tctgattagc taccaaactg tacatcaaaa 3180

cacaacacca atagaaaacc aacaaacaaa ccaactcacc catccaacca aacatctatc 3240

tgctgattag ccaaccagcc aaaaaacaac cagccaatct aaaactagcc acccggaaaa 3300

aatcgatact atagttacaa aaaaagatgg ggcaaatatg gaaacatacg tgaataaact 3360

tcacgagggc tccacataca cagctgctgt tcaatacaat gtcctagaaa aagacgatga 3420

tcctgcatca cttacaatat gggtgcccat gttccaatca tccatgccag cagatctact 3480

cataaaagaa ctagccaatg tcaatatact agtgaaacaa atatccacac ccaaggaccc 3540

ctcattaaga gtcatgataa actcaagaag tgcagtgcta gcacaaatgc ccagcaaatt 3600

taccatatgt gccaatgtgt ccttggatga aagaagcaag ctggcatatg atgtaaccac 3660

accctgtgaa attaaggcat gcagtctaac atgcctaaaa tcaaaaaata tgttaactac 3720

agttaaagat ctcactatga aaacactcaa cccaacacat gacatcattg ctttatgtga 3780

atttgaaaat atagtaacat caaaaaaagt cataatacca acatacctaa gatctatcag 3840

cgtcagaaat aaagatctga acacacttga aaatataaca accactgaat tcaaaaatgc 3900

cattacaaat gcaaaaatca tcccttactc aggattattg ttagtcatca cagtgactga 3960

caacaaagga gcattcaaat acataaagcc acaaagtcaa ttcatagtag atcttggagc 4020

ttacctagaa aaagaaagta tatattatgt tacaacaaat tggaagcaca cagctacacg 4080

atttgcaatc aaacccatgg aagattaacc tttttcctct acatcaatga gtagattcat 4140

acaaactttc taactacatt cttcacttca caatcataat caccaaccct ctgtggttca 4200

atcaatcaaa caaaactcat caagagttcc agatcatccc aagtcattgt tcatcagatc 4260

cagtactcaa ataagttaat aaaaaatcca catggggcaa ataatcattg agggaaatcc 4320

aactaatcac aacatctgtc aacatagaca agtcaacacg ctagataaaa tcaaccaatg 4380

gaaaatacat ccataactat agaattctca agcaaattct ggccttactt tacactaata 4440

cacatgataa caacaataat ctctttgata atcataatct ccatcatgat tgcaatacta 4500

aacaaactct gcgaatataa tgtattccat aacaaaacct ttgagctacc aagagctcga 4560

gtcaatacat agcattcacc aatctgatag ctcaaaacag taaccttgca tttgtaaatg 4620

aactaccctc acttcttcac aaaaccacat caacatctca ccatgcaagc catcatctat 4680

accataaagt agttaattaa aaatggccgg ccagtcataa caatgaacta ggatattaag 4740

accaaaaaca acgctggggc aaatgcaaac atgtccaaaa ccgaggacca acgcaccgcc 4800

aagacactag aaaggacctg ggacactttt aatcatctat tattcatatc atcgtgctta 4860

tacaagttaa atcttaaatc tatagcacaa atcacattat ctattttggc aattataatc 4920

tcaacctcac ttataattgc agccatcata ttcatagcct cggcaaacca caaagtcaca 4980

ctaacaactg caatcataca agatgcaacg aaccagatca agaacacaac cccaacatac 5040

ctcacccaga atccccagca tggaatcagc ttctccaatc tgtccggaac tacatcacaa 5100

tccaccacca tactagcttc aacaacacca agtgctgatt caaccccaca atccacaaca 5160

gtcaagatca aaaacacaac aacaacccaa atattaccta gcaaacccac cacaaaacaa 5220

cgccaaaata aaccacaaaa caaacccaac aatgattttc actttgaagt gttcaatttt 5280

gtaccctgca gcatatgcag caacaatcca acctgctggg ccatctgcaa gagaatacca 5340

aacaaaaaac ctggaaagaa aaccaccacc aagcccacaa aaaaaccaac cctcaagaca 5400

accaaaaaag atcccaaatc ccaaaccaca aaaccaaagg aagtactcac taccaagcct 5460

acaggaaagc caaccatcaa caccactaaa acaaacatca gaactacact gctcacctcc 5520

aacaccaaag gaaatccaga acacacaagt caagaggaaa ccctccactc aaccacctcc 5580

gaaggctatc caagcccatc acaagtccac acaacatccg gtcaagagga aaccctccac 5640

tcaaccacct ccgaaggcta tccaagccca tcacaagtct acacaacatc cgagtaccta 5700

tcacaatctc tatcttcatc caacacaaca aaatgatagt cattaaaaag cacgcgtgta 5760

ttgttgcaaa aagccatgac caaatcaaac agaatcaaaa tcaactctgg ggcaaataac 5820

aatgggttcc aagtcttcta ccaggttccc agtacctcta atgctgaccg tccgagtcat 5880

gctggcactg agttgcgtct gtccggccag cgcccttgat ggcaggcctc ttgcggctgc 5940

agggattgtg gtaacaggag acaaagcggt caacatatac acatcatctc agacagggtc 6000

aatcataatc aagttactcc cgaatatgcc caaggataaa gaggcgtgtg caaaagcacc 6060

gttggaggca tacaacagga cattgactac tttgctcacc cctcttggtg attctatccg 6120

taggatacaa gagtctgtga ccacgtccgg aggaagaaaa cagggacgcc ttataggagc 6180

cattatcggt ggtgtagctc tcggggtcgc gaccgctgca caaataacag cagcctcggc 6240

tttgatacaa gccaatcaaa atgctgccaa catcctccgg ctcaaagaga gcattgctgc 6300

aaccaatgag gctgtacacg aggtcactga cggattatca caactagcag tggcagttgg 6360

gaagatgcag caatttgtta atgaccagtt taataagaca gcccaggaat tggactgtat 6420

aaaaattgca cagcaagttg gtgtagaact caacctgtac ttaactgaat tgactacagt 6480

atttgggccg caaatcactt cccctgcctt aactcagctg actatccagg cgctttacaa 6540

tctagctggt gggaatatgg attacttgtt aactaagtta ggtgtgggga ataaccaact 6600

cagctcatta attagtagtg gcctgatcac cggcaaccct attctgtatg actcacagac 6660

tcaactcttg ggtatacagg taatcctacc ctcagtcggg aatctaaata atatgcgtgc 6720

cacctatctg gaaaccttgt ctgtaagtac aactaaagga tttgcctcag ctcttgtccc 6780

aaaggtagtg acacaggtcg gttctgtgat agaagagctt gacacctcat actgtataga 6840

gaccgatttg gatctatatt gtacaagaat agtgacattc cctatgtctc ctggtattta 6900

ttcctgtttg agtggcaata catctgcttg catgtactca aagacagaag gcgcactcac 6960

tacgccgtat atgaccctca aaggctcagt tattgcaaac tgtaagatga caacatgtag 7020

gtgtgcagat cccccgggta tcatatcgca aaattatgga gaagctgtgt ctctaataga 7080

taggcaatca tgcaatatcc tatccttaga cgggataact ttgaggctca gtggggaatt 7140

cgatgcaact tatcaaagga atatctcaat acaagattct caagttatag tgacaggcaa 7200

tcttgatatc tcaactgagc ttgggaatgt caacaactcg ataagcaatg ctttggataa 7260

gttagaggaa agcaacagca aactagacaa ggtcaatgtc aaattggcca gcacatccgc 7320

tataattata gtaattatag taatattgtt agcattaatt gcagttggac tgcttctata 7380

ctgcaaggcc agaagcacac cagtcacatt aagtaaggat caactgagtg gtataaataa 7440

tattgcattt agtaactgaa taaaaatagc acctaatcat attcttacaa tggttcgcta 7500

tttgaccata gataacccat ctatcattag attatcctaa aatttgaact tcatcacaac 7560

tttcatctat aaaccatctc acttacactt tttaagtaga tttctatttt atagttatat 7620

aaaacagggc ccattgaata ccaaattaac ttactatttg taaaaatgag aattggggca 7680

aatatgtcac gaaggaatcc ttgcaaattc gaaattcgag gtcattgctt gaatggtaaa 7740

aggtgtcatt ttagtcataa ttattttgaa tggccacccc atgcactgct tgtaagacaa 7800

aactttatgt taaacagaat acttaagtct atggataaaa gcatagatac tttgtcagaa 7860

ataagtggag ctgcagagtt ggacagaaca gaagagtatg ccctcggtgt agttggagtg 7920

ctagagagtt atataggatc aataaataat ataactaaac aatcagcatg tgttgccatg 7980

agcaaactcc ttactgaact caacagcgat gacatcaaaa aactaaggga caatgaagag 8040

ccaaactcac ccaaagtaag agtgtacaat actgtcatat catatattga aagcaacagg 8100

aagaacaata aacaaactat ccatctgtta aaaagattgc cagcagacgt attgaagaaa 8160

accatcaaaa acacattgga tatccacaag agcataacca tcaataaccc aaaagaatca 8220

actgttagtg atacgaacga ccatgccaaa aataatgata ctacctgaca aatatccttg 8280

tagtataaat tccatactaa taacaagtaa ttgtagagtc actatgtata atcaaaaaaa 8340

cacactatat atcaatcaaa acaaccaaaa tagccatata tacccaccgg atcaaccatt 8400

caatgaaatc cattggacct ctcaagactt gattgatgca actcaaaatt ttctacaaca 8460

tctaggtatt actgatgata tatacacaat atatatatta gtgtcataat actcaatcct 8520

aatacttacc acatcatcaa attattaact caaacaattc aagctatggg acaaaatgga 8580

tcccattatt agtggaaatt ctgctaatgt ttatctaact gatagttatt taaaaggtgt 8640

tatttctttc tcagaatgta acgctttagg aagttacata ttcaatggtc cttatctcaa 8700

aaatgattat accaacttaa ttagtagaca aaatccatta atagaacaca taaatctaaa 8760

gaaactaaat ataacacagt ccttaatatc taagtatcat aaaggtgaaa taaaaataga 8820

agaacctact tactttcagt cattacttat gacatacaag agtatgacct cttcagaaca 8880

gactactact actaatttac ttaaaaagat aataagaaga gctatagaaa tcagtgatgt 8940

caaagtctat gctatattga ataaactggg gctcaaagaa aaagacaaga ttaaatccaa 9000

taatggacaa gatgaagaca actcagtcat tactaccata atcaaagatg atatactttt 9060

agctgtcaag gataatcaat ctcatcttaa agcagacaaa aatcaatcca caaaacaaaa 9120

agatacaatc aaaacaacac ttttgaagaa attaatgtgt tcgatgcaac atcctccatc 9180

atggttaata cattggttta atttatacac aaaattaaac agcatattaa cacaatatcg 9240

atctagtgag gtaaaaaacc atggttttat attgatagat aatcatactc ttagtggatt 9300

ccaatttatt ttgaatcaat atggttgtat agtttatcat aaggaactca aaagaattac 9360

tgtgacaact tataatcaat tcttgacatg gaaagatatt agccttagta gattaaatgt 9420

ttgtttgatt acatggatta gtaactgttt gaacacatta aacaaaagct taggcttaag 9480

atgtggattc aataatgtta tcttgacaca attattcctt tatggagatt gtatactaaa 9540

actattccac aatgaggggt tctacataat aaaagaggta gagggattta ttatgtctct 9600

aattttaaat ataacagaag aagatcaatt cagaaaacgg ttttataata gtatgctcaa 9660

caacatcaca gatgccgcca acaaagctca aaaaaatctg ctatcaagag tatgtcatac 9720

attattagat aagacaatat cagataatat aataaatggc agatggataa ttctattgag 9780

taagttccta aaattaatta agcttgcagg tgacaataac ctcaacaatc tgagtgaatt 9840

atattttttg ttcagaatat ttggacaccc aatggtagat gaaagacaag ccatggatgc 9900

tgttaaagtt aattgcaacg agaccaaatt ttatttgtta agtagtttga gtatgttaag 9960

aggagctttt atatatagaa ttataaaagg gtttgtaaat aattacaaca gatggcctac 10020

tttaagaaat gccattgtct tacccttaag atggttaact tactataaac taaacactta 10080

tccttccttg ttggaactta cagaaagaga tttgattgtt ctatcaggac tacgtttcta 10140

tcgagagttt cggttgccta aaaaagtgga tcttgaaatg atcataaatg ataaggctat 10200

atcacctcct aaaaatttaa tatggactag tttccctaga aattatatgc cgtcacacat 10260

acaaaattat atagaacatg aaaaattaaa attctctgat agtgataaat caagaagagt 10320

attagagtat tatttaagag ataacaaatt caatgaatgt gatttacaca actgtgtagt 10380

taatcaaagt tatcttaaca acccgaatca tgtggtatca ttgacaggca aagaaagaga 10440

actcagtgta ggtagaatgt ttgcaatgca accaggaatg ttcagacaag ttcaaatatt 10500

agcagagaaa atgatagcag aaaacatatt acaatttttc cctgaaagtc ttacaagata 10560

tggtgatcta gaactacaga aaatattaga attgaaagca ggaataagta acaaatcaaa 10620

tcgttacaat gataattaca acaattacat tagtaagtgc tctatcatca cagatctcag 10680

caaattcaat caagcatttc gatatgaaac atcatgtatt tgtagtgatg tactggatga 10740

actgcatggt gtacaatctc tattttcctg gttacattta actattcctc atgtcacaat 10800

aatatgcaca tataggcatg caccccccta tataaaggat catattgtag atcttaacaa 10860

tgtagatgag caaagtggac tatatagata tcatatgggt ggtatcgaag ggtggtgtca 10920

aaaactatgg accatagaag ctatatcact attagatcta atatctctca aagggaaatt 10980

ctcaattact gctttaatta atggtgacaa tcaatcaata gatataagta aaccagtcag 11040

actcatggaa ggtcaaactc atgctcaagc agattatttg ctagcattaa atagtctcaa 11100

attactgtat aaagagtatg caggaatagg ccacaaatta aaaggaactg agacttatat 11160

atcgagagat atgcaattta tgagtaaaac gatccaacat aacggtgtat attacccagc 11220

tagtataaag aaagtcctaa gagtgggacc gtggataaac actatacttg atgacttcaa 11280

agtgagtcta gaatctatag gtagtttgac acaagaatta gaatatagag gtgaaagtct 11340

attatgcagt ttaatattta gaaatgtatg gttatataat caaattgcat tacaacttaa 11400

aaatcatgca ttatgtaaca acaaattata tttggatata ttaaaagttc taaaacactt 11460

aaaaaccttt tttaatcttg ataacattga tacagcatta acattgtata tgaatttgcc 11520

catgttattt ggtggtggtg atcccaactt gttatatcga agtttctata gaagaactcc 11580

tgatttcctc acagaggcta tagttcactc tgtgttcata cttagttatt atacaaacca 11640

tgatttaaaa gataaacttc aagatctgtc agatgataga ttgaataagt tcttaacatg 11700

cataatcacg tttgataaaa accccaatgc tgaattcgtt acattgatga gagatcctca 11760

agctttagga tctgagaggc aagctaaaat tactagcgaa atcaatagac tggcagttac 11820

cgaggttttg agcacagctc caaacaaaat attttccaaa agtgcacaac actataccac 11880

tacagagata gatcttaatg atattatgca aaatatagaa cctacatatc ctcacgggct 11940

aagagttgtt tatgagagtt taccctttta taaagcagag aaaatagtaa atcttatatc 12000

cggtacaaaa tctataacta acatactgga aaagacttct gccatagact taacagatat 12060

tgatagagcc actgagatga tgaggaaaaa cataactttg cttataagga tattaccatt 12120

agattgtaac agagataaaa gagaaatatt gagtatggaa aacctaagta ttactgaatt 12180

aagcaaatac gttagagaaa gatcttggtc tttatccaat atagttggtg ttacatcacc 12240

cagtatcatg tatacaatgg acataaaata tacaacaagc actatagcta gtggcataat 12300

catagagaaa tataatgtca acagtttaac acgtggtgag agaggaccca ctaaaccatg 12360

ggttggttca tctacacaag agaaaaagac aatgccagtt tataatagac aagttttaac 12420

caaaaaacag agagatcaaa tagatctatt agcaaaattg gattgggtgt atgcatctat 12480

agataacaag gatgaattta tggaggaact tagcatagga actcttgggt taacatatga 12540

gaaggccaaa aaattattcc cacaatattt gagtgttaac tatttgcatc gtcttacagt 12600

cagtagtaga ccatgtgaat tccctgcatc tataccagct tatagaacta caaattatca 12660

ctttgatact agccctatta atcgcatatt aacagaaaag tatggtgatg aagatattga 12720

tatagtattc caaaactgta taagctttgg ccttagctta atgtctgtag tagaacaatt 12780

tactaatgta tgtcctaaca gaattattct catacccaag cttaatgaga tacatttgat 12840

gaaacctccc atattcacag gcgatgttga tattcacaag ttaaaacaag tgatacaaaa 12900

acaacatatg tttttaccag acaaaataag tttgactcaa tatgtggaat tattcttaag 12960

taataaaaca ctcaaatctg gatctaatgt taattctaat ttaatattgg cgcataagat 13020

atctgactat tttcataata cttacatttt gagtactaat ttagctggac attggattct 13080

tattatacaa cttatgaaag attctaaggg tatttttgaa aaagattggg gagagggata 13140

tataactgat catatgttca ttaatttgaa agttttcttc aatgcttata agacatatct 13200

cttgtgtttt cataaaggtt acggcagagc aaagctggag tgtgatatga atacttcaga 13260

tctcctatgt gtattggaat taatagacag tagttattgg aagtctatgt ctaaggtgtt 13320

tttagaacaa aaagttatca aatacattct tagccaggat gcaagtttac atagagtaaa 13380

aggatgtcat agcttcaaac tatggtttct taaacgtctt aatgtagcag aattcacagt 13440

ttgcccttgg gttgttaaca tagattatca tccaacacat atgaaagcaa tattaactta 13500

tattgatctt gttagaatgg gattgataaa tatagataga atatacatta aaaataaaca 13560

caagttcaat gatgagtttt atacttctaa tctgttttac attaattata acttctcaga 13620

taatactcat ctattaacta aacatataag gattgctaat tccgaattag aaagtaatta 13680

caacaaatta tatcatccta caccagaaac cctagaaaat atactaacca atccggttaa 13740

aagtaatgag aaaaagacac tgagtgacta ttgtataggt aaaaatgttg actcaataat 13800

gttaccatcg ttatctaata agaagcttat taaatcgtct acaatgatta gaaccaatta 13860

cagcagacaa gatttgtata atttatttcc tacggttgtg attgataaaa ttatagatca 13920

ttcaggtaat acagccaaat ctaaccaact ttacactact acttctcatc aaatatcctt 13980

agtgcacaat agcacatcac tttattgcat gcttccttgg catcatatta atagattcaa 14040

ttttgtattt agttctacag gttgtaaaat tagtatagag tatattttaa aagatcttaa 14100

aattaaggat cctaattgta tagcattcat aggtgaagga gcagggaatt tattattgcg 14160

tacagtagtg gaacttcatc ctgatataag atatatttac agaagtctga aagattgcaa 14220

tgatcatagt ttaccaattg agtttttaag gctgtacaat ggacatatca acattgatta 14280

tggtgaaaat ttgaccattc ctgctacaga tgcaaccaac aacattcatt ggtcttattt 14340

acatataaag tttgctgaac ctatcagtct ttttgtctgt gatgctgaat tgcctgtaac 14400

agtcaactgg agtaagatta taatagagtg gagcaagcat gtaagaaaat gcaagtactg 14460

ttcttcagtt aataaatgta cattgatagt aaaatatcat gctcaagatg atatcgattt 14520

caaattagac aacataacta tattaaaaac ttatgtatgc ttaggtagta agttaaaggg 14580

atctgaagtt tacttagtcc ttacaatagg tcctgcaaat gtgttcccag tatttaatgt 14640

agtacaaaat gctaaattga tactatcaag aactaaaaat ttcatcatgc ctaaaaaagc 14700

tgataaagag tctattgatg caaatattaa gagtttgata ccctttcttt gttaccctat 14760

aacaaaaaaa ggaattaata ctgcattgtc taaattaaag agtgttgtta gtggagatat 14820

actatcatat tctatagctg gacgtaatga agttttcagc aataaactta taaatcataa 14880

gcatatgaac atcttaaagt ggttcaatca tgttttaaat ttcagatcaa cagaattaaa 14940

ctataatcat ttatatatgg tagaatctac ttatcctcat ctaagtgaat tgttaaacag 15000

cttgacaacc aatgaactta aaaaactgat taaaatcaca ggtagtttgt tatacaactt 15060

ttataatgaa taatgagcaa aaatcttata acaaaaatag ctacacacta acattgtatt 15120

caattatagt tattgaaaat taataattat ataattttta ataacttcta gtgaactaat 15180

cctaaaatta tcattttgat ctaggaagaa taagtttaaa tccaaatcta attggtttat 15240

atgtatatta actaaattac gagatattag tttttgacac tttttttctc gtggccggca 15300

tggtcccagc ctcctcgctg gcgccggctg ggcaacatgc ttcggcatgg cgaatgggac 15360

tagcataacc ccttggggcc tctaaacggg tcttgagggg ttttttg 15407

<210> 6
<211> 17208
<212> DNA
<213> Artificial Sequence

<220>
<223> cRSVA_MVF_A

<400> 6
   taatacgact cactataggt tttttcgcgt ctgatgaggc cgttaggccg aaactcctct 60
   ccggagtcac gcgaaaaaat gcgtacaaca aacttgcgta aaccaaaaaa atggggcaaa 120
   taagaatttg ataagtacca cttaaattta actcctttgg ttagaggcgc gccatgggca 180
   gcaactcatt gagtatgata aaagttagat tgcaaaatct gtttgacaat gatgaagtag 240
   cattgttaaa aataacatgc tatactgaca aattaataca gttaactaat gctttggcta 300
   aggcagttat acatacaatc aaattgaatg gcattgtatt tgtgcatgtt attacaagta 360
   gtgatatttg ccctaataat aatattgtag tgaaatccaa tttcacaaca atgccagtat 420
   tacaaaatgg aggttatata tgggaaatga tggaattaac acactgctct caacctaatg 480
   gcctaataga tgacaattgt gaaattaaat tctccaaaaa actaagtgat tcaacaatga 540
   ccaattatat gaatcaatta tctgaattac ttggatttga cctcaatcca taaatcataa 600
   taaatatcaa ctagcaaatc aatgtcacta acaccattag ttaatataaa acttgacaga 660
   agataaaaat ggggcaaata aatcaattca gccgacccaa ccatggacac aacacacaat 720
   gataccacac cacaaagact gatgatcaca gacatgaggc cattatcgct tgagactata 780
   ataacatctc taaccagaga tatcataaca cataaattta tatacttgat aaatcatgaa 840
   tgcatagtaa gaaaacttga tgaaagacag gccacattta catttctggt caactatgaa 900
   atgaaactat tgcacaaagt gggaagcact aaatataaaa aatatactga atacaacaca 960
   aaatatggca ctttccctat gccaatattt atcaatcatg atgggttctt agaatgcatt 1020
   ggcattaagc ctaccaagca cacacccata atatacaagt atgatctcaa tccatgaata 1080
   tcaaaccaag attcaaacaa tccgaaataa caactttatg cataatcaca ctccatagtc 1140
   caaatggagc ctgaaaatta tagttattta aaattcctgc aggaaggaga gacataagat 1200
   gaaagatggg gcaaatacaa aaatggctct tagcaaagtc aagttgaatg atacactcaa 1260
   caaagatcaa cttctatcat ccagcaaata taccatccaa cggagcacag gagacagcat 1320
   tgacactcct aattatgatg tgcagaaaca cattaataag ttatgtggca tgttattaat 1380
   cacagaagat gctaatcata aattcactgg gttaataggt atgttatatg ctatgtctag 1440
   attaggaaga gaagacacca taaaaatact caaagatgcg ggatatcatg ttaaggcaaa 1500
   tggagtggat gtaacaacac atcgtcaaga cattaatggg aaagaaatga aatttgaagt 1560
   gttaacatta gcaagcttaa caactgaaat tcaaatcaac attgagatag aatctagaaa 1620
   atcctacaaa aaaatgctaa aagaaatggg agaggtggct ccagaataca ggcatgactc 1680
   tcctgattgt gggatgataa tattatgtat agcggcatta gtaataacca aattagcagc 1740
   aggagataga tcaggtctta cagctgtgat taggagagct aataatgtcc taaaaaatga 1800
   aatgaaacgt tataaaggtt tattacccaa ggatatagcc aacagcttct atgaagtgtt 1860
   tgaaaaatat cctcacttta tagatgtttt tgttcatttt ggtatagcac aatcttctac 1920
   cagaggtggc agtagagttg aagggatttt tgcaggattg tttatgaatg cctatggtgc 1980
   agggcaagtg atgttacggt ggggggtctt agcaaaatca gttaaaaaca ttatgttagg 2040
   acacgctagt gtacaagcag aaatggaaca agttgtggag gtgtatgagt atgctcagaa 2100
   attgggtgga gaagcaggat tctaccatat attgaacaac ccaaaagcat cactattatc 2160
   tttgactcaa tttcctcact tctctagtgt agtattgggc aatgctgctg gcctaggcat 2220
   aatgggagaa tacagaggta caccaaggaa tcaagattta tatgatgctg caaaagcata 2280
   tgctgaacaa ctcaaagaaa atggtgtgat taactacagt gtattagatt tgacagcaga 2340
   agaactagag gctatcaaac atcagcttaa tccaaaagat aatgatgtag agctttgagt 2400
   taataaaaaa gtggggcaaa taaatcatca tggaaaagtt tgctcctgaa ttccatggag 2460
   aagatgcaaa caacagagcc accaaattcc tagaatcaat aaagggcaaa ttcacatcac 2520
   ccaaagatcc caagaaaaaa gatagtatca tatctgtcaa ctcaatagat atagaagtaa 2580
   ccaaagaaag ccctataaca tcaaattcaa ccattataaa cccaataaat gagacagatg 2640
   atactgtagg gaacaagccc aattatcaaa gaaagcctct agtaagtttc aaagaagacc 2700
   ctacgccaag tgataatcct ttttcaaaac tatacaaaga aaccatagaa acatttgata 2760
   acaatgaaga agaatctagc tattcatatg aagaaataaa tgatcagaca aacgataata 2820
   taacagcaag attagatagg attgatgaga aattaagtga aatactagga atgcttcaca 2880
   cattagtagt agcgagtgca ggacccacat ctgctcggga tggtataaga gatgccatgg 2940
   ttggtttaag agaagaaatg atagaaaaaa tcagaactga agcattaatg accaatgaca 3000
   gactagaagc tatggcaaga ctcaggaatg aagaaagtga aaagatggca aaagacacat 3060
   cagatgaagt gtctctcaat ccaacatcag agaaactgaa caacctgttg gaagggaatg 3120
   atagtgacaa tgatctatca cttgaagatt tctgattagc taccaaactg tacatcaaaa 3180
   cacaacacca atagaaaacc aacaaacaaa ccaactcacc catccaacca aacatctatc 3240
   tgctgattag ccaaccagcc aaaaaacaac cagccaatct aaaactagcc acccggaaaa 3300
   aatcgatact atagttacaa aaaaagatgg ggcaaatatg gaaacatacg tgaataaact 3360
   tcacgagggc tccacataca cagctgctgt tcaatacaat gtcctagaaa aagacgatga 3420
   tcctgcatca cttacaatat gggtgcccat gttccaatca tccatgccag cagatctact 3480
   cataaaagaa ctagccaatg tcaatatact agtgaaacaa atatccacac ccaaggaccc 3540
   ctcattaaga gtcatgataa actcaagaag tgcagtgcta gcacaaatgc ccagcaaatt 3600
   taccatatgt gccaatgtgt ccttggatga aagaagcaag ctggcatatg atgtaaccac 3660
   accctgtgaa attaaggcat gcagtctaac atgcctaaaa tcaaaaaata tgttaactac 3720
   agttaaagat ctcactatga aaacactcaa cccaacacat gacatcattg ctttatgtga 3780
   atttgaaaat atagtaacat caaaaaaagt cataatacca acatacctaa gatctatcag 3840
   cgtcagaaat aaagatctga acacacttga aaatataaca accactgaat tcaaaaatgc 3900
   cattacaaat gcaaaaatca tcccttactc aggattattg ttagtcatca cagtgactga 3960
   caacaaagga gcattcaaat acataaagcc acaaagtcaa ttcatagtag atcttggagc 4020
   ttacctagaa aaagaaagta tatattatgt tacaacaaat tggaagcaca cagctacacg 4080
   atttgcaatc aaacccatgg aagattaacc tttttcctct acatcaatga gtagattcat 4140
   acaaactttc taactacatt cttcacttca caatcataat caccaaccct ctgtggttca 4200
   atcaatcaaa caaaactcat caagagttcc agatcatccc aagtcattgt tcatcagatc 4260
   cagtactcaa ataagttaat aaaaaatcca catggggcaa ataatcattg agggaaatcc 4320
   aactaatcac aacatctgtc aacatagaca agtcaacacg ctagataaaa tcaaccaatg 4380
   gaaaatacat ccataactat agaattctca agcaaattct ggccttactt tacactaata 4440
   cacatgataa caacaataat ctctttgata atcataatct ccatcatgat tgcaatacta 4500
   aacaaactct gcgaatataa tgtattccat aacaaaacct ttgagctacc aagagctcga 4560
   gtcaatacat agcattcacc aatctgatag ctcaaaacag taaccttgca tttgtaaatg 4620
   aactaccctc acttcttcac aaaaccacat caacatctca ccatgcaagc catcatctat 4680
   accataaagt agttaattaa aaatggccgg ccagtcataa caatgaacta ggatattaag 4740
   accaaaaaca acgctggggc aaatgcaaac atgtccaaaa ccgaggacca acgcaccgcc 4800
   aagacactag aaaggacctg ggacactttt aatcatctat tattcatatc atcgtgctta 4860
   tacaagttaa atcttaaatc tatagcacaa atcacattat ctattttggc aattataatc 4920
   tcaacctcac ttataattgc agccatcata ttcatagcct cggcaaacca caaagtcaca 4980
   ctaacaactg caatcataca agatgcaacg aaccagatca agaacacaac cccaacatac 5040
   ctcacccaga atccccagca tggaatcagc ttctccaatc tgtccggaac tacatcacaa 5100
   tccaccacca tactagcttc aacaacacca agtgctgatt caaccccaca atccacaaca 5160
   gtcaagatca aaaacacaac aacaacccaa atattaccta gcaaacccac cacaaaacaa 5220
   cgccaaaata aaccacaaaa caaacccaac aatgattttc actttgaagt gttcaatttt 5280
   gtaccctgca gcatatgcag caacaatcca acctgctggg ccatctgcaa gagaatacca 5340
   aacaaaaaac ctggaaagaa aaccaccacc aagcccacaa aaaaaccaac cctcaagaca 5400
   accaaaaaag atcccaaatc ccaaaccaca aaaccaaagg aagtactcac taccaagcct 5460
   acaggaaagc caaccatcaa caccactaaa acaaacatca gaactacact gctcacctcc 5520
   aacaccaaag gaaatccaga acacacaagt caagaggaaa ccctccactc aaccacctcc 5580
   gaaggctatc caagcccatc acaagtccac acaacatccg gtcaagagga aaccctccac 5640
   tcaaccacct ccgaaggcta tccaagccca tcacaagtct acacaacatc cgagtaccta 5700
   tcacaatctc tatcttcatc caacacaaca aaatgatagt cattaaaaag cacgcgtgta 5760
   ttgttgcaaa aagccatgac caaatcaaac agaatcaaaa tcaactctgg ggcaaataac 5820
   aatgtccatc atgggtctca aggtgaacgt ctctgccata ttcatggcag tactgttaac 5880
   tctccaaaca cccaccggtc aaatccattg gggcaatctc tctaagatag gggtggtagg 5940
   aataggaagt gcaagctaca aagttatgac tcgttccagc catcaatcat tagtcataaa 6000
   attaatgccc aatataactc tcctcaataa ctgcacgagg gtagagattg cagaatacag 6060
   gagactactg agaacagttt tggaaccaat tagagatgca cttaatgcaa tgacccagaa 6120
   tataagaccg gttcagagtg tagcttcaag taggagacac aagagatttg cgggagtagt 6180
   cctggcaggt gcggccctag gcgttgccac agctgctcag ataacagccg gcattgcact 6240
   tcaccagtcc atgctgaact ctcaagccat cgacaatctg agagcgagcc tggaaactac 6300
   taatcaggca attgagacaa tcagacaagc agggcaggag atgatattgg ctgttcaggg 6360
   tgtccaagac tacatcaata atgagctgat accgtctatg aaccaactat cttgtgattt 6420
   aatcggccag aagctcgggc tcaaattgct cagatactat acagaaatcc tgtcattatt 6480
   tggccccagt ttacggaccc ccatatctgc ggagatatct atccaggctt tgagctatgc 6540
   gcttggagga gacatcaata aggtgttaga aaagctcgga tacagtggag gtgatttact 6600
   gggcatctta gagagcggag gaataaaggc ccggataact cacgtcgaca cagagtccta 6660
   cttcattgtc ctcagtatag cctatccgac gctgtccgag attaaggggg tgattgtcca 6720
   ccggctagag ggggtctcgt acaacatagg ctctcaagag tggtatacca ctgtgcccaa 6780
   gtatgttgca acccaagggt accttatctc gaattttgat gagtcatcgt gtactttcat 6840
   gccagagggg actgtgtgca gccaaaatgc cttgtacccg atgagtcctc tgctccaaga 6900
   atgcctccgg gggtacacca agtcctgtgc tcgtacactc gtatccgggt cttttgggaa 6960
   ccggttcatt ttatcacaag ggaacctaat agccaattgt gcatcaatcc tttgcaagtg 7020
   ttacacaaca ggaacgatca ttaatcaaga ccctgacaag atcctaacat acattgctgc 7080
   cgatcactgc ccggtagtcg aggtgaacgg cgtgaccatc caagtcggga gcaggaggta 7140
   tccagacgct gtgtacttgc acagaattga cctcggtcct cccatatcat tggagaggtt 7200
   ggacgtaggg acaaatctgg ggaatgcaat tgctaagttg gaggatgcca aggaattgtt 7260
   ggagtcatcg gaccagatat tgaggagtat gaaaggttta tcgagcacta gcataattat 7320
   agtaattata gtaatattgt tagcattaat tgcagttgga ctgcttctat actgcaaggc 7380
   cagaagcaca ccagtcacat taagtaagga tcaactgagt ggtataaata atattgcatt 7440
   tagtaactga tagtcattaa aaagcacgcg tgtattgttg caaaaagcca tgaccaaatc 7500
   aaacagaatc aaaatcaact ctggggcaaa taacaatgga gttgccaatc ctcaaaacaa 7560
   atgctattac cacaatcctt gctgcagtca cactctgttt cgcttccagt caaaacatca 7620
   ctgaagaatt ttatcaatca acatgcagtg cagtcagcaa aggctatctt agtgctctaa 7680
   gaactggttg gtatactagt gttataacta tagaattaag taatatcaag gaaaataagt 7740
   gtaatggtac agacgctaag gtaaaattaa taaaacaaga attagataaa tataaaaatg 7800
   ctgtaacaga attgcagttg ctcatgcaaa gcacaccagc agccaacaat cgagccagaa 7860
   gagaactacc aagatttatg aattatacac tcaacaatac caaaaacacc aatgtaacat 7920
   taagtaagaa aaggaaaaga agatttcttg gatttttgtt aggtgttgga tctgcaatcg 7980
   ccagtggcat tgccgtatcc aaggtcctgc acctagaagg ggaagtgaac aaaatcaaaa 8040
   gtgctctact atccacaaac aaggctgtag tcagcttatc taatggagtc agtgtcttaa 8100
   ccagcaaggt gttagacctc aaaaactata tagataaaca gttgttacct attgttaaca 8160
   agcaaagctg cagcatatca aacattgaaa ctgtgataga gttccaacaa aagaacaaca 8220
   gactactaga gattaccaga gaatttagtg ttaatgcagg tgtaactact cctgtaagca 8280
   cttatatgtt aactaatagt gagttattat cattaatcaa tgatatgcct ataacaaatg 8340
   atcagaaaaa gttaatgtcc agcaatgttc aaatagttag acagcaaagt tactctatca 8400
   tgtcaataat aaaagaggaa gtcttggcat atgtagtaca attaccacta tatggtgtaa 8460
   tagatactcc ttgttggaaa ctacacacat cccctttatg tacaaccaac acaaaggaag 8520
   gatccaacat ctgcttaaca agaaccgaca gaggatggta ctgtgacaat gcaggatcag 8580
   tatccttttt cccacaagct gaaacatgta aagttcaatc gaatcgggtg ttttgtgaca 8640
   caatgaacag tttaacatta ccaagtgagg taaatctctg caacattgac atattcaacc 8700
   ccaaatatga ttgcaaaatt atgacttcaa aaacagatgt aagcagctcc gttatcacat 8760
   ctctaggagc cattgtgtca tgctatggca aaaccaaatg tacagcatcc aataaaaatc 8820
   gtgggatcat aaagacattc tctaacgggt gtgattatgt atcaaataag ggggtggata 8880
   ctgtgtctgt aggtaataca ttatattatg taaataagca agaaggcaaa agtctctatg 8940
   taaaaggtga accaataata aatttctatg atccattagt gttcccctct gatgaatttg 9000
   atgcatcaat atctcaagtc aatgagaaaa ttaatcagag tctagcattt atccgtaaat 9060
   cagatgaatt attacataat gtaaatgctg gtaaatccac cacaaatatc atgataacta 9120
   ccataattat agtaattata gtaatattgt tagcattaat tgcagttgga ctgcttctat 9180
   actgcaaggc cagaagcaca ccagtcacat taagtaagga tcaactgagt ggtataaata 9240
   atattgcatt tagtaactga ataaaaatag cacctaatca tattcttaca atggttcgct 9300
   atttgaccat agataaccca tctatcatta gattatccta aaatttgaac ttcatcacaa 9360
   ctttcatcta taaaccatct cacttacact ttttaagtag atttctattt tatagttata 9420
   taaaacaggg cccattgaat accaaattaa cttactattt gtaaaaatga gaattggggc 9480
   aaatatgtca cgaaggaatc cttgcaaatt cgaaattcga ggtcattgct tgaatggtaa 9540
   aaggtgtcat tttagtcata attattttga atggccaccc catgcactgc ttgtaagaca 9600
   aaactttatg ttaaacagaa tacttaagtc tatggataaa agcatagata ctttgtcaga 9660
   aataagtgga gctgcagagt tggacagaac agaagagtat gccctcggtg tagttggagt 9720
   gctagagagt tatataggat caataaataa tataactaaa caatcagcat gtgttgccat 9780
   gagcaaactc cttactgaac tcaacagcga tgacatcaaa aaactaaggg acaatgaaga 9840
   gccaaactca cccaaagtaa gagtgtacaa tactgtcata tcatatattg aaagcaacag 9900
   gaagaacaat aaacaaacta tccatctgtt aaaaagattg ccagcagacg tattgaagaa 9960
   aaccatcaaa aacacattgg atatccacaa gagcataacc atcaataacc caaaagaatc 10020
   aactgttagt gatacgaacg accatgccaa aaataatgat actacctgac aaatatcctt 10080
   gtagtataaa ttccatacta ataacaagta attgtagagt cactatgtat aatcaaaaaa 10140
   acacactata tatcaatcaa aacaaccaaa atagccatat atacccaccg gatcaaccat 10200
   tcaatgaaat ccattggacc tctcaagact tgattgatgc aactcaaaat tttctacaac 10260
   atctaggtat tactgatgat atatacacaa tatatatatt agtgtcataa tactcaatcc 10320
   taatacttac cacatcatca aattattaac tcaaacaatt caagctatgg gacaaaatgg 10380
   atcccattat tagtggaaat tctgctaatg tttatctaac tgatagttat ttaaaaggtg 10440
   ttatttcttt ctcagaatgt aacgctttag gaagttacat attcaatggt ccttatctca 10500
   aaaatgatta taccaactta attagtagac aaaatccatt aatagaacac ataaatctaa 10560
   agaaactaaa tataacacag tccttaatat ctaagtatca taaaggtgaa ataaaaatag 10620
   aagaacctac ttactttcag tcattactta tgacatacaa gagtatgacc tcttcagaac 10680
   agactactac tactaattta cttaaaaaga taataagaag agctatagaa atcagtgatg 10740
   tcaaagtcta tgctatattg aataaactgg ggctcaaaga aaaagacaag attaaatcca 10800
   ataatggaca agatgaagac aactcagtca ttactaccat aatcaaagat gatatacttt 10860
   tagctgtcaa ggataatcaa tctcatctta aagcagacaa aaatcaatcc acaaaacaaa 10920
   aagatacaat caaaacaaca cttttgaaga aattaatgtg ttcgatgcaa catcctccat 10980
   catggttaat acattggttt aatttataca caaaattaaa cagcatatta acacaatatc 11040
   gatctagtga ggtaaaaaac catggtttta tattgataga taatcatact cttagtggat 11100
   tccaatttat tttgaatcaa tatggttgta tagtttatca taaggaactc aaaagaatta 11160
   ctgtgacaac ttataatcaa ttcttgacat ggaaagatat tagccttagt agattaaatg 11220
   tttgtttgat tacatggatt agtaactgtt tgaacacatt aaacaaaagc ttaggcttaa 11280
   gatgtggatt caataatgtt atcttgacac aattattcct ttatggagat tgtatactaa 11340
   aactattcca caatgagggg ttctacataa taaaagaggt agagggattt attatgtctc 11400
   taattttaaa tataacagaa gaagatcaat tcagaaaacg gttttataat agtatgctca 11460
   acaacatcac agatgccgcc aacaaagctc aaaaaaatct gctatcaaga gtatgtcata 11520
   cattattaga taagacaata tcagataata taataaatgg cagatggata attctattga 11580
   gtaagttcct aaaattaatt aagcttgcag gtgacaataa cctcaacaat ctgagtgaat 11640
   tatatttttt gttcagaata tttggacacc caatggtaga tgaaagacaa gccatggatg 11700
   ctgttaaagt taattgcaac gagaccaaat tttatttgtt aagtagtttg agtatgttaa 11760
   gaggagcttt tatatataga attataaaag ggtttgtaaa taattacaac agatggccta 11820
   ctttaagaaa tgccattgtc ttacccttaa gatggttaac ttactataaa ctaaacactt 11880
   atccttcctt gttggaactt acagaaagag atttgattgt tctatcagga ctacgtttct 11940
   atcgagagtt tcggttgcct aaaaaagtgg atcttgaaat gatcataaat gataaggcta 12000
   tatcacctcc taaaaattta atatggacta gtttccctag aaattatatg ccgtcacaca 12060
   tacaaaatta tatagaacat gaaaaattaa aattctctga tagtgataaa tcaagaagag 12120
   tattagagta ttatttaaga gataacaaat tcaatgaatg tgatttacac aactgtgtag 12180
   ttaatcaaag ttatcttaac aacccgaatc atgtggtatc attgacaggc aaagaaagag 12240
   aactcagtgt aggtagaatg tttgcaatgc aaccaggaat gttcagacaa gttcaaatat 12300
   tagcagagaa aatgatagca gaaaacatat tacaattttt ccctgaaagt cttacaagat 12360
   atggtgatct agaactacag aaaatattag aattgaaagc aggaataagt aacaaatcaa 12420
   atcgttacaa tgataattac aacaattaca ttagtaagtg ctctatcatc acagatctca 12480
   gcaaattcaa tcaagcattt cgatatgaaa catcatgtat ttgtagtgat gtactggatg 12540
   aactgcatgg tgtacaatct ctattttcct ggttacattt aactattcct catgtcacaa 12600
   taatatgcac atataggcat gcacccccct atataaagga tcatattgta gatcttaaca 12660
   atgtagatga gcaaagtgga ctatatagat atcatatggg tggtatcgaa gggtggtgtc 12720
   aaaaactatg gaccatagaa gctatatcac tattagatct aatatctctc aaagggaaat 12780
   tctcaattac tgctttaatt aatggtgaca atcaatcaat agatataagt aaaccagtca 12840
   gactcatgga aggtcaaact catgctcaag cagattattt gctagcatta aatagtctca 12900
   aattactgta taaagagtat gcaggaatag gccacaaatt aaaaggaact gagacttata 12960
   tatcgagaga tatgcaattt atgagtaaaa cgatccaaca taacggtgta tattacccag 13020
   ctagtataaa gaaagtccta agagtgggac cgtggataaa cactatactt gatgacttca 13080
   aagtgagtct agaatctata ggtagtttga cacaagaatt agaatataga ggtgaaagtc 13140
   tattatgcag tttaatattt agaaatgtat ggttatataa tcaaattgca ttacaactta 13200
   aaaatcatgc attatgtaac aacaaattat atttggatat attaaaagtt ctaaaacact 13260
   taaaaacctt ttttaatctt gataacattg atacagcatt aacattgtat atgaatttgc 13320
   ccatgttatt tggtggtggt gatcccaact tgttatatcg aagtttctat agaagaactc 13380
   ctgatttcct cacagaggct atagttcact ctgtgttcat acttagttat tatacaaacc 13440
   atgatttaaa agataaactt caagatctgt cagatgatag attgaataag ttcttaacat 13500
   gcataatcac gtttgataaa aaccccaatg ctgaattcgt tacattgatg agagatcctc 13560
   aagctttagg atctgagagg caagctaaaa ttactagcga aatcaataga ctggcagtta 13620
   ccgaggtttt gagcacagct ccaaacaaaa tattttccaa aagtgcacaa cactatacca 13680
   ctacagagat agatcttaat gatattatgc aaaatataga acctacatat cctcacgggc 13740
   taagagttgt ttatgagagt ttaccctttt ataaagcaga gaaaatagta aatcttatat 13800
   ccggtacaaa atctataact aacatactgg aaaagacttc tgccatagac ttaacagata 13860
   ttgatagagc cactgagatg atgaggaaaa acataacttt gcttataagg atattaccat 13920
   tagattgtaa cagagataaa agagaaatat tgagtatgga aaacctaagt attactgaat 13980
   taagcaaata cgttagagaa agatcttggt ctttatccaa tatagttggt gttacatcac 14040
   ccagtatcat gtatacaatg gacataaaat atacaacaag cactatagct agtggcataa 14100
   tcatagagaa atataatgtc aacagtttaa cacgtggtga gagaggaccc actaaaccat 14160
   gggttggttc atctacacaa gagaaaaaga caatgccagt ttataataga caagttttaa 14220
   ccaaaaaaca gagagatcaa atagatctat tagcaaaatt ggattgggtg tatgcatcta 14280
   tagataacaa ggatgaattt atggaggaac ttagcatagg aactcttggg ttaacatatg 14340
   agaaggccaa aaaattattc ccacaatatt tgagtgttaa ctatttgcat cgtcttacag 14400
   tcagtagtag accatgtgaa ttccctgcat ctataccagc ttatagaact acaaattatc 14460
   actttgatac tagccctatt aatcgcatat taacagaaaa gtatggtgat gaagatattg 14520
   atatagtatt ccaaaactgt ataagctttg gccttagctt aatgtctgta gtagaacaat 14580
   ttactaatgt atgtcctaac agaattattc tcatacccaa gcttaatgag atacatttga 14640
   tgaaacctcc catattcaca ggcgatgttg atattcacaa gttaaaacaa gtgatacaaa 14700
   aacaacatat gtttttacca gacaaaataa gtttgactca atatgtggaa ttattcttaa 14760
   gtaataaaac actcaaatct ggatctaatg ttaattctaa tttaatattg gcgcataaga 14820
   tatctgacta ttttcataat acttacattt tgagtactaa tttagctgga cattggattc 14880
   ttattataca acttatgaaa gattctaagg gtatttttga aaaagattgg ggagagggat 14940
   atataactga tcatatgttc attaatttga aagttttctt caatgcttat aagacatatc 15000
   tcttgtgttt tcataaaggt tacggcagag caaagctgga gtgtgatatg aatacttcag 15060
   atctcctatg tgtattggaa ttaatagaca gtagttattg gaagtctatg tctaaggtgt 15120
   ttttagaaca aaaagttatc aaatacattc ttagccagga tgcaagttta catagagtaa 15180
   aaggatgtca tagcttcaaa ctatggtttc ttaaacgtct taatgtagca gaattcacag 15240
   tttgcccttg ggttgttaac atagattatc atccaacaca tatgaaagca atattaactt 15300
   atattgatct tgttagaatg ggattgataa atatagatag aatatacatt aaaaataaac 15360
   acaagttcaa tgatgagttt tatacttcta atctgtttta cattaattat aacttctcag 15420
   ataatactca tctattaact aaacatataa ggattgctaa ttccgaatta gaaagtaatt 15480
   acaacaaatt atatcatcct acaccagaaa ccctagaaaa tatactaacc aatccggtta 15540
   aaagtaatga gaaaaagaca ctgagtgact attgtatagg taaaaatgtt gactcaataa 15600
   tgttaccatc gttatctaat aagaagctta ttaaatcgtc tacaatgatt agaaccaatt 15660
   acagcagaca agatttgtat aatttatttc ctacggttgt gattgataaa attatagatc 15720
   attcaggtaa tacagccaaa tctaaccaac tttacactac tacttctcat caaatatcct 15780
   tagtgcacaa tagcacatca ctttattgca tgcttccttg gcatcatatt aatagattca 15840
   attttgtatt tagttctaca ggttgtaaaa ttagtataga gtatatttta aaagatctta 15900
   aaattaagga tcctaattgt atagcattca taggtgaagg agcagggaat ttattattgc 15960
   gtacagtagt ggaacttcat cctgatataa gatatattta cagaagtctg aaagattgca 16020
   atgatcatag tttaccaatt gagtttttaa ggctgtacaa tggacatatc aacattgatt 16080
   atggtgaaaa tttgaccatt cctgctacag atgcaaccaa caacattcat tggtcttatt 16140
   tacatataaa gtttgctgaa cctatcagtc tttttgtctg tgatgctgaa ttgcctgtaa 16200
   cagtcaactg gagtaagatt ataatagagt ggagcaagca tgtaagaaaa tgcaagtact 16260
   gttcttcagt taataaatgt acattgatag taaaatatca tgctcaagat gatatcgatt 16320
   tcaaattaga caacataact atattaaaaa cttatgtatg cttaggtagt aagttaaagg 16380
   gatctgaagt ttacttagtc cttacaatag gtcctgcaaa tgtgttccca gtatttaatg 16440
   tagtacaaaa tgctaaattg atactatcaa gaactaaaaa tttcatcatg cctaaaaaag 16500
   ctgataaaga gtctattgat gcaaatatta agagtttgat accctttctt tgttacccta 16560
   taacaaaaaa aggaattaat actgcattgt ctaaattaaa gagtgttgtt agtggagata 16620
   tactatcata ttctatagct ggacgtaatg aagttttcag caataaactt ataaatcata 16680
   agcatatgaa catcttaaag tggttcaatc atgttttaaa tttcagatca acagaattaa 16740
   actataatca tttatatatg gtagaatcta cttatcctca tctaagtgaa ttgttaaaca 16800
   gcttgacaac caatgaactt aaaaaactga ttaaaatcac aggtagtttg ttatacaact 16860
   tttataatga ataatgagca aaaatcttat aacaaaaata gctacacact aacattgtat 16920
   tcaattatag ttattgaaaa ttaataatta tataattttt aataacttct agtgaactaa 16980
   tcctaaaatt atcattttga tctaggaaga ataagtttaa atccaaatct aattggttta 17040
   tatgtatatt aactaaatta cgagatatta gtttttgaca ctttttttct cgtggccggc 17100
   atggtcccag cctcctcgct ggcgccggct gggcaacatg cttcggcatg gcgaatggga 17160
   ctagcataac cccttggggc ctctaaacgg gtcttgaggg gttttttg 17208

<210> 7

<211>     17217
<212>     DNA
<213>     Artificial Sequence

<220>
<223>     cRSVA_NDVF_A

<400>     7
taatacgact cactataggt tttttcgcgt ctgatgaggc cgttaggccg aaactcctct      60

ccggagtcac gcgaaaaaat gcgtacaaca aacttgcgta aaccaaaaaa atggggcaaa     120

taagaatttg ataagtacca cttaaattta actcctttgg ttagaggcgc gccatgggca     180

gcaactcatt gagtatgata aaagttagat tgcaaaatct gtttgacaat gatgaagtag     240

cattgttaaa aataacatgc tatactgaca aattaataca gttaactaat gctttggcta     300

aggcagttat acatacaatc aaattgaatg gcattgtatt tgtgcatgtt attacaagta     360

gtgatatttg ccctaataat aatattgtag tgaaatccaa tttcacaaca atgccagtat     420

tacaaaatgg aggttatata tgggaaatga tggaattaac acactgctct caacctaatg     480

gcctaataga tgacaattgt gaaattaaat tctccaaaaa actaagtgat tcaacaatga     540

ccaattatat gaatcaatta tctgaattac ttggatttga cctcaatcca taaatcataa     600

taaatatcaa ctagcaaatc aatgtcacta acaccattag ttaatataaa acttgacaga     660

agataaaaat ggggcaaata aatcaattca gccgacccaa ccatggacac aacacacaat     720

gataccacac cacaaagact gatgatcaca gacatgaggc cattatcgct tgagactata     780

ataacatctc taaccagaga tatcataaca cataaattta tatacttgat aaatcatgaa     840

tgcatagtaa gaaaacttga tgaaagacag gccacattta catttctggt caactatgaa     900

atgaaactat tgcacaaagt gggaagcact aaatataaaa aatatactga atacaacaca     960

aaatatggca ctttccctat gccaatattt atcaatcatg atgggttctt agaatgcatt    1020

ggcattaagc ctaccaagca cacacccata atatacaagt atgatctcaa tccatgaata    1080

tcaaaccaag attcaaacaa tccgaaataa caactttatg cataatcaca ctccatagtc    1140

caaatggagc ctgaaaatta tagttattta aaattcctgc aggaaggaga gacataagat    1200

gaaagatggg gcaaatacaa aaatggctct tagcaaagtc aagttgaatg atacactcaa    1260

caaagatcaa cttctatcat ccagcaaata taccatccaa cggagcacag gagacagcat    1320

tgacactcct aattatgatg tgcagaaaca cattaataag ttatgtggca tgttattaat    1380

cacagaagat gctaatcata aattcactgg gttaataggt atgttatatg ctatgtctag    1440

attaggaaga gaagacacca taaaaatact caaagatgcg ggatatcatg ttaaggcaaa    1500

tggagtggat gtaacaacac atcgtcaaga cattaatggg aaagaaatga aatttgaagt    1560

gttaacatta gcaagcttaa caactgaaat tcaaatcaac attgagatag aatctagaaa    1620

atcctacaaa aaaatgctaa aagaaatggg agaggtggct ccagaataca ggcatgactc 1680

tcctgattgt gggatgataa tattatgtat agcggcatta gtaataacca aattagcagc 1740

aggagataga tcaggtctta cagctgtgat taggagagct aataatgtcc taaaaaatga 1800

aatgaaacgt tataaaggtt tattacccaa ggatatagcc aacagcttct atgaagtgtt 1860

tgaaaaatat cctcacttta tagatgtttt tgttcatttt ggtatagcac aatcttctac 1920

cagaggtggc agtagagttg aagggatttt tgcaggattg tttatgaatg cctatggtgc 1980

agggcaagtg atgttacggt ggggggtctt agcaaaatca gttaaaaaca ttatgttagg 2040

acacgctagt gtacaagcag aaatggaaca agttgtggag gtgtatgagt atgctcagaa 2100

attgggtgga gaagcaggat tctaccatat attgaacaac ccaaaagcat cactattatc 2160

tttgactcaa tttcctcact tctctagtgt agtattgggc aatgctgctg gcctaggcat 2220

aatgggagaa tacagaggta caccaaggaa tcaagattta tatgatgctg caaaagcata 2280

tgctgaacaa ctcaaagaaa atggtgtgat taactacagt gtattagatt tgacagcaga 2340

agaactagag gctatcaaac atcagcttaa tccaaaagat aatgatgtag agctttgagt 2400

taataaaaaa gtggggcaaa taaatcatca tggaaaagtt tgctcctgaa ttccatggag 2460

aagatgcaaa caacagagcc accaaattcc tagaatcaat aaagggcaaa ttcacatcac 2520

ccaaagatcc caagaaaaaa gatagtatca tatctgtcaa ctcaatagat atagaagtaa 2580

ccaaagaaag ccctataaca tcaaattcaa ccattataaa cccaataaat gagacagatg 2640

atactgtagg gaacaagccc aattatcaaa gaaagcctct agtaagtttc aaagaagacc 2700

ctacgccaag tgataatcct ttttcaaaac tatacaaaga aaccatagaa acatttgata 2760

acaatgaaga agaatctagc tattcatatg aagaaataaa tgatcagaca aacgataata 2820

taacagcaag attagatagg attgatgaga aattaagtga aatactagga atgcttcaca 2880

cattagtagt agcgagtgca ggacccacat ctgctcggga tggtataaga gatgccatgg 2940

ttggtttaag agaagaaatg atagaaaaaa tcagaactga agcattaatg accaatgaca 3000

gactagaagc tatggcaaga ctcaggaatg aagaaagtga aaagatggca aaagacacat 3060

cagatgaagt gtctctcaat ccaacatcag agaaactgaa caacctgttg gaagggaatg 3120

atagtgacaa tgatctatca cttgaagatt tctgattagc taccaaactg tacatcaaaa 3180

cacaacacca atagaaaacc aacaaacaaa ccaactcacc catccaacca aacatctatc 3240

tgctgattag ccaaccagcc aaaaaacaac cagccaatct aaaactagcc acccggaaaa 3300

aatcgatact atagttacaa aaaaagatgg ggcaaatatg gaaacatacg tgaataaact 3360

tcacgagggc tccacataca cagctgctgt tcaatacaat gtcctagaaa aagacgatga 3420

tcctgcatca cttacaatat gggtgcccat gttccaatca tccatgccag cagatctact 3480

cataaaagaa ctagccaatg tcaatatact agtgaaacaa atatccacac ccaagggacc 3540

ctcattaaga gtcatgataa actcaagaag tgcagtgcta gcacaaatgc ccagcaaatt 3600

taccatatgt gccaatgtgt ccttggatga aagaagcaag ctggcatatg atgtaaccac 3660

accctgtgaa attaaggcat gcagtctaac atgcctaaaa tcaaaaaata tgttaactac 3720

agttaaagat ctcactatga aaacactcaa cccaacacat gacatcattg ctttatgtga 3780

atttgaaaat atagtaacat caaaaaaagt cataatacca acatacctaa gatctatcag 3840

cgtcagaaat aaagatctga acacacttga aaatataaca accactgaat tcaaaaatgc 3900

cattacaaat gcaaaaatca tcccttactc aggattattg ttagtcatca cagtgactga 3960

caacaaagga gcattcaaat acataaagcc acaaagtcaa ttcatagtag atcttggagc 4020

ttacctagaa aaagaaagta tatattatgt tacaacaaat tggaagcaca cagctacacg 4080

atttgcaatc aaacccatgg aagattaacc tttttcctct acatcaatga gtagattcat 4140

acaaactttc taactacatt cttcacttca caatcataat caccaaccct ctgtggttca 4200

atcaatcaaa caaaactcat caagagttcc agatcatccc aagtcattgt tcatcagatc 4260

cagtactcaa ataagttaat aaaaaatcca catggggcaa ataatcattg agggaaatcc 4320

aactaatcac aacatctgtc aacatagaca agtcaacacg ctagataaaa tcaaccaatg 4380

gaaaatacat ccataactat agaattctca agcaaattct ggccttactt tacactaata 4440

cacatgataa caacaataat ctctttgata atcataatct ccatcatgat tgcaatacta 4500

aacaaactct gcgaatataa tgtattccat aacaaaacct ttgagctacc aagagctcga 4560

gtcaatacat agcattcacc aatctgatag ctcaaaacag taaccttgca tttgtaaatg 4620

aactaccctc acttcttcac aaaaccacat caacatctca ccatgcaagc catcatctat 4680

accataaagt agttaattaa aaatggccgg ccagtcataa caatgaacta ggatattaag 4740

accaaaaaca acgctggggc aaatgcaaac atgtccaaaa ccgaggacca acgcaccgcc 4800

aagacactag aaaggacctg ggacactttt aatcatctat tattcatatc atcgtgctta 4860

tacaagttaa atcttaaatc tatagcacaa atcacattat ctattttggc aattataatc 4920

tcaacctcac ttataattgc agccatcata ttcatagcct cggcaaacca caaagtcaca 4980

ctaacaactg caatcataca agatgcaacg aaccagatca agaacacaac cccaacatac 5040

ctcacccaga atccccagca tggaatcagc ttctccaatc tgtccggaac tacatcacaa 5100

tccaccacca tactagcttc aacaacacca agtgctgatt caaccccaca atccacaaca 5160

gtcaagatca aaaacacaac aacaacccaa atattaccta gcaaacccac cacaaaacaa 5220

cgccaaaata aaccacaaaa caaacccaac aatgattttc actttgaagt gttcaatttt 5280

gtaccctgca gcatatgcag caacaatcca acctgctggg ccatctgcaa gagaatacca 5340

aacaaaaaac ctggaaagaa aaccaccacc aagcccacaa aaaaaccaac cctcaagaca 5400

accaaaaaag atcccaaatc ccaaaccaca aaaccaaagg aagtactcac taccaagcct 5460

acaggaaagc caaccatcaa caccactaaa acaaacatca gaactacact gctcacctcc 5520

aacaccaaag gaaatccaga acacacaagt caagaggaaa ccctccactc aaccacctcc 5580

gaaggctatc caagcccatc acaagtccac acaacatccg gtcaagagga aaccctccac 5640

tcaaccacct ccgaaggcta tccaagccca tcacaagtct acacaacatc cgagtaccta 5700

tcacaatctc tatcttcatc caacacaaca aaatgatagt cattaaaaag cacgcgtgta 5760

ttgttgcaaa aagccatgac caaatcaaac agaatcaaaa tcaactctgg ggcaaataac 5820

aatgggttcc aagtcttcta ccaggttccc agtacctcta atgctgaccg tccgagtcat 5880

gctggcactg agttgcgtct gtccggccag cgcccttgat ggcaggcctc ttgcggctgc 5940

agggattgtg gtaacaggag acaaagcggt caacatatac acatcatctc agacagggtc 6000

aatcataatc aagttactcc cgaatatgcc caaggataaa gaggcgtgtg caaaagcacc 6060

gttggaggca tacaacagga cattgactac tttgctcacc cctcttggtg attctatccg 6120

taggatacaa gagtctgtga ccacgtccgg aggaagaaaa cagggacgcc ttataggagc 6180

cattatcggt ggtgtagctc tcggggtcgc gaccgctgca caaataacag cagcctcggc 6240

tttgatacaa gccaatcaaa atgctgccaa catcctccgg ctcaaagaga gcattgctgc 6300

aaccaatgag gctgtacacg aggtcactga cggattatca caactagcag tggcagttgg 6360

gaagatgcag caatttgtta atgaccagtt taataagaca gcccaggaat tggactgtat 6420

aaaaattgca cagcaagttg gtgtagaact caacctgtac ttaactgaat tgactacagt 6480

atttgggccg caaatcactt cccctgcctt aactcagctg actatccagg cgctttacaa 6540

tctagctggt gggaatatgg attacttgtt aactaagtta ggtgtgggga ataaccaact 6600

cagctcatta attagtagtg gcctgatcac cggcaaccct attctgtatg actcacagac 6660

tcaactcttg ggtatacagg taatcctacc ctcagtcggg aatctaaata atatgcgtgc 6720

cacctatctg gaaaccttgt ctgtaagtac aactaaagga tttgcctcag ctcttgtccc 6780

aaaggtagtg acacaggtcg gttctgtgat agaagagctt gacacctcat actgtataga 6840

gaccgatttg gatctatatt gtacaagaat agtgacattc cctatgtctc ctggtattta 6900

ttcctgtttg agtggcaata catctgcttg catgtactca aagacagaag gcgcactcac 6960

tacgccgtat atgaccctca aaggctcagt tattgcaaac tgtaagatga caacatgtag 7020

gtgtgcagat cccccgggta tcatatcgca aaattatgga gaagctgtgt ctctaataga 7080

taggcaatca tgcaatatcc tatccttaga cgggataact ttgaggctca gtggggaatt 7140

cgatgcaact tatcaaagga atatctcaat acaagattct caagttatag tgacaggcaa 7200

tcttgatatc tcaactgagc ttgggaatgt caacaactcg ataagcaatg ctttggataa 7260

gttagaggaa agcaacagca aactagacaa ggtcaatgtc aaattggcca gcacatccgc 7320

tataattata gtaattatag taatattgtt agcattaatt gcagttggac tgcttctata 7380

ctgcaaggcc agaagcacac cagtcacatt aagtaaggat caactgagtg gtataaataa 7440

tattgcattt agtaactgat agtcattaaa aagcacgcgt gtattgttgc aaaaagccat 7500

gaccaaatca aacagaatca aaatcaactc tggggcaaat aacaatggag ttgccaatcc 7560

tcaaaacaaa tgctattacc acaatccttg ctgcagtcac actctgtttc gcttccagtc 7620

aaaacatcac tgaagaattt tatcaatcaa catgcagtgc agtcagcaaa ggctatctta 7680

gtgctctaag aactggttgg tatactagtg ttataactat agaattaagt aatatcaagg 7740

aaaataagtg taatggtaca gacgctaagg taaaattaat aaaacaagaa ttagataaat 7800

ataaaaatgc tgtaacagaa ttgcagttgc tcatgcaaag cacaccagca gccaacaatc 7860

gagccagaag agaactacca agatttatga attatacact caacaatacc aaaaacacca 7920

atgtaacatt aagtaagaaa aggaaaagaa gatttcttgg atttttgtta ggtgttggat 7980

ctgcaatcgc cagtggcatt gccgtatcca aggtcctgca cctagaaggg gaagtgaaca 8040

aaatcaaaag tgctctacta tccacaaaca aggctgtagt cagcttatct aatggagtca 8100

gtgtcttaac cagcaaggtg ttagacctca aaaactatat agataaacag ttgttaccta 8160

ttgttaacaa gcaaagctgc agcatatcaa acattgaaac tgtgatagag ttccaacaaa 8220

agaacaacag actactagag attaccagag aatttagtgt taatgcaggt gtaactactc 8280

ctgtaagcac ttatatgtta actaatagtg agttattatc attaatcaat gatatgccta 8340

taacaaatga tcagaaaaag ttaatgtcca gcaatgttca aatagttaga cagcaaagtt 8400

actctatcat gtcaataata aaagaggaag tcttggcata tgtagtacaa ttaccactat 8460

atggtgtaat agatactcct tgttggaaac tacacacatc ccctttatgt acaaccaaca 8520

caaaggaagg atccaacatc tgcttaacaa gaaccgacag aggatggtac tgtgacaatg 8580

caggatcagt atcctttttc ccacaagctg aaacatgtaa agttcaatcg aatcgggtgt 8640

tttgtgacac aatgaacagt ttaacattac caagtgaggt aaatctctgc aacattgaca 8700

tattcaaccc caaatatgat tgcaaaatta tgacttcaaa aacagatgta agcagctccg 8760

ttatcacatc tctaggagcc attgtgtcat gctatggcaa aaccaaatgt acagcatcca 8820

ataaaaatcg tgggatcata aagacattct ctaacgggtg tgattatgta tcaaataagg 8880

gggtggatac tgtgtctgta ggtaatacat tatattatgt aaataagcaa gaaggcaaaa 8940

gtctctatgt aaaaggtgaa ccaataataa atttctatga tccattagtg ttcccctctg 9000

atgaatttga tgcatcaata tctcaagtca atgagaaaat taatcagagt ctagcattta 9060

tccgtaaatc agatgaatta ttacataatg taaatgctgg taaatccacc acaaatatca 9120

tgataactac cataattata gtaattatag taatattgtt agcattaatt gcagttggac 9180

tgcttctata ctgcaaggcc agaagcacac cagtcacatt aagtaaggat caactgagtg 9240

gtataaataa tattgcattt agtaactgaa taaaaatagc acctaatcat attcttacaa 9300

tggttcgcta tttgaccata gataacccat ctatcattag attatcctaa aatttgaact 9360

tcatcacaac tttcatctat aaaccatctc acttacactt tttaagtaga tttctatttt 9420

atagttatat aaaacagggc ccattgaata ccaaattaac ttactatttg taaaaatgag 9480

aattggggca aatatgtcac gaaggaatcc ttgcaaattc gaaattcgag gtcattgctt 9540

gaatggtaaa aggtgtcatt ttagtcataa ttattttgaa tggccacccc atgcactgct 9600

tgtaagacaa aactttatgt taaacagaat acttaagtct atggataaaa gcatagatac 9660

tttgtcagaa ataagtggag ctgcagagtt ggacagaaca gaagagtatg ccctcggtgt 9720

agttggagtg ctagagagtt atataggatc aataaataat ataactaaac aatcagcatg 9780

tgttgccatg agcaaactcc ttactgaact caacagcgat gacatcaaaa aactaaggga 9840

caatgaagag ccaaactcac ccaaagtaag agtgtacaat actgtcatat catatattga 9900

aagcaacagg aagaacaata aacaaactat ccatctgtta aaaagattgc cagcagacgt 9960

attgaagaaa accatcaaaa acacattgga tatccacaag agcataacca tcaataaccc 10020

aaaagaatca actgttagtg atacgaacga ccatgccaaa aataatgata ctacctgaca 10080

aatatccttg tagtataaat tccatactaa taacaagtaa ttgtagagtc actatgtata 10140

atcaaaaaaa cacactatat atcaatcaaa acaaccaaaa tagccatata tacccaccgg 10200

atcaaccatt caatgaaatc cattggacct ctcaagactt gattgatgca actcaaaatt 10260

ttctacaaca tctaggtatt actgatgata tatacacaat atatatatta gtgtcataat 10320

actcaatcct aatacttacc acatcatcaa attattaact caaacaattc aagctatggg 10380

acaaaatgga tcccattatt agtggaaatt ctgctaatgt ttatctaact gatagttatt 10440

taaaaggtgt tatttctttc tcagaatgta acgctttagg aagttacata ttcaatggtc 10500

cttatctcaa aaatgattat accaacttaa ttagtagaca aaatccatta atagaacaca 10560

taaatctaaa gaaactaaat ataacacagt ccttaatatc taagtatcat aaaggtgaaa 10620

taaaaataga agaacctact tactttcagt cattacttat gacatacaag agtatgacct 10680

cttcagaaca gactactact actaatttac ttaaaaagat aataagaaga gctatagaaa 10740

tcagtgatgt caaagtctat gctatattga ataaactggg gctcaaagaa aaagacaaga 10800

ttaaatccaa taatggacaa gatgaagaca actcagtcat tactaccata atcaaagatg 10860

atatactttt agctgtcaag gataatcaat ctcatcttaa agcagacaaa aatcaatcca 10920

caaaacaaaa agatacaatc aaaacaacac ttttgaagaa attaatgtgt tcgatgcaac 10980

atcctccatc atggttaata cattggttta atttatacac aaaattaaac agcatattaa 11040

cacaatatcg atctagtgag gtaaaaaacc atggttttat attgatagat aatcatactc 11100

ttagtggatt ccaatttatt ttgaatcaat atggttgtat agtttatcat aaggaactca 11160

aaagaattac tgtgacaact tataatcaat tcttgacatg gaaagatatt agccttagta 11220

gattaaatgt ttgtttgatt acatggatta gtaactgttt gaacacatta aacaaaagct 11280

taggcttaag atgtggattc aataatgtta tcttgacaca attattcctt tatggagatt 11340

gtatactaaa actattccac aatgaggggt tctacataat aaaagaggta gagggattta 11400

ttatgtctct aattttaaat ataacagaag aagatcaatt cagaaaacgg ttttataata 11460

gtatgctcaa caacatcaca gatgccgcca acaaagctca aaaaaatctg ctatcaagag 11520

tatgtcatac attattagat aagacaatat cagataatat aataaatggc agatggataa 11580

ttctattgag taagttccta aaattaatta agcttgcagg tgacaataac ctcaacaatc 11640

tgagtgaatt atattttttg ttcagaatat ttggacaccc aatggtagat gaaagacaag 11700

ccatggatgc tgttaaagtt aattgcaacg agaccaaatt ttatttgtta agtagtttga 11760

gtatgttaag aggagctttt atatatagaa ttataaaagg gtttgtaaat aattacaaca 11820

gatggcctac tttaagaaat gccattgtct tacccttaag atggttaact tactataaac 11880

taaacactta tccttccttg ttggaactta cagaaagaga tttgattgtt ctatcaggac 11940

tacgtttcta tcgagagttt cggttgccta aaaaagtgga tcttgaaatg atcataaatg 12000

ataaggctat atcacctcct aaaaatttaa tatggactag tttccctaga aattatatgc 12060

cgtcacacat acaaaattat atagaacatg aaaaattaaa attctctgat agtgataaat 12120

caagaagagt attagagtat tatttaagag ataacaaatt caatgaatgt gatttacaca 12180

actgtgtagt taatcaaagt tatcttaaca acccgaatca tgtggtatca ttgacaggca 12240

aagaaagaga actcagtgta ggtagaatgt ttgcaatgca accaggaatg ttcagacaag 12300

ttcaaatatt agcagagaaa atgatagcag aaaacatatt acaatttttc cctgaaagtc 12360

ttacaagata tggtgatcta gaactacaga aaatattaga attgaaagca ggaataagta 12420

acaaatcaaa tcgttacaat gataattaca acaattacat tagtaagtgc tctatcatca 12480

cagatctcag caaattcaat caagcatttc gatatgaaac atcatgtatt tgtagtgatg 12540

tactggatga actgcatggt gtacaatctc tattttcctg gttacattta actattcctc 12600

atgtcacaat aatatgcaca tataggcatg caccccccta tataaaggat catattgtag 12660

atcttaacaa tgtagatgag caaagtggac tatatagata tcatatgggt ggtatcgaag 12720

ggtggtgtca aaaactatgg accatagaag ctatatcact attagatcta atatctctca 12780

aagggaaatt ctcaattact gctttaatta atggtgacaa tcaatcaata gatataagta 12840

aaccagtcag actcatggaa ggtcaaactc atgctcaagc agattatttg ctagcattaa 12900

atagtctcaa attactgtat aaagagtatg caggaatagg ccacaaatta aaaggaactg 12960

agacttatat atcgagagat atgcaattta tgagtaaaac gatccaacat aacggtgtat 13020

attacccagc tagtataaag aaagtcctaa gagtggaccc gtggataaac actatacttg 13080

atgacttcaa agtgagtcta gaatctatag gtagtttgac acaagaatta gaatatagag 13140

gtgaaagtct attatgcagt ttaatattta gaaatgtatg gttatataat caaattgcat 13200

tacaacttaa aaatcatgca ttatgtaaca acaaattata tttggatata ttaaaagttc 13260

taaaacactt aaaaaccttt tttaatcttg ataacattga tacagcatta acattgtata 13320

tgaatttgcc catgttattt ggtggtggtg atcccaactt gttatatcga agtttctata 13380

gaagaactcc tgatttcctc acagaggcta tagttcactc tgtgttcata cttagttatt 13440

atacaaacca tgatttaaaa gataaacttc aagatctgtc agatgataga ttgaataagt 13500

tcttaacatg cataatcacg tttgataaaa accccaatgc tgaattcgtt acattgatga 13560

gagatcctca agctttagga tctgagaggc aagctaaaat tactagcgaa atcaatagac 13620

tggcagttac cgaggttttg agcacagctc caaacaaaat attttccaaa agtgcacaac 13680

actataccac tacagagata gatcttaatg atattatgca aaatatagaa cctacatatc 13740

ctcacgggct aagagttgtt tatgagagtt taccctttta taaagcagag aaaatagtaa 13800

atcttatatc cggtacaaaa tctataacta acatactgga aaagacttct gccatagact 13860

taacagatat tgatagagcc actgagatga tgaggaaaaa cataactttg cttataagga 13920

tattaccatt agattgtaac agagataaaa gagaaatatt gagtatggaa aacctaagta 13980

ttactgaatt aagcaaatac gttagagaaa gatcttggtc tttatccaat atagttggtg 14040

ttacatcacc cagtatcatg tatacaatgg acataaaata tacaacaagc actatagcta 14100

gtggcataat catagagaaa tataatgtca acagtttaac acgtggtgag agaggaccca 14160

ctaaaccatg ggttggttca tctacacaag agaaaaagac aatgccagtt tataatagac 14220

aagttttaac caaaaaacag agagatcaaa tagatctatt agcaaaattg gattgggtgt 14280

atgcatctat agataacaag gatgaattta tggaggaact tagcatagga actcttgggt 14340

taacatatga gaaggccaaa aaattattcc cacaatattt gagtgttaac tatttgcatc 14400

gtcttacagt cagtagtaga ccatgtgaat tccctgcatc tataccagct tatagaacta 14460

caaattatca ctttgatact agccctatta atcgcatatt aacagaaaag tatggtgatg 14520

aagatattga tatagtattc caaaactgta taagctttgg ccttagctta atgtctgtag 14580

tagaacaatt tactaatgta tgtcctaaca gaattattct catacccaag cttaatgaga 14640

tacatttgat gaaacctccc atattcacag gcgatgttga tattcacaag ttaaaacaag 14700

tgatacaaaa acaacatatg tttttaccag acaaaataag tttgactcaa tatgtggaat 14760

tattcttaag taataaaaca ctcaaatctg gatctaatgt taattctaat ttaatattgg 14820

cgcataagat atctgactat tttcataata cttacatttt gagtactaat ttagctggac 14880

attggattct tattatacaa cttatgaaag attctaaggg tatttttgaa aaagattggg 14940

gagagggata tataactgat catatgttca ttaatttgaa agttttcttc aatgcttata 15000

agacatatct cttgtgtttt cataaaggtt acggcagagc aaagctggag tgtgatatga 15060

atacttcaga tctcctatgt gtattggaat taatagacag tagttattgg aagtctatgt 15120

ctaaggtgtt tttagaacaa aaagttatca aatacattct tagccaggat gcaagtttac 15180

atagagtaaa aggatgtcat agcttcaaac tatggtttct taaacgtctt aatgtagcag 15240

aattcacagt ttgcccttgg gttgttaaca tagattatca tccaacacat atgaaagcaa 15300

tattaactta tattgatctt gttagaatgg gattgataaa tatagataga atatacatta 15360

aaaataaaca caagttcaat gatgagtttt atacttctaa tctgttttac attaattata 15420

acttctcaga taatactcat ctattaacta aacatataag gattgctaat tccgaattag 15480

aaagtaatta caacaaatta tatcatccta caccagaaac cctagaaaat atactaacca 15540

atccggttaa aagtaatgag aaaaagacac tgagtgacta ttgtataggt aaaaatgttg 15600

actcaataat gttaccatcg ttatctaata agaagcttat taaatcgtct acaatgatta 15660

gaaccaatta cagcagacaa gatttgtata atttatttcc tacggttgtg attgataaaa 15720

ttatagatca ttcaggtaat acagccaaat ctaaccaact ttacactact acttctcatc 15780

aaatatcctt agtgcacaat agcacatcac tttattgcat gcttccttgg catcatatta 15840

atagattcaa ttttgtattt agttctacag gttgtaaaat tagtatagag tatattttaa 15900

aagatcttaa aattaaggat cctaattgta tagcattcat aggtgaagga gcagggaatt 15960

tattattgcg tacagtagtg gaacttcatc ctgatataag atatatttac agaagtctga 16020

aagattgcaa tgatcatagt ttaccaattg agtttttaag gctgtacaat ggacatatca 16080

acattgatta tggtgaaaat ttgaccattc ctgctacaga tgcaaccaac aacattcatt 16140

ggtcttattt acatataaag tttgctgaac ctatcagtct ttttgtctgt gatgctgaat 16200

tgcctgtaac agtcaactgg agtaagatta taatagagtg gagcaagcat gtaagaaaat 16260

gcaagtactg ttcttcagtt aataaatgta cattgatagt aaaatatcat gctcaagatg 16320

atatcgattt caaattagac aacataacta tattaaaaac ttatgtatgc ttaggtagta 16380

agttaaaggg atctgaagtt tacttagtcc ttacaatagg tcctgcaaat gtgttcccag 16440

tatttaatgt agtacaaaat gctaaattga tactatcaag aactaaaaat ttcatcatgc 16500

ctaaaaaagc tgataaagag tctattgatg caaatattaa gagtttgata ccctttcttt 16560

gttaccctat aacaaaaaaa ggaattaata ctgcattgtc taaattaaag agtgttgtta 16620

gtggagatat actatcatat tctatagctg gacgtaatga agttttcagc aataaactta 16680

taaatcataa gcatatgaac atcttaaagt ggttcaatca tgttttaaat ttcagatcaa 16740

cagaattaaa ctataatcat ttatatatgg tagaatctac ttatcctcat ctaagtgaat 16800

tgttaaacag cttgacaacc aatgaactta aaaaactgat taaaatcaca ggtagtttgt 16860

tatacaactt ttataatgaa taatgagcaa aaatcttata acaaaaatag ctacacacta 16920

acattgtatt caattatagt tattgaaaat taataattat ataattttta ataacttcta 16980

gtgaactaat cctaaaatta tcattttgat ctaggaagaa taagtttaaa tccaaatcta 17040

attggtttat atgtatatta actaaattac gagatattag tttttgacac tttttttctc 17100

gtggccggca tggtcccagc ctcctcgctg gcgccggctg ggcaacatgc ttcggcatgg 17160

cgaatgggac tagcataacc ccttggggcc tctaaacggg tcttgagggg ttttttg 17217

<210> 8
<211> 1176
<212> DNA
<213> respiratory syncytial virus

<400> 8
atggctctta gcaaagtcaa gttgaatgat acactcaaca aagatcaact tctatcatcc 60

agcaaatata ccatccaacg gagcacagga gacagcattg acactcctaa ttatgatgtg 120

cagaaacaca ttaataagtt atgtggcatg ttattaatca cagaagatgc taatcataaa 180

ttcactgggt taataggtat gttatatgct atgtctagat taggaagaga agacaccata 240

aaaatactca aagatgcggg atatcatgtt aaggcaaatg gagtggatgt aacaacacat 300

cgtcaagaca ttaatgggaa agaaatgaaa tttgaagtgt taacattagc aagcttaaca 360

actgaaattc aaatcaacat tgagatagaa tctagaaaat cctacaaaaa aatgctaaaa 420

gaaatgggag aggtggctcc agaatacagg catgactctc ctgattgtgg gatgataata 480

ttatgtatag cggcattagt aataaccaaa ttagcagcag gagatagatc aggtcttaca 540

gctgtgatta ggagagctaa taatgtccta aaaaatgaaa tgaaacgtta taaaggttta 600

ttacccaagg atatagccaa cagcttctat gaagtgtttg aaaaatatcc tcactttata 660

gatgtttttg ttcattttgg tatagcacaa tcttctacca gaggtggcag tagagttgaa 720

gggatttttg caggattgtt tatgaatgcc tatggtgcag ggcaagtgat gttacggtgg 780

ggggtcttag caaaatcagt taaaaacatt atgttaggac acgctagtgt acaagcagaa 840

atggaacaag ttgtggaggt gtatgagtat gctcagaaat tgggtggaga agcaggattc 900

taccatatat tgaacaaccc aaaagcatca ctattatctt tgactcaatt tcctcacttc 960

tctagtgtag tattgggcaa tgctgctggc ctaggcataa tgggagaata cagaggtaca 1020

ccaaggaatc aagatttata tgatgctgca aaagcatatg ctgaacaact caaagaaaat 1080

ggtgtgatta actacagtgt attagatttg acagcagaag aactagaggc tatcaaacat 1140

cagcttaatc caaaagataa tgatgtagag ctttga 1176

<210>    9
<211>    726
<212>    DNA
<213>    respiratory syncytial virus

<400>    9
atggaaaagt ttgctcctga attccatgga gaagatgcaa acaacagagc caccaaattc      60

ctagaatcaa taaagggcaa attcacatca cccaaagatc ccaagaaaaa agatagtatc     120

atatctgtca actcaataga tatagaagta accaaagaaa gccctataac atcaaattca     180

accattataa acccaataaa tgagacagat gatactgtag ggaacaagcc caattatcaa     240

agaaagcctc tagtaagttt caaagaagac cctacgccaa gtgataatcc tttttcaaaa     300

ctatacaaag aaaccataga aacatttgat aacaatgaag aagaatctag ctattcatat     360

gaagaaataa atgatcagac aaacgataat ataacagcaa gattagatag gattgatgag     420

aaattaagtg aaatactagg aatgcttcac acattagtag tagcgagtgc aggacccaca     480

tctgctcggg atggtataag agatgccatg gttggtttaa gagaagaaat gatagaaaaa     540

atcagaactg aagcattaat gaccaatgac agactagaag ctatggcaag actcaggaat     600

gaagaaagtg aaaagatggc aaaagacaca tcagatgaag tgtctctcaa tccaacatca     660

gagaaactga acaacctgtt ggaagggaat gatagtgaca atgatctatc acttgaagat     720

ttctga                                                                726

<210>    10
<211>    585
<212>    DNA
<213>    respiratory syncytial virus

<400>    10
atgtcacgaa ggaatccttg caaattcgaa attcgaggtc attgcttgaa tggtaaaagg      60

tgtcatttta gtcataatta ttttgaatgg ccaccccatg cactgcttgt aagacaaaac     120

tttatgttaa acagaatact taagtctatg gataaaagca tagatacttt gtcagaaata     180

agtggagctg cagagttgga cagaacagaa gagtatgccc tcggtgtagt tggagtgcta     240

gagagttata taggatcaat aaataatata actaaacaat cagcatgtgt tgccatgagc     300

aaactcctta ctgaactcaa cagcgatgac atcaaaaaac taagggacaa tgaagagcca     360

aactcaccca aagtaagagt gtacaatact gtcatatcat atattgaaag caacaggaag     420

aacaataaac aaactatcca tctgttaaaa agattgccag cagacgtatt gaagaaaacc     480

atcaaaaaca cattggatat ccacaagagc ataaccatca ataacccaaa agaatcaact     540

gttagtgata cgaacgacca tgccaaaaat aatgatacta cctga                     585

<210>    11
<211>    6498

<212>    DNA
<213>    respiratory syncytial virus

<400>    11
atggatccca ttattagtgg aaattctgct aatgtttatc taactgatag ttatttaaaa      60

ggtgttattt ctttctcaga atgtaacgct ttaggaagtt acatattcaa tggtccttat     120

ctcaaaaatg attataccaa cttaattagt agacaaaatc cattaataga acacataaat     180

ctaaagaaac taaatataac acagtcctta atatctaagt atcataaagg tgaaataaaa     240

atagaagaac ctacttactt tcagtcatta cttatgacat acaagagtat gacctcttca     300

gaacagacta ctactactaa tttacttaaa aagataataa gaagagctat agaaatcagt     360

gatgtcaaag tctatgctat attgaataaa ctggggctca aagaaaaaga caagattaaa     420

tccaataatg gacaagatga agacaactca gtcattacta ccataatcaa agatgatata     480

cttttagctg tcaaggataa tcaatctcat cttaaagcag acaaaaatca atccacaaaa     540

caaaaagata caatcaaaac aacacttttg aagaaattaa tgtgttcgat gcaacatcct     600

ccatcatggt taatacattg gtttaattta tacacaaaat taaacagcat attaacacaa     660

tatcgatcta gtgaggtaaa aaaccatggt tttatattga tagataatca tactcttagt     720

ggattccaat ttattttgaa tcaatatggt tgtatagttt atcataagga actcaaaaga     780

attactgtga caacttataa tcaattcttg acatggaaag atattagcct tagtagatta     840

aatgtttgtt tgattacatg gattagtaac tgtttgaaca cattaaacaa aagcttaggc     900

ttaagatgtg gattcaataa tgttatcttg acacaattat tcctttatgg agattgtata     960

ctaaaactat tccacaatga ggggttctac ataataaaag aggtagaggg atttattatg    1020

tctctaattt taaatataac agaagaagat caattcagaa aacggtttta taatagtatg    1080

ctcaacaaca tcacagatgc cgccaacaaa gctcaaaaaa atctgctatc aagagtatgt    1140

catacattat tagataagac aatatcagat aatataataa atggcagatg gataattcta    1200

ttgagtaagt tcctaaaatt aattaagctt gcaggtgaca ataacctcaa caatctgagt    1260

gaattatatt ttttgttcag aatatttgga cacccaatgg tagatgaaag acaagccatg    1320

gatgctgtta aagttaattg caacgagacc aaattttatt tgttaagtag tttgagtatg    1380

ttaagaggag cttttatata tagaattata aaagggtttg taaataatta caacagatgg    1440

cctactttaa gaaatgccat tgtcttaccc ttaagatggt taacttacta taaactaaac    1500

acttatcctt ccttgttgga acttacagaa agagatttga ttgttctatc aggactacgt    1560

ttctatcgag agtttcggtt gcctaaaaaa gtggatcttg aaatgatcat aaatgataag    1620

gctatatcac ctcctaaaaa tttaatatgg actagtttcc ctagaaatta tatgccgtca    1680

cacatacaaa attatataga acatgaaaaa ttaaaattct ctgatagtga taaatcaaga    1740

agagtattag agtattattt aagagataac aaattcaatg aatgtgattt acacaactgt    1800

gtagttaatc aaagttatct taacaacccg aatcatgtgg tatcattgac aggcaaagaa 1860

agagaactca gtgtaggtag aatgtttgca atgcaaccag gaatgttcag acaagttcaa 1920

atattagcag agaaaatgat agcagaaaac atattacaat ttttccctga aagtcttaca 1980

agatatggtg atctagaact acagaaaata ttagaattga aagcaggaat aagtaacaaa 2040

tcaaatcgtt acaatgataa ttacaacaat tacattagta agtgctctat catcacagat 2100

ctcagcaaat tcaatcaagc atttcgatat gaaacatcat gtatttgtag tgatgtactg 2160

gatgaactgc atggtgtaca atctctattt tcctggttac atttaactat tcctcatgtc 2220

acaataatat gcacatatag gcatgcaccc ccctatataa aggatcatat tgtagatctt 2280

aacaatgtag atgagcaaag tggactatat agatatcata tgggtggtat cgaagggtgg 2340

tgtcaaaaac tatggaccat agaagctata tcactattag atctaatatc tctcaaaggg 2400

aaattctcaa ttactgcttt aattaatggt gacaatcaat caatagatat aagtaaacca 2460

gtcagactca tggaaggtca aactcatgct caagcagatt atttgctagc attaaatagt 2520

ctcaaattac tgtataaaga gtatgcagga ataggccaca aattaaaagg aactgagact 2580

tatatatcga gagatatgca atttatgagt aaaacgatcc aacataacgg tgtatattac 2640

ccagctagta taaagaaagt cctaagagtg ggaccgtgga taaacactat acttgatgac 2700

ttcaaagtga gtctagaatc tataggtagt ttgacacaag aattagaata tagaggtgaa 2760

agtctattat gcagtttaat atttagaaat gtatggttat ataatcaaat tgcattacaa 2820

cttaaaaatc atgcattatg taacaacaaa ttatatttgg atatattaaa agttctaaaa 2880

cacttaaaaa ccttttttaa tcttgataac attgatacag cattaacatt gtatatgaat 2940

ttgcccatgt tatttggtgg tggtgatccc aacttgttat atcgaagttt ctatagaaga 3000

actcctgatt tcctcacaga ggctatagtt cactctgtgt tcatacttag ttattataca 3060

aaccatgatt taaaagataa acttcaagat ctgtcagatg atagattgaa taagttctta 3120

acatgcataa tcacgtttga taaaaacccc aatgctgaat tcgttacatt gatgagagat 3180

cctcaagctt taggatctga gaggcaagct aaaattacta gcgaaatcaa tagactggca 3240

gttaccgagg ttttgagcac agctccaaac aaaatatttt ccaaaagtgc acaacactat 3300

accactacag agatagatct taatgatatt atgcaaaata tagaacctac atatcctcac 3360

gggctaagag ttgtttatga gagtttaccc ttttataaag cagagaaaat agtaaatctt 3420

atatccggta caaaatctat aactaacata ctggaaaaga cttctgccat agacttaaca 3480

gatattgata gagccactga gatgatgagg aaaaacataa ctttgcttat aaggatatta 3540

ccattagatt gtaacagaga taaaagagaa atattgagta tggaaaacct aagtattact 3600

gaattaagca aatacgttag agaaagatct tggtctttat ccaatatagt tggtgttaca 3660

tcacccagta tcatgtatac aatggacata aaatatacaa caagcactat agctagtggc 3720

ataatcatag agaaatataa tgtcaacagt ttaacacgtg gtgagagagg acccactaaa 3780

ccatgggttg gttcatctac acaagagaaa aagacaatgc cagtttataa tagacaagtt 3840

ttaaccaaaa aacagagaga tcaaatagat ctattagcaa aattggattg ggtgtatgca 3900

tctatagata acaaggatga atttatggag gaacttagca taggaactct tgggttaaca 3960

tatgagaagg ccaaaaaatt attcccacaa tatttgagtg ttaactattt gcatcgtctt 4020

acagtcagta gtagaccatg tgaattccct gcatctatac cagcttatag aactacaaat 4080

tatcactttg atactagccc tattaatcgc atattaacag aaaagtatgg tgatgaagat 4140

attgatatag tattccaaaa ctgtataagc tttggcctta gcttaatgtc tgtagtagaa 4200

caatttacta atgtatgtcc taacagaatt attctcatac ccaagcttaa tgagatacat 4260

ttgatgaaac ctcccatatt cacaggcgat gttgatattc acaagttaaa acaagtgata 4320

caaaaacaac atatgttttt accagacaaa ataagtttga ctcaatatgt ggaattattc 4380

ttaagtaata aaacactcaa atctggatct aatgttaatt ctaatttaat attggcgcat 4440

aagatatctg actattttca taatacttac attttgagta ctaatttagc tggacattgg 4500

attcttatta tacaacttat gaaagattct aagggtattt ttgaaaaaga ttggggagag 4560

ggatatataa ctgatcatat gttcattaat ttgaaagttt tcttcaatgc ttataagaca 4620

tatctcttgt gttttcataa aggttacggc agagcaaagc tggagtgtga tatgaatact 4680

tcagatctcc tatgtgtatt ggaattaata gacagtagtt attggaagtc tatgtctaag 4740

gtgtttttag aacaaaaagt tatcaaatac attcttagcc aggatgcaag tttacataga 4800

gtaaaaggat gtcatagctt caaactatgg tttcttaaac gtcttaatgt agcagaattc 4860

acagtttgcc cttgggttgt taacatagat tatcatccaa cacatatgaa agcaatatta 4920

acttatattg atcttgttag aatgggattg ataaatatag atagaatata cattaaaaat 4980

aaacacaagt tcaatgatga gttttatact tctaatctgt tttacattaa ttataacttc 5040

tcagataata ctcatctatt aactaaacat ataaggattg ctaattccga attagaaagt 5100

aattacaaca aattatatca tcctacacca gaaaccctag aaaatatact aaccaatccg 5160

gttaaaagta atgagaaaaa gacactgagt gactattgta taggtaaaaa tgttgactca 5220

ataatgttac catcgttatc taataagaag cttattaaat cgtctacaat gattagaacc 5280

aattacagca gacaagattt gtataattta tttcctacgg ttgtgattga taaaattata 5340

gatcattcag gtaatacagc caaatctaac caactttaca ctactacttc tcatcaaata 5400

tccttagtgc acaatagcac atcactttat tgcatgcttc cttggcatca tattaataga 5460

ttcaattttg tatttagttc tacaggttgt aaaattagta tagagtatat tttaaaagat 5520

cttaaaatta aggatcctaa ttgtatagca ttcataggtg aaggagcagg gaatttatta 5580

ttgcgtacag tagtggaact tcatcctgat ataagatata tttacagaag tctgaaagat 5640

tgcaatgatc atagtttacc aattgagttt ttaaggctgt acaatggaca tatcaacatt 5700

gattatggtg aaaatttgac cattcctgct acagatgcaa ccaacaacat tcattggtct 5760

tatttacata taaagtttgc tgaacctatc agtctttttg tctgtgatgc tgaattgcct 5820

gtaacagtca actggagtaa gattataata gagtggagca agcatgtaag aaaatgcaag 5880

tactgttctt cagttaataa atgtacattg atagtaaaat atcatgctca agatgatatc 5940

gatttcaaat tagacaacat aactatatta aaaacttatg tatgcttagg tagtaagtta 6000

aagggatctg aagtttactt agtccttaca ataggtcctg caaatgtgtt cccagtattt 6060

aatgtagtac aaaatgctaa attgatacta tcaagaacta aaaatttcat catgcctaaa 6120

aaagctgata aagagtctat tgatgcaaat attaagagtt tgataccctt tctttgttac 6180

cctataacaa aaaaaggaat taatactgca ttgtctaaat taaagagtgt tgttagtgga 6240

gatatactat catattctat agctggacgt aatgaagttt tcagcaataa acttataaat 6300

cataagcata tgaacatctt aaagtggttc aatcatgttt taaatttcag atcaacagaa 6360

ttaaactata atcatttata tatggtagaa tctacttatc ctcatctaag tgaattgtta 6420

aacagcttga caaccaatga acttaaaaaa ctgattaaaa tcacaggtag tttgttatac 6480

aacttttata atgaataa 6498